(19) Europäisches Patentamt
European Patent Office
Office européen des brevets

(11) Publication number: **0 210 804**
**B1**

(12) # EUROPEAN PATENT SPECIFICATION

(45) Date of publication of the patent specification: **10.05.89**

(51) Int. Cl.⁴: **C 07 F 9/09**, C 07 F 9/165, C 07 F 9/65, A 61 K 31/665, A 61 K 31/675

(21) Application number: **86305559.6**

(22) Date of filling: **18.07.86**

(54) **Phosphate ester derivatives, their preparation and their therapeutic use.**

(30) Priority: **26.07.85 JP 163965/85**

(43) Date of publication of application: **04.02.87 Bulletin 87/6**

(45) Publication of the grant of the patent: **10.05.89 Bulletin 89/19**

(84) Designated Contracting States: **AT BE CH DE FR GB IT LI LU NL SE**

(56) References cited:
**EP-A-0 035 375**
**DE-A-2 437 832**

(73) Proprietor: **SANKYO COMPANY LIMITED, No. 1-6, 3-chome Nihonbashi Honcho Chuo- ku, Tokyo (JP)**

(72) Inventor: **Nakamura, Norio Sankyo Chemical Research Labs., Sankyo Co. Limited No. 2-58, 1-chome, Hiromachi, Shinagawa- ku Tokyo 140 (JP)**
Inventor: **Ookawa, Nobuyuki Sankyo Chemical Research Labs., Sankyo Co. Limited No. 2-58, 1-chome, Hiromachi, Shinagawa- ku Tokyo 140 (JP)**
Inventor: **Koike, Hiroyuki Sankyo Biological Research Labs., Sankyo Co. Limited No. 2-58, 1-chome, Hiromachi, Shinagawa- ku Tokyo 140 (JP)**
Inventor: **Sada, Toshio Sankyo Biological Research Labs., Sankyo Co. Limited No. 2-58, 1-chome, Hiromachi, Shinagawa- ku Tokyo 140 (JP)**
Inventor: **Oshima, Takeshi Sankyo Biological Research Labs., Sankyo Co. Limited No. 2-58, 1-chome, Hiromachi, Shinagawa- ku Tokyo 140 (JP)**
Inventor: **Iizuka, Yoshio Sankyo Biological Research Labs., Sankyo Co. Limited No. 2-58, 1-chome, Hiromachi, Shinagawa- ku Tokyo 140 (JP)**

(74) Representative: **Tubby, David George, MARKS & CLERK 57- 60 Lincoln's Inn Fields, London WC2A 3LS (GB)**

**EP 0 210 804 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1989

## Description

The present invention relates to a series of novel phosphate ester derivatives containing a cyclic ether system and provides processes for producing these compounds and compositions containing them for therapeutic use, especially for use as antagonists to platelet activating factor (hereinafter abbreviated to "PAF").

Natural PAF, at least as isolated from mammalian tissues, is a mixture of from 2 to 5 phospholipids, the number depending upon the nature of the original tissue. The major constituents of PAF may be represented by the formula (A):

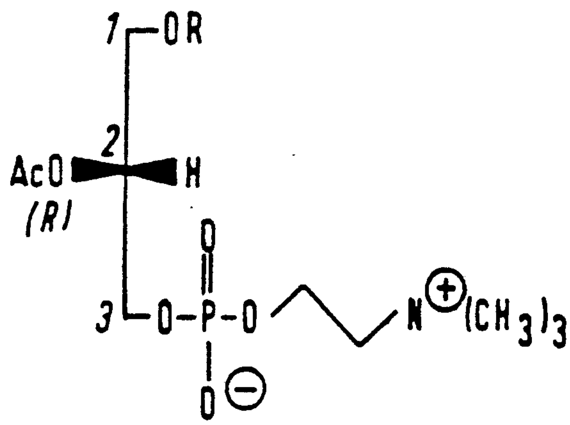

(A)

in which

R represents a long chain aliphatic hydrocarbon group, which may be saturated or unsaturated. Natural PAF is laevorotatory and the various components of natural PAF may be identified, for example as: 1-$C_{16:0}$ = formula (A) where

R represents a hexadecyl group; 1-$C_{18:0}$ = formula (A) where

R represents an octadecyl group: 1-$C_{18:1}$ = formula (A) where

R represents a 9(Z)-octadecenyl group.

PAF exhibits a strong plate let activating and aggregating effect. It also has a hypotensive effect and increases vasopermeability; it is believed to be an active agent in the induction of the shock state (for example endotoxin-induced shock) and to act as a mediator of inflammatory disease. Accordingly, PAF antagonists have been investigated with a view to developing new types of anti-shock agent and of anti-inflammatory agent. Accordingly, analogues of natural PAF's have been investigated in an attempt to find such PAF antagonists. Currently, two compounds are known as PAF antagonists. The compound of formula (B):

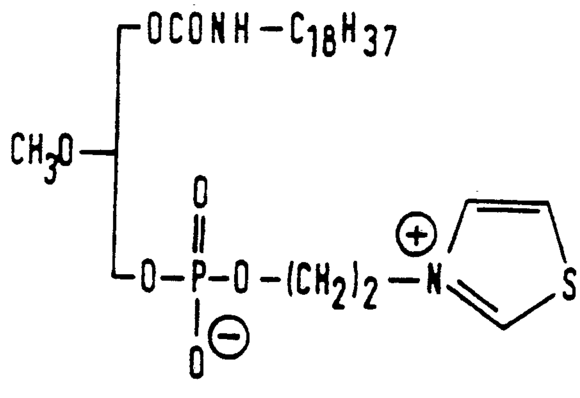

(B)

(also known as CV-3988) is disclosed in US patent No. 4 408 052, whilst the compound of formula (C):

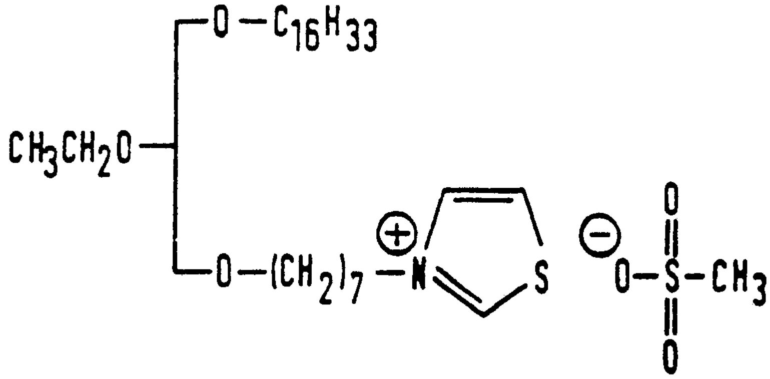

(C)

(known as ONO-6240) is disclosed in European Patent Publication No. 146 258. These compounds, however, are unsatisfactory for one or more of the following reasons: they lack sufficient intensity of antagonism towards PAF; the duration of their effect is insufficient; biological utilization is inadequate.

We have now discovered a series of PAF antagonists which are phospholipid derivatives having a cyclic structure, which is different from the glycerin chain derivatives already known, for example those of formulae (B) and (C) disclosed above. Structurally, the prior art compounds closest to the compounds of the invention are those compounds disclosed in European Patent Application No. 86 300 278.8. These prior compounds may be represented by the formula (D):

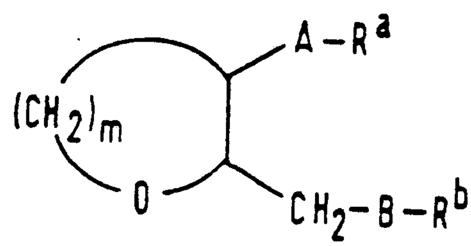

(D)

where

A, B and m are as defined hereafter in relation to the compounds of the invention, whilst one of $R^a$ and $R^b$ represents a $C_{10}$-$C_{22}$ alkyl group and the other represents a phosphoric ester group of formula (II), as defined hereafter in relation to the compounds of the invention. However, these prior compounds are useful as anti-tumour agents, and appear to be substantially free from PAF-like activity and from PAF antagonistic activity. On the contrary, the compounds of the present invention have been found to be excellent PAF antagonists, resulting in anti-asthmatic, anti-inflammatory and anti-shock activities which have excellent duration, biological utilization and level of activity.

The compounds of the invention are phosphate esters of formula (I):

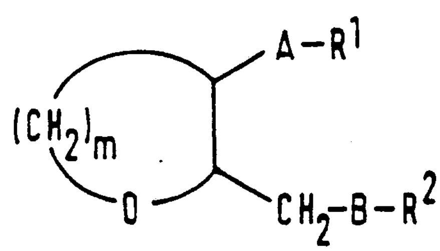

(I)

in which:

m is an integer of from 2 to 4;

A and B are the same or different and each represents an oxygen atom or a sulphur atom; and

one of $R^1$ and $R^2$ represents a group of formula $-CONH-R^3$, where $R^3$ represents a $C_{10}$-$C_{22}$ alkyl group, and the other represents a group of formula (II):

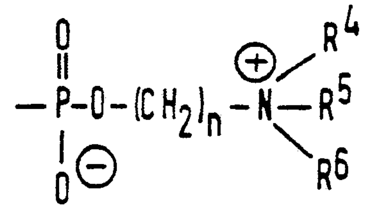

(II)

in which:

n represents an integer of from 2 to 10; and

$R^4$, $R^5$ and $R^6$ are the same or different and each represents a hydrogen atom or a $C_1$-$C_6$ alkyl group, or $R^4$ and $R^5$ or $R^4$ $R^5$ and $R^6$, together with the nitrogen atom to which they are attached, form a heterocyclic ring which may be aromatic or partly or wholly saturated:

and pharmaceutically acceptable salts thereof.

The invention also provides a pharmaceutical composition for the treatment of inflammation or shock, comprising a PAF antagonist in combination with a pharmaceutically acceptable carrier or diluent, wherein the PAF antagonist is selected from the group consisting of compounds of formula (I) and pharmaceutically acceptable salts thereof.

The compounds of the invention may be used for the treatment or prophylaxis of asthma, inflammation or shock by administering an amount of the PAF antagonist to an animal (which may be a mammal, e. g. human) sufficient to effect treatment or prophylaxis of inflammation or shock.

The compounds of the invention may be prepared by reacting a compound of formula (III):

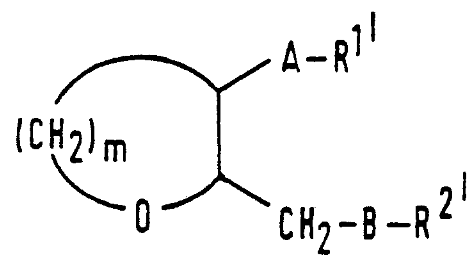

(III)

in which m, A and a are as defined above; and

one of $R^{1'}$ and $R^{2'}$ represents said group of formula -CONH-$R^3$ and the other represents a group of formula (IV):

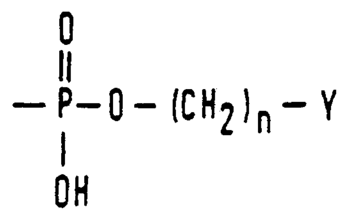

(IV)

(in which n is as defined and Y represents a halogen atom) or a group of formula (V):

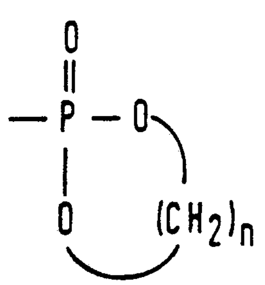

(V)

(in which n is as defined above) with an amine compound of formula (VI):

$R^4NR^5R^6$ (VI)

(in which $R^4$, $R^5$ and $R^6$ are as defined above).

The compounds of the invention can exist in the form of an inner salt, i. e. as shown above in which one of $R^1$ and $R^2$ represents a group of formula (II):

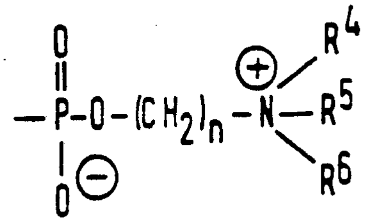

(II)

(in which n, $R^4$, $R^5$ and $R^6$ are as defined above) or it may exist in the form of a salt, in which one of $R^1$ and $R^2$ represents a group of formula (IIa):

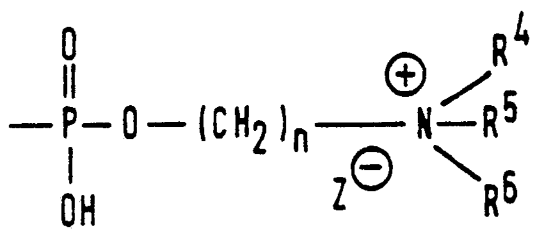

(IIa)

(in which n, $R^4$, $R^5$ and $R^6$ are as defined above, and Z represents a pharmaceutically acceptable anion, preferably a hydroxy group, a halogen atom, a $C_1$-$C_6$ alkylsulphonyloxy group or an arylsulphonyloxy group).

Salts in which one of $R^1$ and $R^2$ represents the aforementioned group of formula (IIa) can also form salts with cations, particularly metals (e. g. alkali metals such as sodium or potassium or alkaline earth metals such as calcium or magnesium), in which the cation replaces the hydrogen atom of the hydroxy group attached to the phosphorus atom in the group of formula (IIa).

Where Z in the above formula (IIa) represents a halogen atom, this may be, for example, a chlorine, bromine or iodine atom. Where Z represents an alkylsulphonyloxy group, the alkyl part is $C_1$-$C_6$ and may be a straight or branched chain group; examples include the methanesulphonyloxy and ethanesulphonyloxy groups. Where Z represents an arylsulphonyloxy group, the aryl part is a $C_6$-$C_{10}$ carbocyclic aryl group, which may be substituted or unsubstituted and, if substituted, may have from 1 to 3 substituents preferably selected from $C_1$-$C_4$ alkyl (preferably methyl) groups, halogen atoms, $C_1$-$C_4$ alkoxy groups and nitro groups. Examples of such arylsulphonyloxy groups include the benzenesulphonyloxy and p-toluenesulphonyloxy groups.

In the compounds of the invention, one of $R^1$ and $R^2$ represents the above defined group of formula (II) [or (IIa)], whilst the other represents an alkylcarbamoyl group of formula -CONH-$R^3$ where $R^3$ represents a $C_{10}$-$C_{22}$ alkyl group, which may be a straight or branched chain group. Examples of such alkyl groups include the decyl, 3-methylnonyl, 8-methylnonyl, 3-ethyloctyl, 3,7-dimethyloctyl, undecyl, dodecyl, tridecyl, tetradecyl, pentadecyl, hexadecyl, 1-methylpentadecyl, 14-methylpentadecyl, 13,13-dimethyltetradecyl, heptadecyl, 15-methylhexadecyl, octadecyl, 1-methylheptadecyl, nonadecyl, icosyl, henicosyl and docosyl groups. Straight and branched chain alkyl groups having from 16 to 18 carbon atoms, for example the hexadecyl, 1-methylpentadecyl, 14-methylpentadecyl, 13,13-dimethyltetradecyl, heptadecyl, 15-methylhexadecyl, octadecyl and 1-methylheptadecyl groups are preferred.

Where $R^4$, $R^5$ or $R^6$ represents an alkyl group, this is a lower alkyl group having from 1 to 6 carbon atoms and it may be a straight or branched chain group. The group more preferably has from 1 to 5 carbon atoms and examples of such groups include the methyl, ethyl, propyl, isopropyl, butyl, isobutyl, sec-butyl, t-butyl and pentyl groups, of which the methyl and ethyl groups are preferred.

Where one of $R^4$, $R^5$ and $R^6$ (for example $R^6$) represents a hydrogen atom, the group of formula (II) which is represented by one of $R^1$ and $R^2$, may be represented by the following formula (IIb):

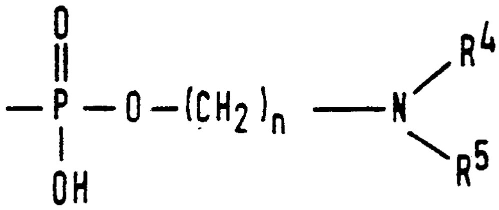

(IIb)

(in which $R^4$, $R^5$ and n are as defined above) which is tautomeric with the group of formula (IIc):

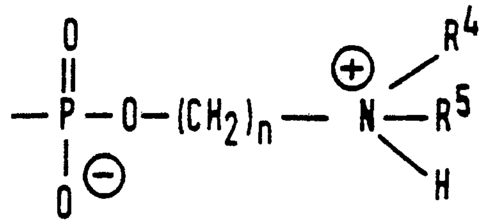

(IIc)

(in which $R^4$, $R^5$ and n are as defined above).

Where $R^4$ and $R^5$ or $R^4$, $R^5$ and $R^6$ represents a heterocyclic group this will generally contain from 3 to 10 ring atoms and, in general, of these, at least one, preferably from 1 to 4 and more preferably 1 or 2, atoms (including the nitrogen atom to which $R^4$, $R^5$ and $R^6$ are attached) are nitrogen, oxygen or sulphur hetero-atoms. Specific examples of such groups are given below. Such groups may be unsubstituted or may have at least one substituent, the maximum number of substituents being dictated by the number of substitutable positions and other factors, such as steric constraints. However, in general, where the groups are substituted, from 1 to 3 substituents are preferred. Examples of such substituents include: $C_1$-$C_6$ preferably $C_1$-$C_5$, alkyl groups (such as those exemplified above in relation to $R^4$, $R^5$ and $R^6$), $C_1$-$C_6$, preferably $C_1$-$C_5$, alkoxy groups (such as the methoxy, ethoxy, propoxy, isopropoxy, butoxy and isobutoxy groups, preferably the methoxy or ethoxy groups), $C_1$-$C_5$ hydroxyalkyl groups (such as the hydroxymethyl, 1-hydroxyethyl, 2-hydroxyethyl, 1-hydroxypropyl, 3-hydroxypropyl, 4-hydroxybutyl and 5-hydroxypentyl groups), carbamoyl groups, mono- and di-alkylcarbamoyl groups in which the or each alkyl part $C_1$-$C_5$ (such as the methylcarbamoyl, ethylcarbamoyl, propylcarbamoyl, isopropylcarbamoyl, butylcarbamoyl, isobutylcarbamoyl, sec-butylcarbamoyl, t-butylcarbamoyl, pentylcarbamoyl, t-pentylcarbamoyl, dimethylcarbamoyl, diethylcarbamoyl, dipropylcarbamoyl, dibutylcarbamoyl, methylethylcarbamoyl and methylpropylcarbamoyl groups) and halogen atoms (such as the fluorine, chlorine or bromine atoms).

Where $R^4$ and $R^5$, together with the nitrogen atom to which they are attached, form a heterocyclic ring system, this is necessarily a non-aromatic ring system and should contain from 3 to 10, preferably from 3 to 7 and more preferably from 5 to 7, ring atoms (including the aforementioned nitrogen atom) and have at least one hetero-atom (the aforementioned nitrogen atom). It may have additionally from 0 to 3 nitrogen, oxygen or sulphur hetero-atoms. Examples of such non-aromatic heterocyclic groups include the 1-pyrrolidinyl, piperidino, 1-azepinyl, 1-aziridinyl, 1-azetidinyl, morpholino and thiomorpholino ( = perhydro-1,4-thiazin-4-yl) groups. Of these, the 1-pyrrolidinyl and piperidino groups are preferred. These non-aromatic heterocyclic groups may be substituted or unsubstituted and, if substituted, the substituents may be selected from those defined above in relation to heterocyclic groups generally.

Where $R^4$, $R^5$ and $R^6$, together with the nitrogen atom to which they are attached, form a heterocyclic ring, this may be an aromatic heterocyclic group or may be a non-aromatic heterocyclic group in which the aforementioned nitrogen atom is doubly bonded, but it is preferably an aromatic heterocyclic ring, and preferably has from 5 to 10 ring atoms, of which at least the aforementioned nitrogen atom is a hetero-atom; it may contain from 0 to 3 additional hetero-atoms selected from nitrogen, oxygen and sulphur atoms. Examples of such aromatic heterocyclic rings include the 1-pyridyl, 3-thiazolyl, 3-oxazolyl, 1-pyridazinyl, 1-quinolyl, 2-isoquinolyl, 1-imidazolyl and N-triazolyl groups. Such groups may be substituted or unsubstituted and, if substituted, may have at least one (and preferably from 1 to 3) substituents selected from those substituents identified generally above, of these, 5- and 6- membered aromatic heterocyclic groups, such as the unsubstituted 1-pyridyl and 3-thiazolyl groups, are preferred.

The value n is an integer from 2 to 10, preferably from 2 to 6, e. g. 2 or 3.

Since the carbon atoms at the α- and β-positions (relative to the ether oxygen atom) of the ether ring are asymmetric, a total of four stereoisomers of each compound is possible, in which each asymmetric carbon atom is in the R-configuration or the S-configuration, i. e. R,R, R,S, S,S and S,R isomers. The chiral synthesis of the compounds of the invention can be possible. The present invention envisages both the individual isolated isomers, as well as mixtures of these isomers. Where the process of the invention produces the compounds of the invention in the form of mixtures of isomers, these may, if desired, be employed as the mixtures, or the mixtures may be separated into individual isomers by conventional separation or resolution techniques.

Of the compounds of the invention, we prefer those compounds in which:

(1) m is 2 or 3.
(2) n is an integer from 2 to 6.
(3a) $R^4$, $R^5$ and $R^6$ are the same or different and each represents a hydrogen atom or a $C_1$-$C_6$ alkyl group.
(3b) $R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group.
(3c) $R^4$, $R^5$ and $R^6$, together with the nitrogen atom to which they are attached, represent an aromatic heterocyclic group having from 5 to 10 ring atoms, said heterocyclic group being unsubstituted or having at least one $C_1$-$C_5$ alkyl, $C_1$-$C_5$ alkoxy, $C_1$-$C_5$ hydroxyalkyl, carbamoyl or halogen substituent.
(4) $R^1$ represents said group of formula $-CONHR^3$ and $R^2$ represents said group of formula (II).
(5) $R^3$ represents an alkyl group having from 16 to 18 carbon atoms.

In particular, preferred compounds are compounds of formula (I), in which:

$R^1$ represents said group of formula $-CONHR^3$;
$R^2$ represents said group of formula (II);
$R^4$, $R^5$ and $R^6$ are the same or different and each represents a hydrogen atom or a $C_1$-$C_6$ alkyl group;
or
$R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group;
or
$R^4$, $R^5$ and $R^6$, together with the nitrogen atom to which they are attached, represent an aromatic heterocyclic group having from 5 to 10 ring atoms, said group being unsubstituted or having at least one $C_1$-$C_5$ alkyl, $C_1$-$C_5$ alkoxy, $C_1$-$C_5$ hydroxyalkyl, carbamoyl or halogen substituent;
n is an integer from 2 to 6; and
m is 2 or 3.

More preferred compounds are compounds of formula (I), in which:
$R^1$ represents a group of formula $-CONHR^3$, where $R^3$ represents a $C_{16}$-$C_{18}$ alkyl group;
$R^2$ represents said group of formula (II);
$R^4$, $R^5$ and $R^6$ are the same or different and each represents a $C_1$-$C_6$ alkyl group;
or
$R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group;
or
$R^4$, $R^5$ and $R^6$, together with the nitrogen atom to which they are attached, represent an aromatic heterocyclic group having from 5 to 7 ring atoms, said group being unsubstituted or having at least one $C_1$-$C_5$ alkyl or $C_1$-$C_5$ hydroxyalkyl substituent;
n is an integer from 2 to 6; and
m is 2 or 3.

The most preferred compounds are compounds of formula (I), in which:
$R^1$ represents a group of formula $-CONHR^3$ where $R^3$ represents a $C_{16}$-$C_{18}$ alkyl-group;
$R^2$ represents said group of formula (II);
$R^4$, $R^5$ and $R^6$ are the same or different and each represents a $C_1$-$C_6$ alkyl groups
or
$R^4$ and $R^5$, together with the nitrogen atom to which they are attached, represent a piperidino or 1-pyrrolidinyl group, and $R^6$ represents a hydrogen atom or $C_1$-$C_6$ alkyl group;
or
$R^4$, $R^5$ and $R^6$, together with the nitrogen atom to which they are attached, represent a 1-pyridyl or 1-thiazolyl group which is unsubstituted or has at least one $C_1$-$C_5$ alkyl or $C_1$-$C_5$ hydroxyalkyl substituent;
n is an integer from 2 to 6; and
m is 2 or 3.

Examples of certain preferred compounds of the present invention are given in the following Tables 1 - 12, in which compounds of formula (I - 1) are as defined in Table 1, compounds of formula (I - 2) are as defined in Table 2 and so on.

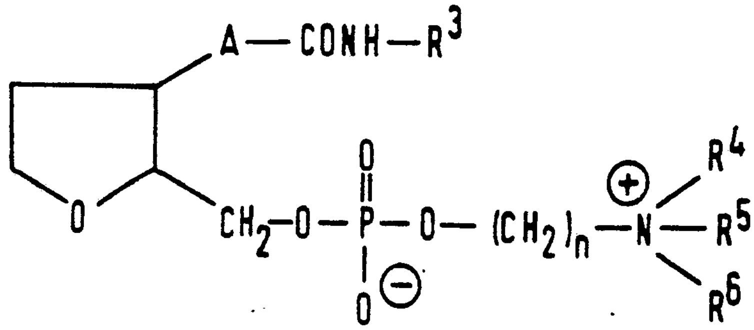

(I - 1)

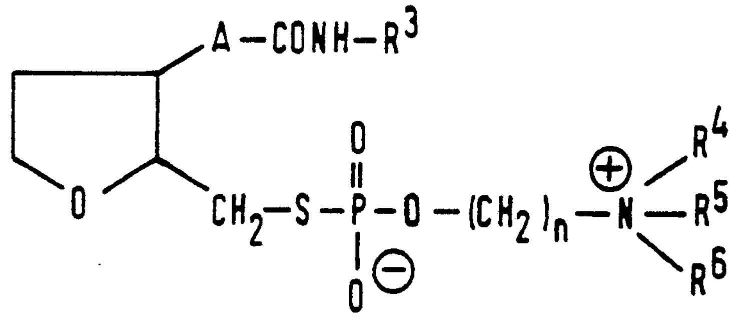

(I-2)

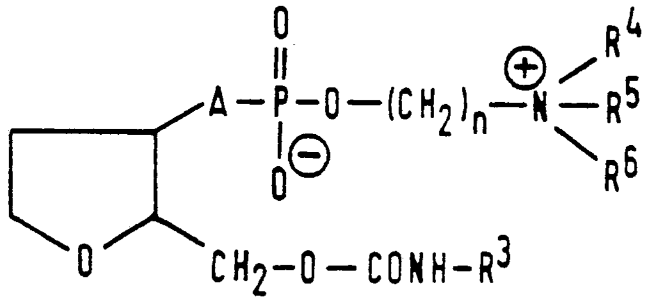

(I-3)

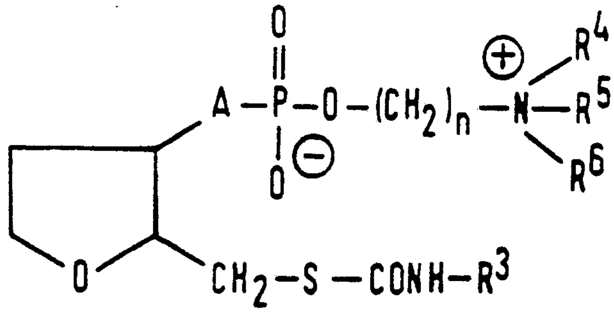

(I-4)

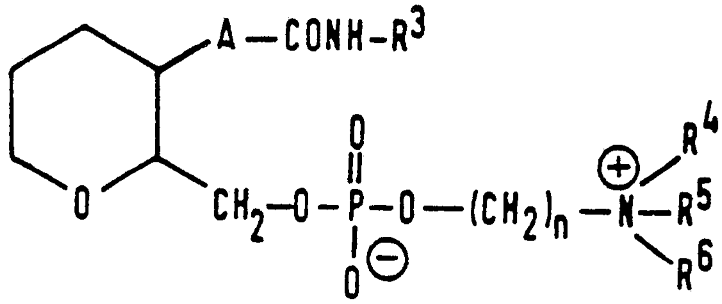

(I-5)

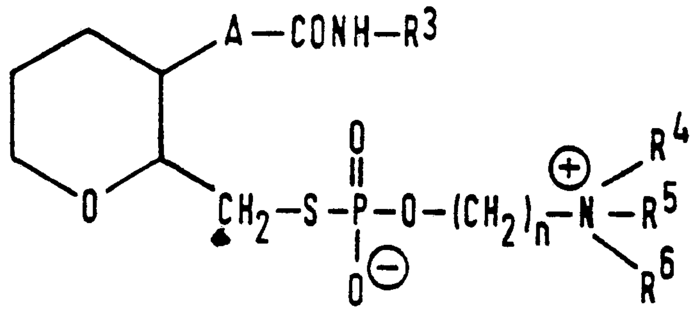

(I-6)

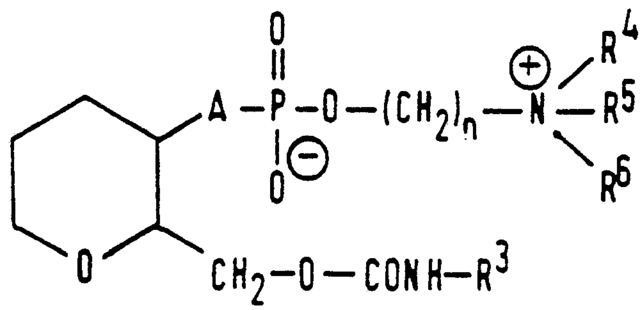

(I-7)

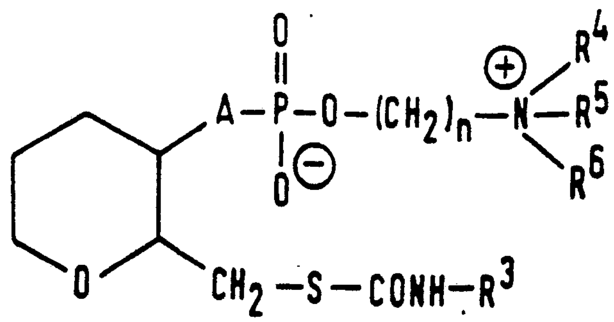

(I-8)

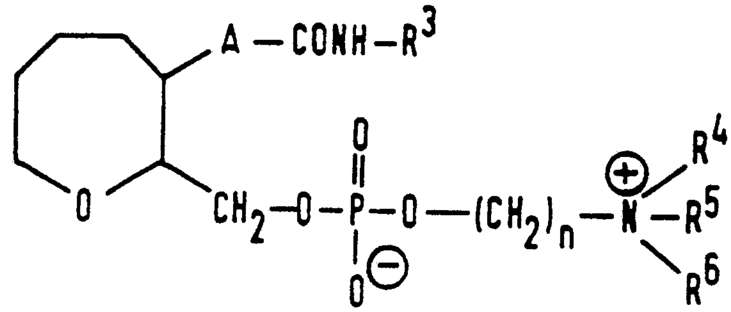

(I-9)

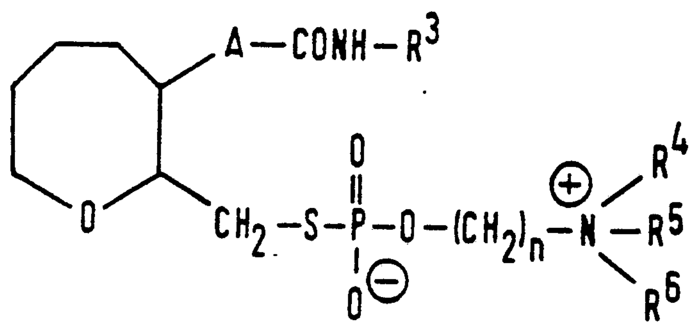

(I-10)

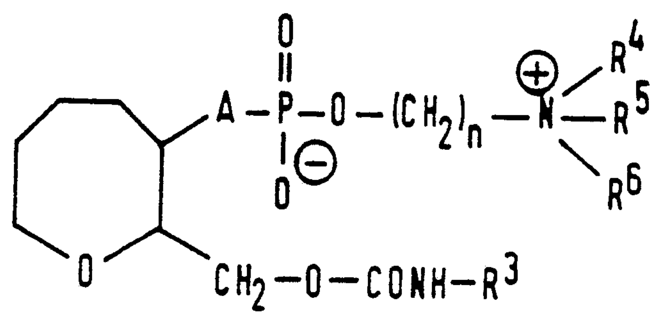

(I-11)

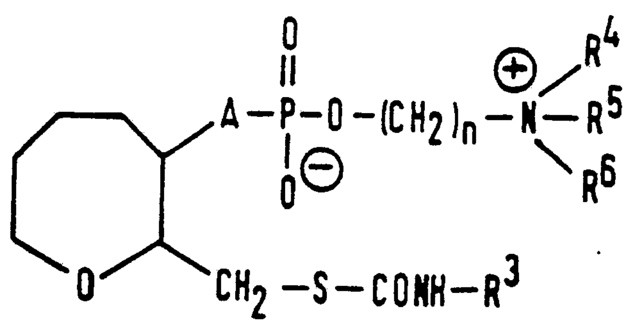

(I-12)

In the following Tables, the abbreviations used have the meanings:

| | |
|---|---|
| Me | methyl |
| Pip+ | piperidinium |
| Pyr+ | pyridinium |
| Thi+ | 1,3-thiazolium |

All alkyl groups are "normal", i. e. straight chain, except where explicitly shown otherwise.

Table 1

| Cpd No. | $R^3$ | A | n | $-N^+R^4R^5R^6$ |
|---|---|---|---|---|
| 1 | $C_{17}H_{35}$ | O | 2 | Thi+ |
| 2 | $C_{16}H_{33}$ | O | 3 | Thi+ |
| 3 | $C_{18}H_{37}$ | O | 2 | Thi+ |
| 4 | $C_{18}H_{37}$ | O | 3 | Thi+ |
| 5 | $C_{17}H_{35}$ | O | 2 | $-N^+H_3$ |
| 6 | $C_{17}H_{35}$ | O | 2 | $-N^+Me_3$ |
| 7 | $C_{18}H_{37}$ | O | 2 | $-N^+Me_3$ |
| 8 | $C_{17}H_{35}$ | O | 3 | 1-Me-Pip+ |
| 9 | $C_{18}H_{37}$ | O | 2 | Pyr+ |
| 10 | $C_{17}H_{35}$ | S | 2 | Thi+ |
| 11 | $C_{18}H_{37}$ | S | 2 | $-N^+Me_3$ |
| 12 | $C_{14}H_{29}CH(Me)$- | S | 3 | Thi+ |
| 13 | $Me_3C-(CH_2)_{12}$- | S | 2 | $-N^+H_3$ |
| 14 | $Me_2CH-(CH_2)_{13}$- | S | 3 | $-N^+Me_3$ |
| 15 | $C_{16}H_{33}$ | S | 2 | 1-Me-Pip+ |
| 16 | $C_{18}H_{37}$ | S | 2 | Pyr+ |
| 17 | $C_{17}H_{35}$ | O | 4 | $-N^+H_3$ |
| 18 | $C_{17}H_{35}$ | O | 5 | 1-Me-Pip+ |
| 19 | $C_{17}H_{35}$ | S | 6 | Thi+ |
| 20 | $C_{14}H_{29}CH(Me)$- | S | 6 | Thi+ |
| 21 | $Me_3C-(CH_2)_{12}$- | S | 7 | $-N^+H_3$ |
| 22 | $Me_2CH-(CH_2)_{13}$- | S | 8 | $-N^+Me_3$ |
| 23 | $C_{16}H_{33}$ | S | 9 | 1-Me-Pip+ |
| 24 | $C_{18}H_{37}$ | S | 10 | Pyr+ |

Table 2

| Cpd No. | $R^3$ | A | n | $-N^+R^4R^5R^6$ |
|---|---|---|---|---|
| 25 | $C_{16}H_{33}$ | O | 2 | Thi+ |
| 26 | $C_{18}H_{37}$ | O | 2 | Thi+ |
| 27 | $C_{16}H_{33}$ | O | 3 | Thi+ |
| 28 | $C_{18}H_{37}$ | O | 3 | Thi+ |
| 29 | $C_{14}H_{29}CH(Me)$- | O | 2 | Thi+ |
| 30 | $Me_3C-(CH_2)_{12}$- | O | 2 | $-N^+H_3$ |
| 31 | $Me_2CH-(CH_2)_{13}$- | O | 2 | $-N^+HMe_2$ |
| 32 | $C_{16}H_{33}$ | S | 2 | $-N^+HMe_2$ |
| 33 | $C_{18}H_{37}$ | S | 3 | $-N^+HMe_2$ |
| 34 | $C_{16}H_{33}$ | S | 2 | $-N^+H_3$ |
| 35 | $C_{18}H_{37}$ | S | 2 | $-N^+Me_3$ |
| 36 | $C_{18}H_{37}$ | O | 4 | Thi+ |
| 37 | $Me_3C-(CH_2)_{12}$- | O | 6 | $-N^+H_3$ |
| 38 | $Me_2CH_2(CH_2)_{13}$- | O | 8 | $-N^+HMe_2$ |

Table 3

| Cpd No. | $R^3$ | A | n | $-N^+R^4R^5R^6$ |
|---|---|---|---|---|
| 39 | $C_{17}H_{35}$ | O | 2 | Thi+ |
| 40 | $C_{16}H_{33}$ | O | 3 | Thi+ |
| 41 | $C_{18}H_{37}$ | O | 2 | Thi+ |
| 42 | $C_{18}H_{37}$ | O | 3 | Thi+ |
| 43 | $C_{17}H_{35}$ | O | 2 | $-N^+H_3$ |
| 44 | $C_{16}H_{33}$ | O | 3 | $-N^+Me_3$ |
| 45 | $C_{18}H_{37}$ | O | 2 | $-N^+HMe_2$ |
| 46 | $C_{17}H_{35}$ | O | 3 | 1-Me-Pip+ |
| 47 | $C_{18}H_{37}$ | O | 2 | Pyr+ |
| 48 | $C_{17}H_{35}$ | S | 2 | Thi+ |
| 49 | $C_{18}H_{37}$ | S | 3 | Thi+ |
| 50 | $C_{14}H_{29}CH(Me)-$ | S | 3 | Thi+ |
| 51 | $Me_3C-(CH_2)_{12}-$ | S | 2 | $-N^+H_3$ |
| 52 | $Me_2CH-(CH_2)_{13}-$ | S | 3 | $-N^+Me_3$ |
| 53 | $C_{16}H_{33}$ | S | 2 | 1-Me-Pip+ |
| 54 | $C_{18}H_{37}$ | S | 2 | Pyr+ |
| 55 | $C_{18}H_{37}$ | O | 4 | Thi+ |
| 56 | $C_{18}H_{37}$ | O | 5 | Thi+ |
| 57 | $C_{17}H_{35}$ | O | 6 | $-N^+H_3$ |
| 58 | $C_{16}H_{33}$ | O | 7 | $-N^+Me_3$ |
| 59 | $C_{18}H_{37}$ | O | 8 | $-N^+HMe_2$ |
| 60 | $C_{17}H_{35}$ | O | 9 | 1-Me-Pip+ |
| 61 | $C_{18}H_{37}$ | O | 10 | Pyr+ |

Table 4

| Cpd No. | $R^3$ | A | n | $-N^+R^4R^5R^6$ |
|---|---|---|---|---|
| 62 | $C_{16}H_{33}$ | O | 2 | Thi+ |
| 63 | $C_{18}H_{37}$ | O | 2 | Thi+ |
| 64 | $C_{16}H_{33}$ | O | 3 | Thi+ |
| 65 | $C_{18}H_{37}$ | O | 3 | Thi+ |
| 66 | $C_{14}H_{29}CH(Me)-$ | O | 2 | Thi+ |
| 67 | $Me_3C-(CH_2)_{12}-$ | O | 2 | $-N^+H_3$ |
| 68 | $Me_2CH-(CH_2)_{13}-$ | O | 2 | $-N^+HMe_2$ |
| 69 | $C_{16}H_{33}$ | S | 2 | $-N^+HMe_2$ |
| 70 | $C_{18}H_{37}$ | S | 3 | $-N^+HMe_2$ |
| 71 | $C_{16}H_{33}$ | S | 2 | $-N^+H_3$ |
| 72 | $C_{18}H_{37}$ | S | 2 | $-N^+Me_3$ |
| 73 | $C_{14}H_{29}CH(Me)-$ | O | 5 | Thi+ |
| 74 | $Me_3C-(CH_2)_{12}-$ | O | 7 | $-N^+H_3$ |
| 75 | $Me_2CH-(CH_2)_{13}-$ | O | 9 | $-N^+HMe_2$ |

Table 5

| Cpd No. | $R^3$ | A | n | $-N^+R^4R^5R^6$ |
|---|---|---|---|---|
| 76 | $C_{16}H_{33}$ | O | 2 | Thi+ |
| 77 | $C_{16}H_{33}$ | O | 3 | Thi+ |
| 78 | $C_{17}H_{35}$ | O | 2 | Thi+ |
| 79 | $C_{17}H_{35}$ | O | 3 | Thi+ |
| 80 | $C_{18}H_{37}$ | O | 2 | Thi+ |
| 81 | $C_{18}H_{37}$ | O | 3 | Thi+ |
| 82 | $C_{16}H_{33}$ | O | 2 | $-N^+H_3$ |
| 83 | $C_{17}H_{35}$ | O | 2 | $-N^+Me_3$ |
| 84 | $C_{18}H_{37}$ | O | 2 | $-N^+Me_3$ |
| 85 | $C_{18}H_{37}$ | O | 3 | 1-Me-Pip+ |
| 86 | $C_{18}H_{37}$ | O | 2 | Pyr+ |
| 87 | $C_{17}H_{35}$ | S | 2 | Thi+ |
| 88 | $C_{14}H_{29}CH(Me)-$ | S | 3 | Thi+ |
| 89 | $C_{16}H_{33}$ | S | 2 | Thi+ |
| 90 | $Me_3C-(CH_2)_{12}-$ | S | 3 | Thi+ |
| 91 | $Me_2CH-(CH_2)_{13}-$ | S | 2 | Thi+ |
| 92 | $C_{18}H_{37}$ | S | 2 | Thi+ |
| 93 | $C_{18}H_{37}$ | S | 2 | $-N^+Me_3$ |
| 94 | $C_{18}H_{37}$ | S | 2 | $-N^+HMe_2$ |
| 95 | $C_{18}H_{37}$ | S | 2 | 1-Me-Pip+ |
| 96 | $C_{16}H_{33}$ | S | 2 | Pyr+ |
| 97 | $C_{17}H_{35}$ | O | 6 | Thi+ |
| 98 | $C_{17}H_{35}$ | O | 6 | 4-Me-5-(2-OHEt)Thi+ |
| 99 | $C_{17}H_{35}$ | S | 4 | Thi+ |
| 100 | $C_{17}H_{35}$ | S | 5 | Thi+ |
| 101 | $C_{17}H_{35}$ | S | 6 | Thi+ |
| 102 | $C_{17}H_{35}$ | S | 6 | 4-Me-5-(2-OHEt)Thi+ |
| 103 | $C_{17}H_{35}$ | O | 6 | $-N^+Me_3$ |

Table 6

| Cpd No. | $R^3$ | A | n | $-N^+R^4R^5R^6$ |
|---|---|---|---|---|
| 104 | $C_{14}H_{29}CH(Me)-$ | O | 2 | Thi+ |
| 105 | $C_{17}H_{35}$ | O | 2 | Thi+ |
| 106 | $Me_3C-(CH_2)_{12}-$ | O | 2 | Thi+ |
| 107 | $C_{18}H_{37}$ | O | 3 | Thi+ |
| 108 | $Me_2CH-(CH_2)_{13}-$ | O | 2 | Thi+ |
| 109 | $C_{18}H_{37}$ | O | 2 | $-N^+H_3$ |
| 110 | $C_{18}H_{37}$ | O | 2 | $-N^+Me_3$ |
| 111 | $C_{16}H_{33}$ | O | 2 | $-N^+HMe_2$ |
| 112 | $C_{16}H_{33}$ | O | 2 | 1-Me-Pip+ |
| 113 | $C_{18}H_{37}$ | O | 2 | Pyr+ |
| 114 | $C_{17}H_{35}$ | S | 2 | Thi+ |
| 115 | $C_{18}H_{37}$ | S | 2 | Thi+ |
| 116 | $C_{17}H_{35}$ | S | 3 | $-N^+Me_3$ |
| 117 | $C_{18}H_{37}$ | O | 4 | $-N^+Me_3$ |
| 118 | $C_{16}H_{33}$ | O | 6 | $-N^+HMe_2$ |
| 119 | $C_{16}H_{33}$ | O | 8 | 1-Me-Pip+ |
| 120 | $C_{18}H_{37}$ | O | 10 | Pyr+ |

Table 7

| Cpd No. | $R^3$ | A | n | $-N^+R^4R^5R^6$ |
|---|---|---|---|---|
| 121 | $C_{16}H_{33}$ | O | 2 | Thi+ |
| 122 | $C_{16}H_{33}$ | O | 3 | Thi+ |
| 123 | $C_{17}H_{35}$ | O | 2 | Thi+ |
| 124 | $C_{17}H_{35}$ | O | 3 | Thi+ |
| 125 | $C_{18}H_{37}$ | O | 2 | Thi+ |
| 126 | $C_{18}H_{37}$ | O | 3 | Thi+ |
| 127 | $C_{16}H_{33}$ | O | 2 | $-N^+H_3$ |
| 128 | $C_{16}H_{33}$ | O | 3 | $-N^+Me_3$ |
| 129 | $C_{18}H_{37}$ | O | 2 | $-N^+HMe_2$ |
| 130 | $C_{18}H_{37}$ | O | 3 | 1-Me-Pip+ |
| 131 | $C_{18}H_{37}$ | O | 2 | Pyr+ |
| 132 | $C_{16}H_{33}$ | S | 2 | Thi+ |
| 133 | $C_{14}H_{29}CH(Me)-$ | S | 3 | Thi+ |
| 134 | $C_{18}H_{37}$ | S | 2 | Thi+ |
| 135 | $Me_3C-(CH_2)_{12}-$ | S | 3 | Thi+ |
| 136 | $Me_2CH-(CH_2)_{13}-$ | S | 2 | Thi+ |
| 137 | $C_{16}H_{33}$ | S | 2 | $-N^+H_3$ |
| 138 | $C_{16}H_{33}$ | S | 2 | $-N^+Me_3$ |
| 139 | $C_{18}H_{37}$ | S | 2 | $-N^+HMe_2$ |
| 140 | $C_{18}H_{37}$ | S | 2 | 1-Me-Pip+ |
| 141 | $C_{16}H_{33}$ | S | 2 | Pyr+ |
| 142 | $C_{18}H_{37}$ | O | 4 | 1-Me-Pip+ |
| 143 | $C_{18}H_{37}$ | O | 5 | Pyr+ |
| 144 | $C_{16}H_{33}$ | S | 6 | $-N^+H_3$ |
| 145 | $C_{18}H_{37}$ | S | 7 | $-N^+HMe_2$ |
| 146 | $C_{18}H_{37}$ | S | 8 | 1-Me-Pip+ |

Table 8

| Cpd No. | $R^3$ | A | n | $-N^+R^4R^5R^6$ |
|---|---|---|---|---|
| 147 | $C_{14}H_{29}CH(Me)-$ | O | 2 | Thi+ |
| 148 | $C_{17}H_{35}$ | O | 3 | Thi+ |
| 149 | $Me_3C-(CH_2)_{12}$ | O | 2 | Thi+ |
| 150 | $C_{18}H_{37}$ | O | 3 | Thi+ |
| 151 | $Me_2CH-(CH_2)_{13}-$ | O | 2 | Thi+ |
| 152 | $C_{18}H_{37}$ | O | 2 | $-N^+H_3$ |
| 153 | $C_{18}H_{37}$ | O | 2 | $-N^+Me_3$ |
| 154 | $C_{16}H_{33}$ | O | 2 | $-N^+HMe_2$ |
| 155 | $C_{16}H_{33}$ | O | 2 | 1-Me-Pip+ |
| 156 | $C_{18}H_{37}$ | O | 2 | Pyr+ |
| 157 | $C_{17}H_{35}$ | S | 2 | Thi+ |
| 158 | $C_{18}H_{37}$ | S | 3 | Thi+ |
| 159 | $C_{17}H_{35}$ | S | 2 | $-N^+Me_3$ |
| 160 | $C_{14}H_{29}CH(Me)-$ | O | 4 | Thi+ |
| 161 | $Me_3C-(CH_2)_{12}-$ | O | 5 | Thi+ |
| 162 | $Me_2CH-(CH_2)_{13}-$ | O | 6 | Thi+ |
| 163 | $C_{18}H_{37}$ | O | 7 | $-N^+H_3$ |

Table 9

| Cpd No. | $R^3$ | A | n | $-N+R^4R^5R^6$ |
|---|---|---|---|---|
| 164 | $C_{17}H_{35}$ | O | 2 | Thi+ |
| 165 | $C_{18}H_{37}$ | O | 3 | Thi+ |
| 166 | $C_{17}H_{35}$ | O | 2 | $-N+Me_3$ |
| 167 | $C_{17}H_{35}$ | S | 2 | Thi+ |
| 168 | $C_{18}H_{37}$ | S | 3 | Thi+ |
| 169 | $C_{17}H_{35}$ | S | 2 | $-N+HMe_2$ |
| 170 | $C_{17}H_{35}$ | O | 6 | Thi+ |

Table 10

| Cpd No. | $R^3$ | A | n | $-N+R^4R^5R^6$ |
|---|---|---|---|---|
| 171 | $C_{17}H_{35}$ | O | 2 | Thi+ |
| 172 | $C_{18}H_{37}$ | O | 2 | $-N+Me_3$ |
| 173 | $C_{17}H_{35}$ | S | 2 | Thi+ |
| 174 | $C_{18}H_{37}$ | S | 2 | $-N+Me_3$ |

Table 11

| Cpd No. | $R^3$ | A | n | $-N+R^4R^5R^6$ |
|---|---|---|---|---|
| 175 | $C_{17}H_{35}$ | O | 2 | Thi+ |
| 176 | $C_{18}H_{37}$ | O | 2 | $-N+Me_3$ |
| 177 | $C_{18}H_{37}$ | S | 2 | Thi+ |
| 178 | $C_{17}H_{35}$ | S | 2 | $-N+Me_3$ |

Table 12

| Cpd No. | $R^3$ | A | n | $-N+R^4R^5R^6$ |
|---|---|---|---|---|
| 179 | $C_{17}H_{35}$ | O | 2 | Thi+ |
| 180 | $C_{18}H_{37}$ | O | 2 | $-N+Me_3$ |
| 181 | $C_{17}H_{35}$ | S | 2 | Thi+ |
| 182 | $C_{18}H_{37}$ | S | 2 | $-N+Me_3$ |

Of the compounds listed above, preferred compounds are compounds No. 1, 2, 3, 4, 9, 19, 26, 36, 76, 77, 78, 80, 83, 85, 86, 87, 97, 98, 99 100, 101, 102, 103, 105, 165, 166, 167 and 170.

Because of the presence of asymmetric carbon atoms at the α- and β-positions (relative to the ether oxygen atom in the ether ring), the compounds of the invention can exist, as explained previously, as four different stereoisomers. Specific preferred compounds are those having the following formulae (a) - (y) and their mirror images:

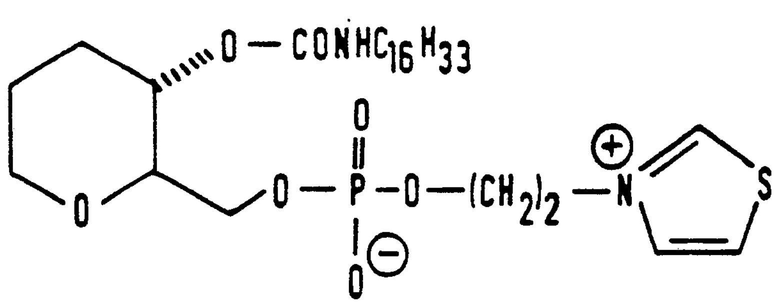

(a)

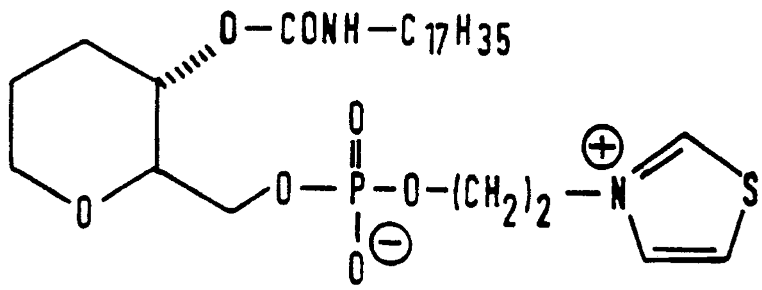

(b)

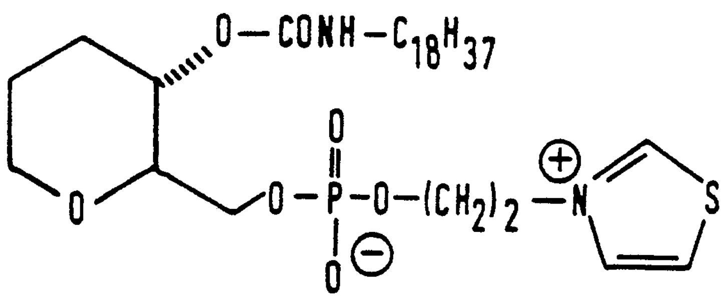

(c)

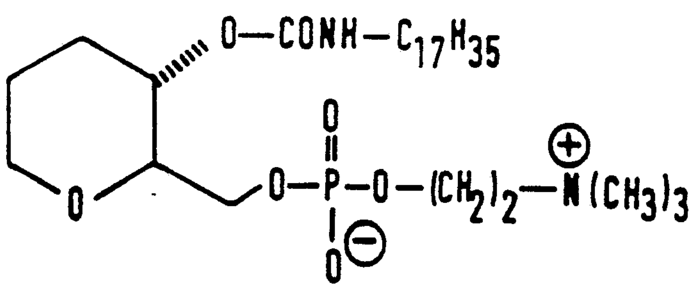

(d)

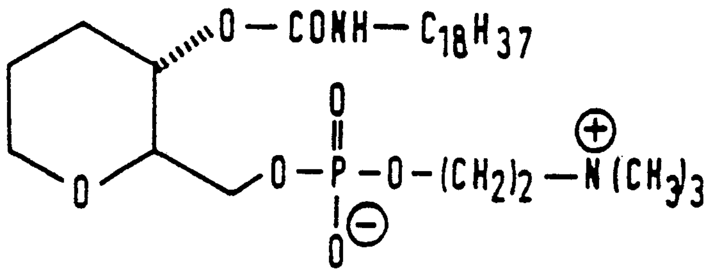

(e)

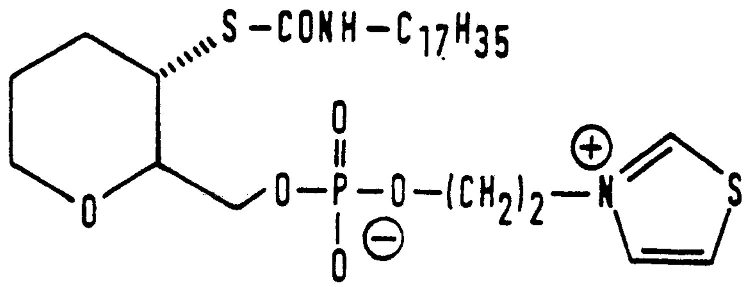

(f)

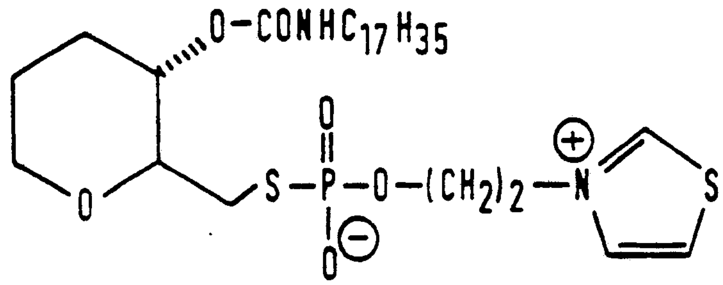

(g)

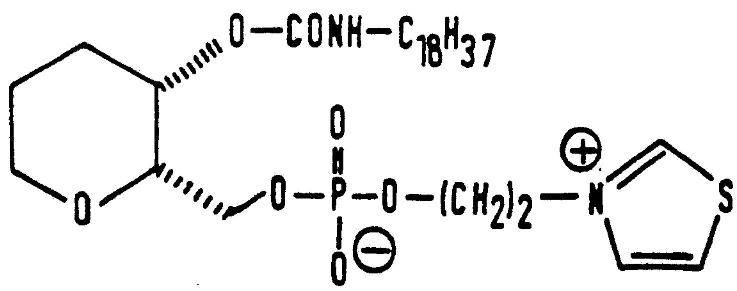

(h)

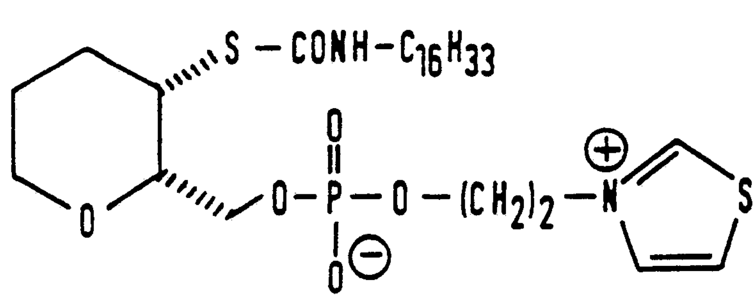

(i)

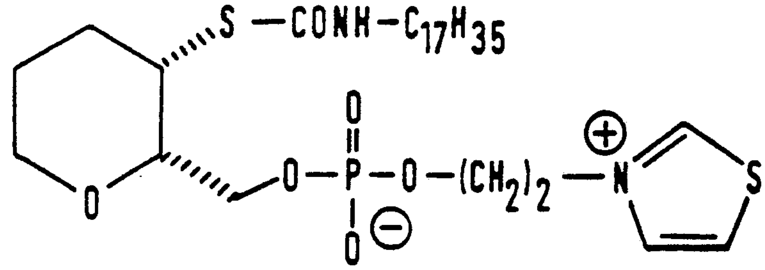

(j)

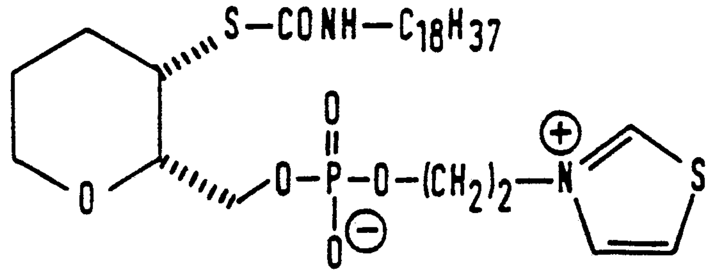

(k)

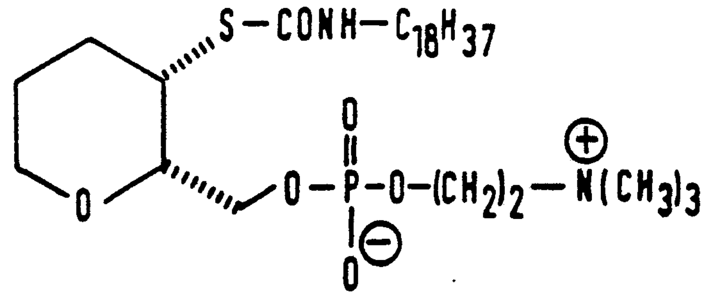

(l)

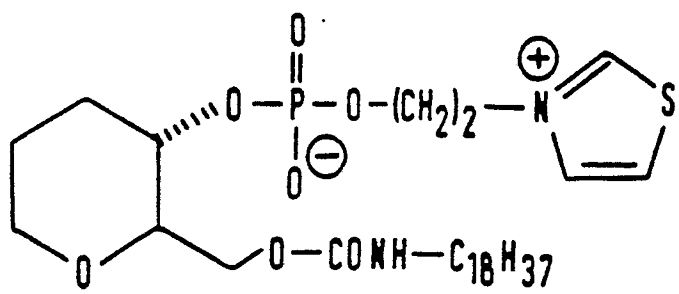

(m)

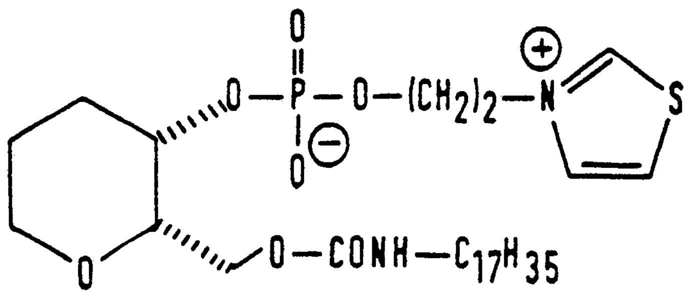

(n)

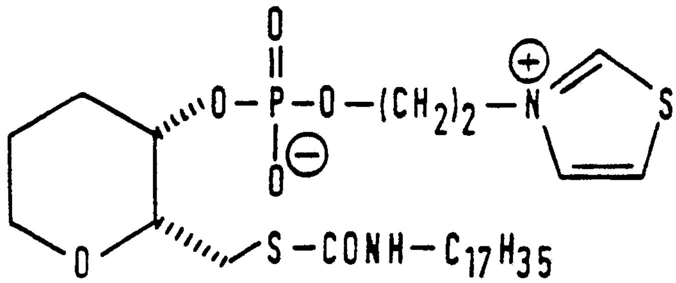

(o)

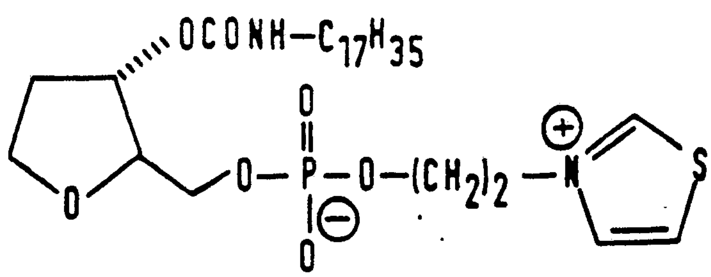

(p)

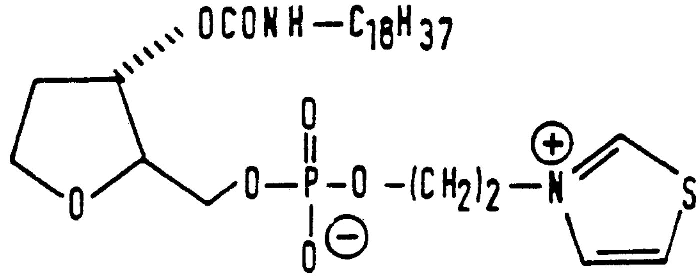

(q)

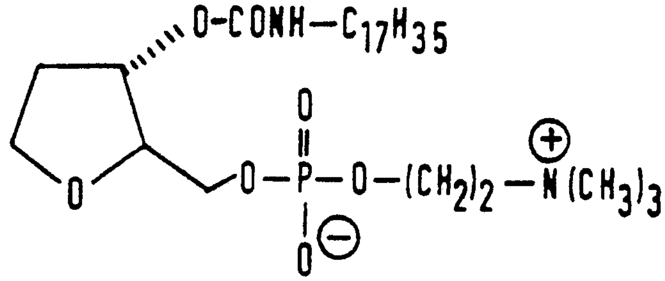

(r)

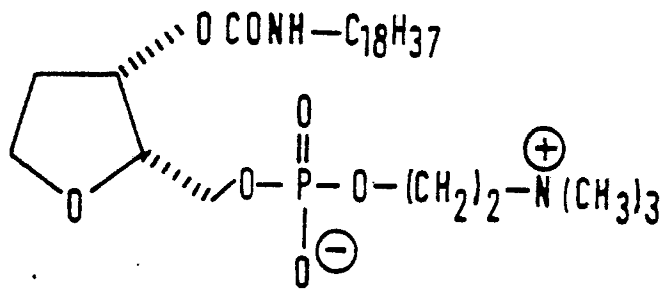

(s)

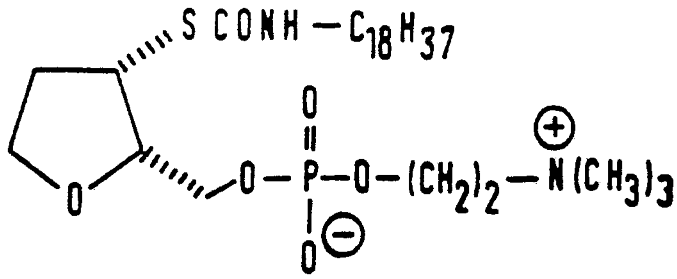

(t)

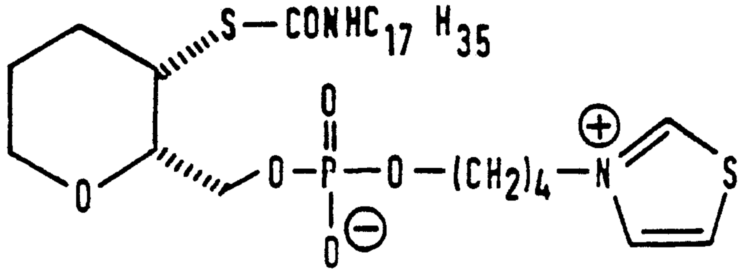

(u)

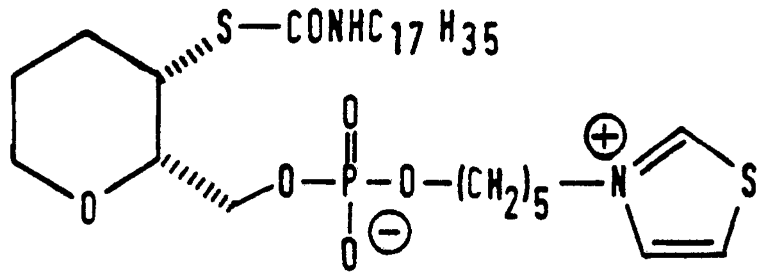

(v)

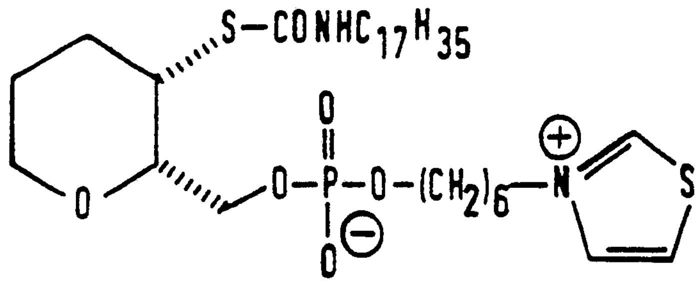

(w)

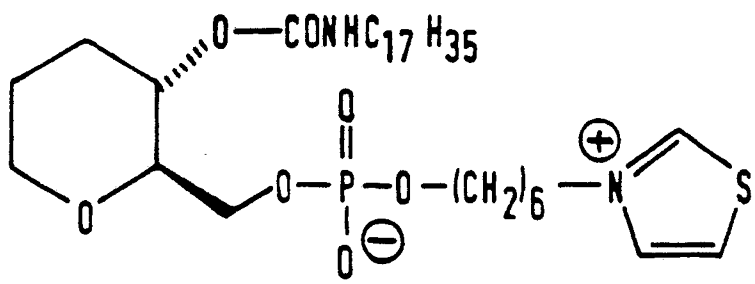

(x)

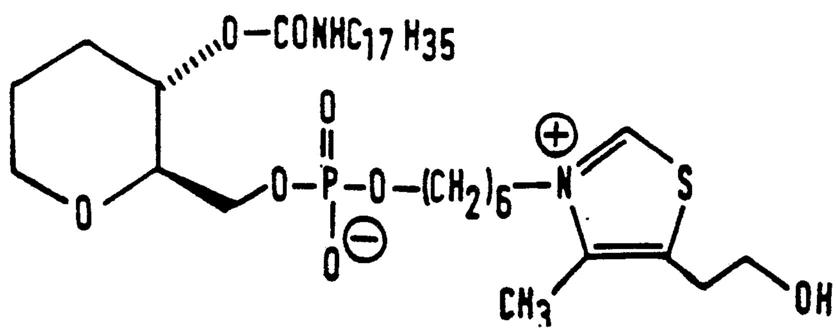

(y)

Of the compounds shown above, compounds (b), (c), (d), (g), (j), (k), (p), (u), (v), (w), (x) and (y) and their mirror images (b'), (c'), (d'), (g'), (j'), (k'), (p'), (u'), (v'), (w'), (x') and (y') are particularly preferred:

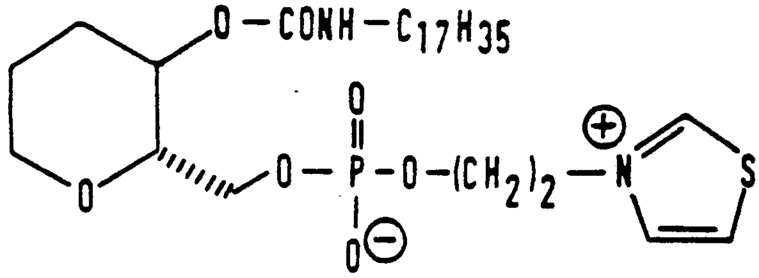

(b')

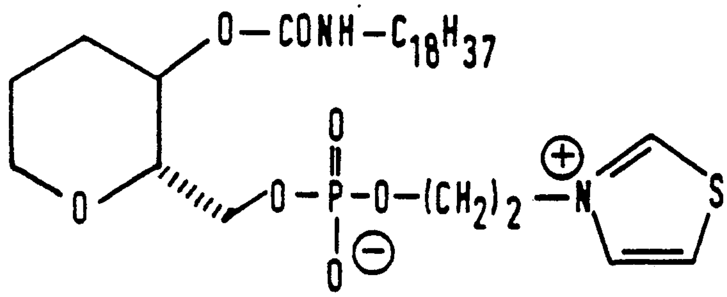

(c')

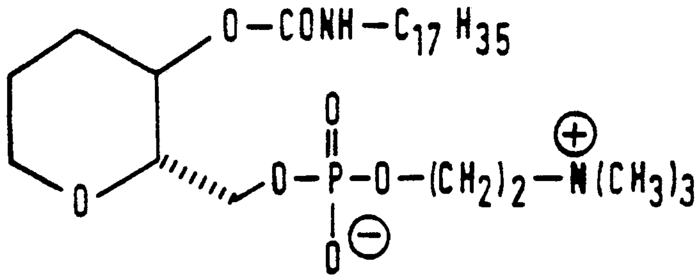

(d')

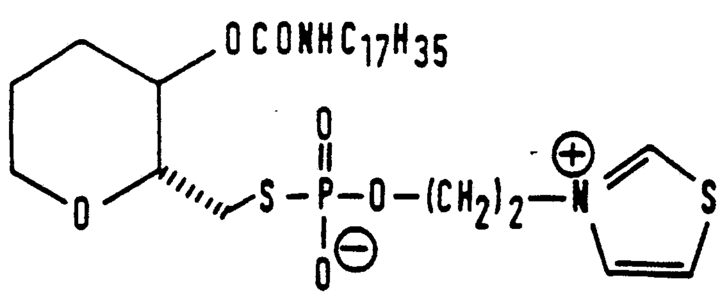

(g')

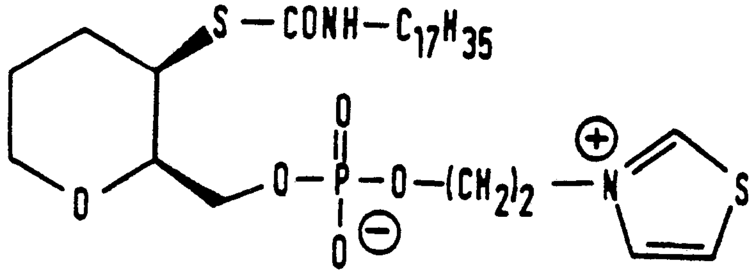

(j')

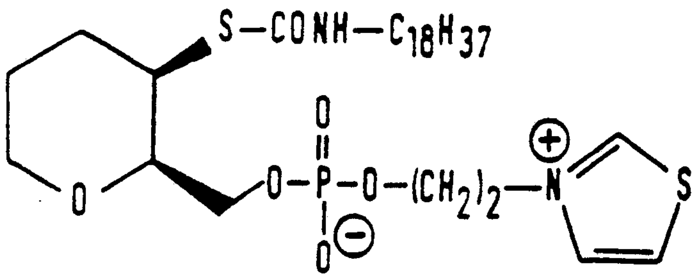

(k')

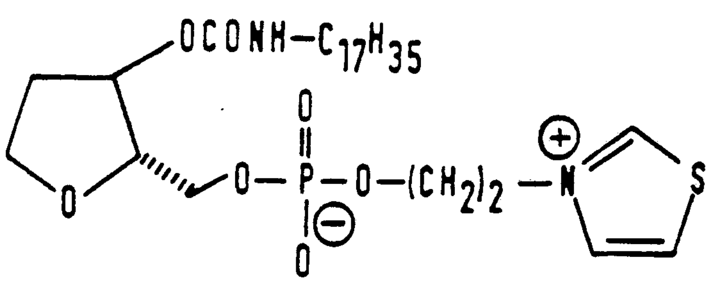

(p')

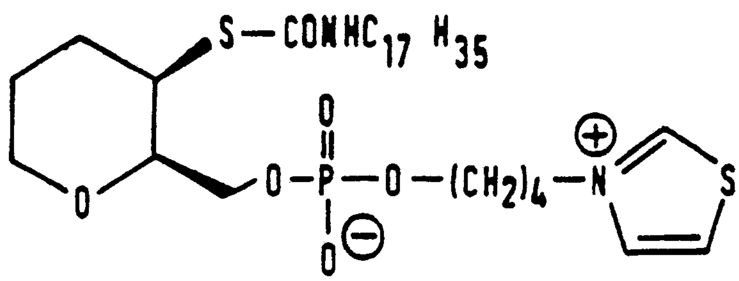

(u')

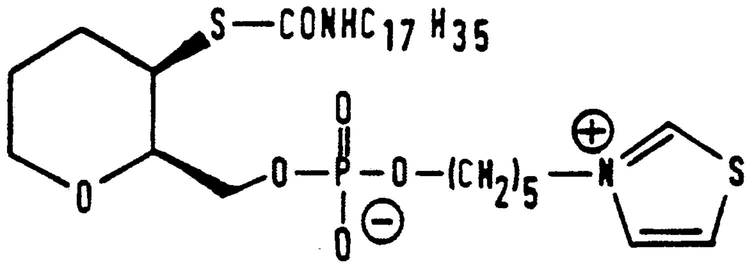

(v')

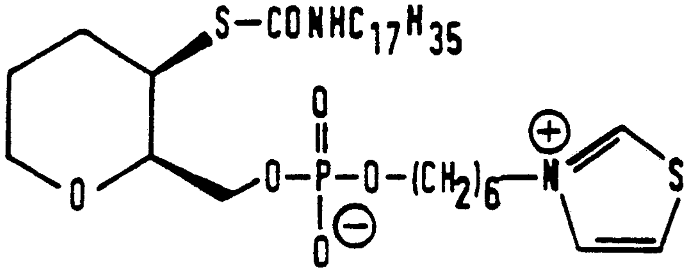

(w')

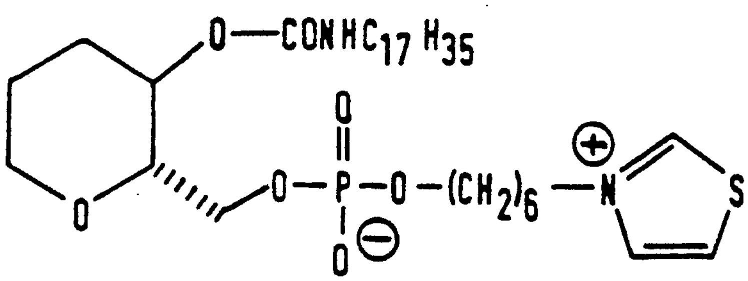

(x')

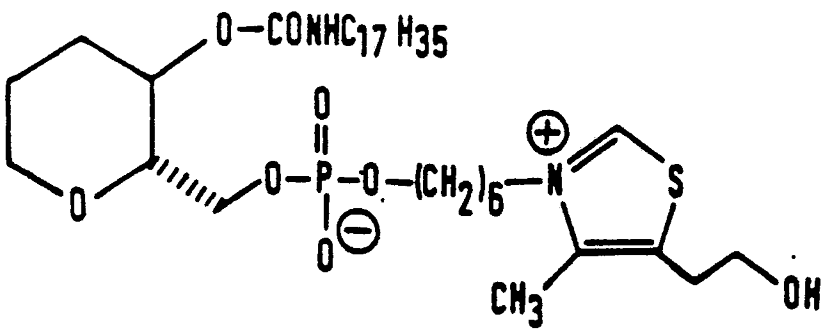

(y')

The compounds of the present invention may be prepared as illustrated by the following reaction scheme:

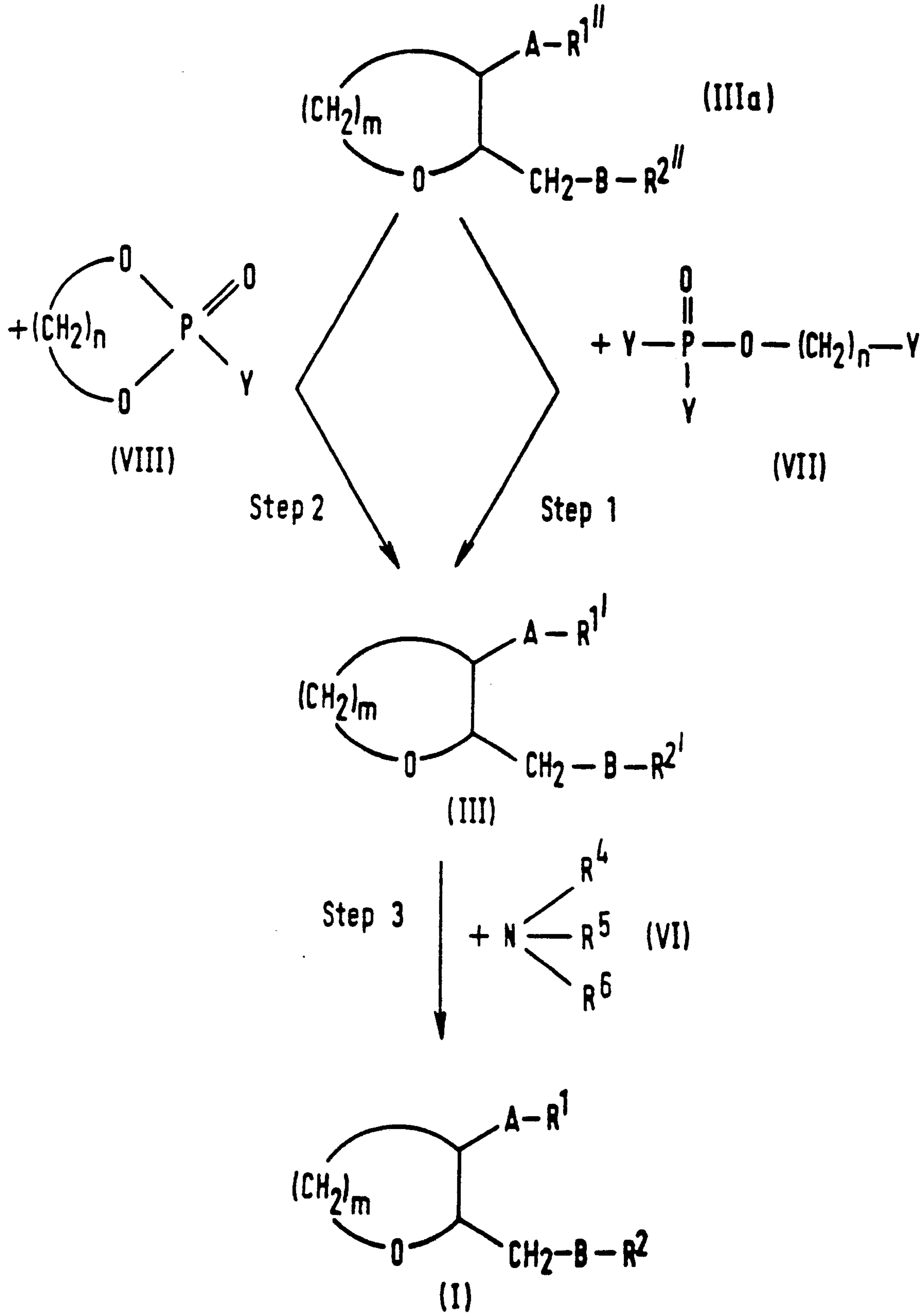


(IIIa)

(VIII)

(VII)

(III)

(VI)

(I)

In the above formulae, $R^1$, $R^2$, $R^4$, $R^5$, $R^6$, $R^{1'}$, $R^{2'}$, A, B, Y, m and n are as defined above. One of $R^{1''}$ and $R^{2''}$ represents a group of formula -CONH-$R^3$ (the same as $R^1$ or $R^2$) and the other represents a hydrogen atom. In the case of the compound of formula (VII), the three halogen atoms represented by Y may be the same or

different. The nature of the halogen atom represented by Y is not particularly critical, since these halogen atoms are eliminated during the reactions of Steps 1 and 2; suitable halogen atoms include the chlorine, bromine and iodine atoms.

Steps 1 and 2 of this reaction scheme are alternatives. In step 1, the hydroxy or mercapto group, -AH or -BH, of the cyclic ether of formula (IIIa) is reacted with a haloalkyl phosphorodihalidate (preferably phosphorodichloridate) of formula (VII) and the product of this reaction is then treated with water, to give the compound of formula (III) in which $R^{1'}$ or $R^{2'}$ represents the aforementioned group of formula (IV), alternatively, in step 2, the compound of formula (IIIa) is reacted with the cyclic phosphoryl halide, preferably chloride, of formula (VIII), to give the compound of formula (III) in which $R^{1'}$ or $R^{2'}$ represents the aforementioned group of formula (V).

Both the reaction with the compound (VII) (Step 1) and that with the compound (VIII) (Step 2) are preferably effected in the presence of a solvent, the nature of which is not critical, provided that it does not adversely affect the reaction and that it can dissolve the reagents, at least to some degree, preferred solvents are: halogenated hydrocarbons, preferably halogenated aliphatic hydrocarbons, such as methylene chloride, chloroform or 1,2-dichloroethane; ethers, such as diethyl ether, tetrahydrofuran or dioxane; and aromatic hydrocarbons, such as benzene or toluene.

Both reactions are preferably also effected in the presence of a base, the nature of which is not particularly critical, provided that it does not adversely affect the reagents. Suitable bases include amines, particularly triethylamine, diethylamine or pyridine.

Both reactions will take place over a wide range of temperatures, and the specific temperature chosen is not particularly critical. In the case of the reaction involving the haloalkyl phosphorodihalidate of formula (VII), the preferred temperature is within the range from 0 to 120°C. In the case of the reaction involving the cyclic phosphoryl halide of formula (VIII), the preferred temperature is within the range of from 20 to 120°C.

The time required for the reaction will vary, depending upon many factors, including the nature of the reagents, solvent and base employed, as well as the reaction temperature. However, within the reaction temperatures indicated above, both reactions will normally be complete within a period of from 2 to 24 hours.

The treatment with water in Step 1 may be effected at any convenient temperature, e. g. room temperature or the temperature employed for the reaction with the haloalkyl phosphorodihalidate (VII) or any temperature in between. It is conveniently effected simply by adding water to the reaction mixture.

After completion of the reaction, the resulting compound of formula (III) can be isolated from the reaction mixture by conventional means. For example, the solution containing the desired product may be concentrated by evaporating off the solvent under reduced pressure and then the residue purified by such conventional techniques as the chromatography techniques, particularly silica gel column chromatography, or recrystallization.

In Step 3 of the reaction scheme, the ω-haloalkyl phosphate of formula (III) [$R^{1'}$ or $R^{2'}$ represents the aforementioned group of formula (IV)] or cyclic phosphate of formula (III) [$R^{1'}$ or $R^{2'}$ represents the aforementioned group of formula (V)] is reacted with the amine of formula (VI) in a suitable solvent to give the desired compound of formula (I), which may be in the form of an inner salt.

There is no particular limitation on the nature of the solvent to be employed, provided that it has no adverse effect upon the reaction and that it can dissolve the reagents, at least to some degree. Suitable solvents include: aromatic hydrocarbons, such as benzene, toluene or xylene; halogenated hydrocarbons, preferably halogenated aliphatic hydrocarbons, such as methylene chloride or chloroform; lower alkanols, such as methanol, ethanol or isopropanol; amides, such as dimethylformamide; ethers, such as diethyl ether, tetrahydrofuran or dioxane; nitriles, such as acetonitrile; and water. A single one of these solvents or a mixture of any two or more, e. g. two or three, thereof may be employed. For example, a suitable mixture might be chloroform, dimethylformamide and isopropanolin an appropriate ratio, e. g. a volume ratio of about 3 : 5 : 5.

The temperature at which the reaction is carried out is not particularly critical, although we generally prefer to carry out the reaction at a temperature of from 20 to 80°C. The reaction is preferably effected in a nitrogen atmosphere and may be carried out in a conventional reaction vessel or preferably in a tightly sealed container (e. g. a sealed tube). The time required for the reaction will vary over a wide range, depending upon the reagents and solvent employed and the reaction temperature, but a period of from 1 hour to 6 days will normally suffice.

After completion of the reaction, the product of this reaction, which may be the compound of formula (I) itself (i. e. the inner salt) or may be a salt thereof, may be isolated from the reaction mixture by conventional means. For example, a suitable recovery technique would comprise concentrating the reaction mixture by evaporating off the solvent under reduced pressure and then purifying the residue by conventional techniques, such as the various chromatography techniques and especially silica gel column chromatography.

In the case of the reaction involving the cyclic phosphate of formula (III) [$R^{1'}$ or $R^{2'}$ represents the aforementioned group of formula (V)], the inner salt of formula (I) will normally be obtained directly. Similarly, in the case of the reaction involving the ω-haloalkyl phosphate of formula (III) [$R^{1'}$ or $R^{2'}$ represents the aforementioned group of formula (IV)] and where the amine of formula (VI) is pyridine, the inner salt will normally be obtained directly.

However, in other cases involving the ω-haloalkyl phosphate of formula (III) [$R^{1'}$ or $R^{2'}$ represents the aforementioned group of formula (IV)], the product of Step 3 will normally be a salt of the compound of formula (I), i. e. $R^1$ or $R^2$ will represent a group of formula (IIa). In this case, if the inner salt itself is desired, this

may be produced by treating its salt with an ion-exchange resin (for example resin MB-3, produced by Rohm and Haas Co.) or with a silver salt (for example silver carbonate or silver acetate).

If desired, the compound of formula (I) or salt thereof may be converted to a salt of any other ion by known methods.

The cyclic ether compounds of formula (IIIa) used as starting materials in the above sequence of reactions have previously been described in European Patent Application No. 86 300 278.8 and can be prepared from compounds of formula (IX), shown in the following reaction schemes, (specifically, 3,4-dihydro-2H-pyran, dihydrofuran or 6,7-dihydrooxepine) they can be prepared stereoselectively (with respect to the two asymmetric carbon atoms identified above) by any of the reaction schemes shown below:

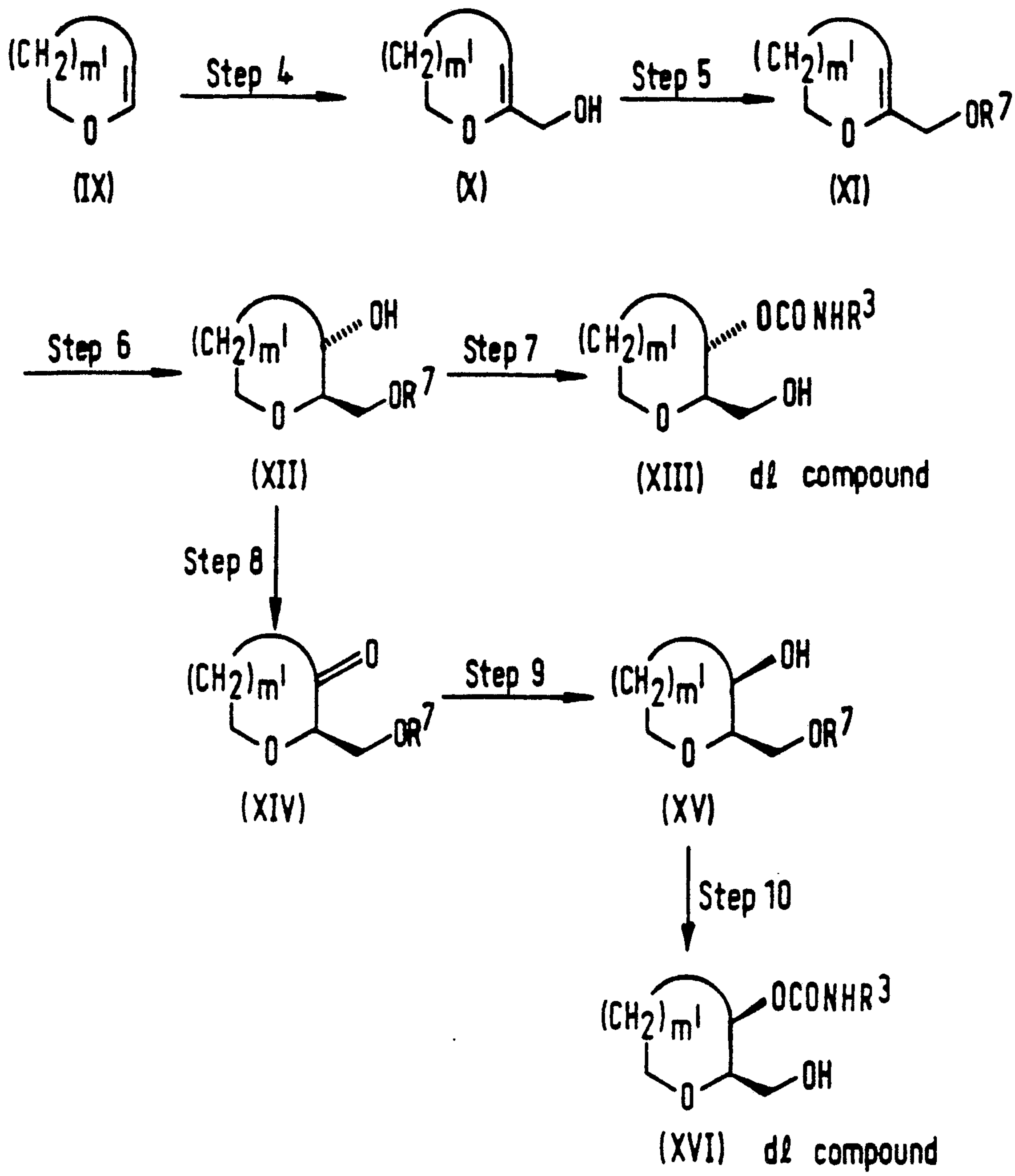


(IX) (X) (XI)

(XII) (XIII) dl compound

(XIV) (XV)

(XVI) dl compound

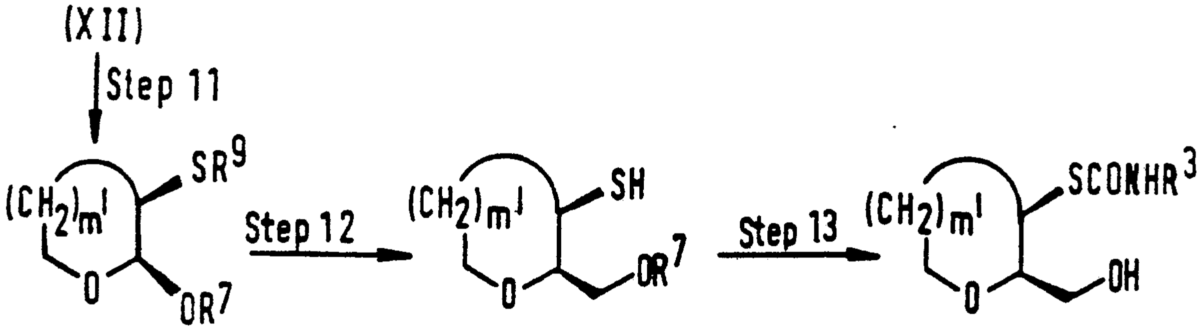


(XVII) (XVIII) (XIX) dℓ compound

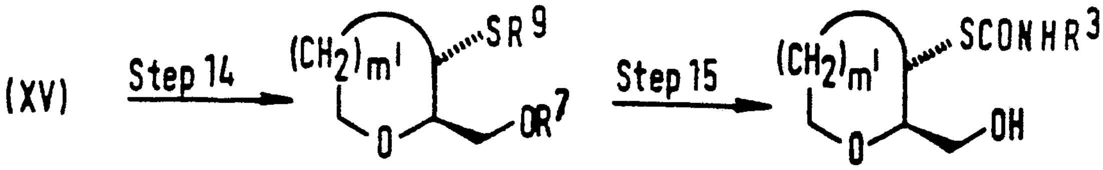


(XX) (XXI) dℓ compound

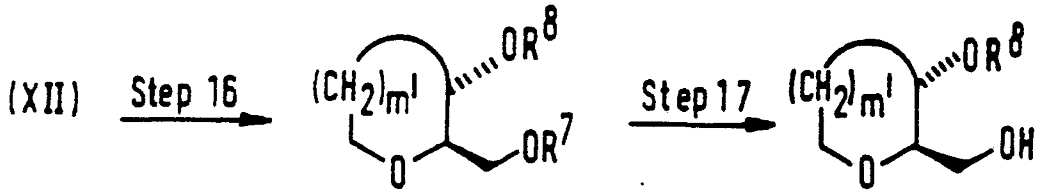


(XXII) (XXIII)

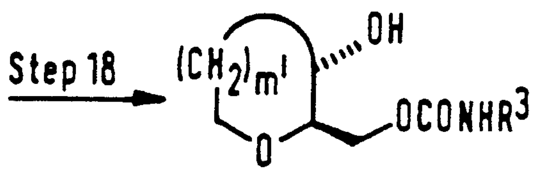


(XXIV) dℓ compound

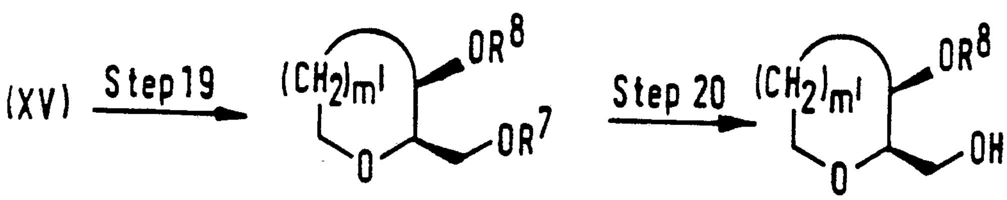


(XXV) (XXVI)

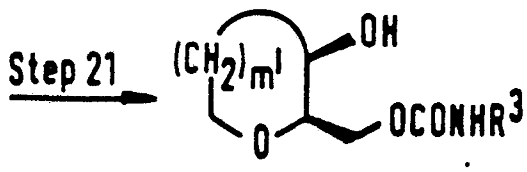


(XXVII) dℓ compound

(XVII)

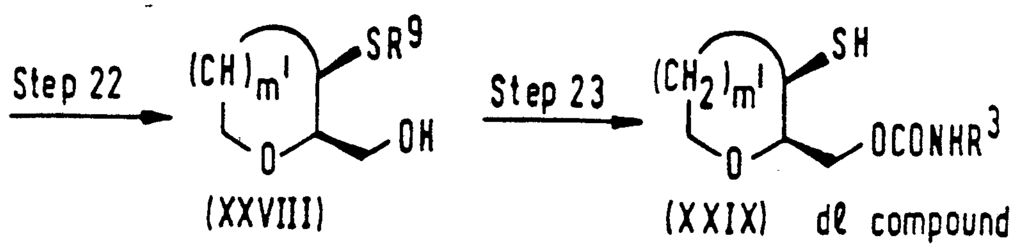


(XXVIII)

(XXIX) dℓ compound

(XX)

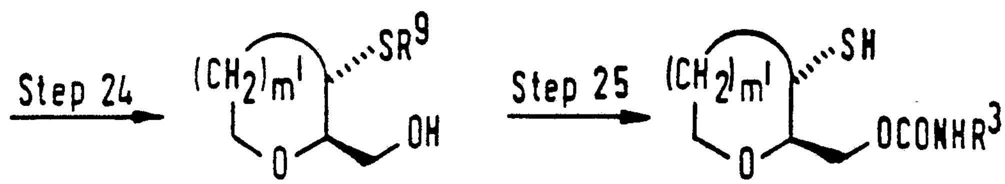


(XXX)

(XXXI)

(XXIII)

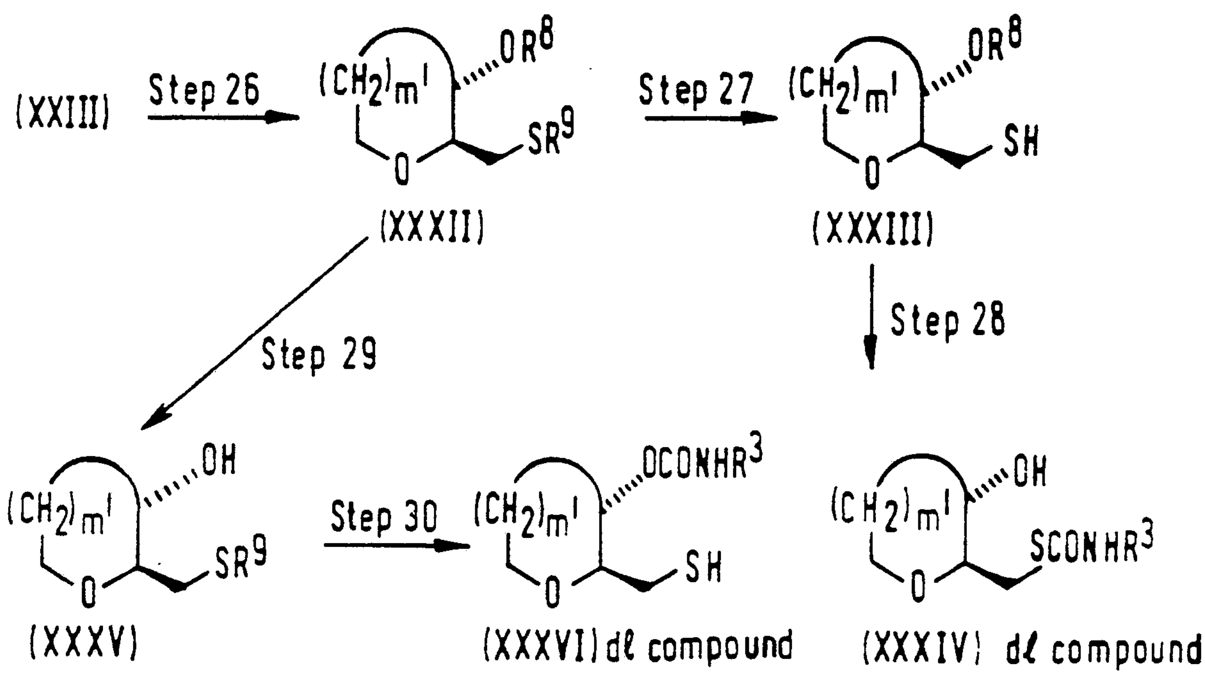


(XXXII)

(XXXIII)

(XXXV)

(XXXVI) dℓ compound

(XXXIV) dℓ compound

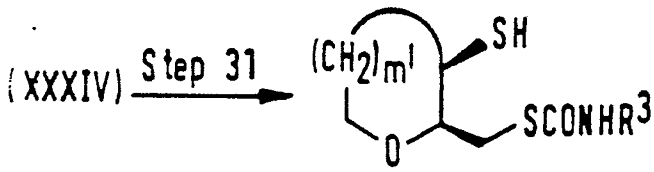


(XXXVII) dl compound

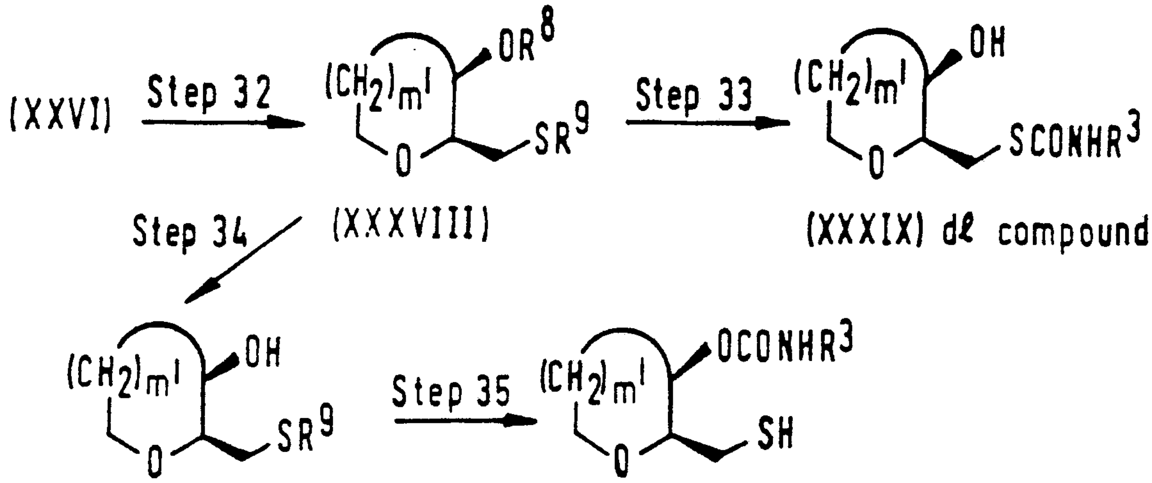


(XL) (XLI) dl compound

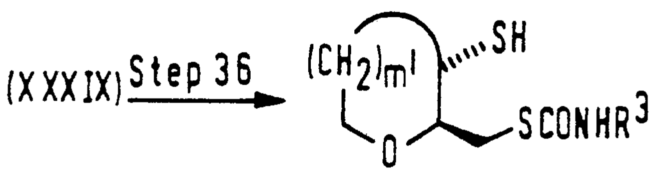


(XLII) dl compound

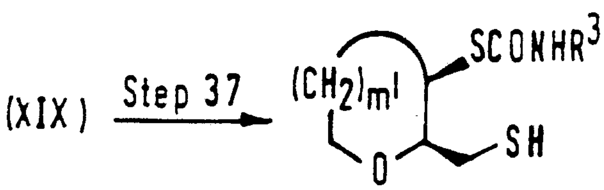


(XLIII) dl compound

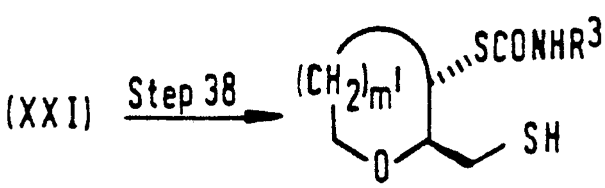


(XLIV) dl compound

In these formulae, $R^3$ is as defined above and m' = (m-1).

$R^7$ and $R^8$ are both hydroxy-protecting groups, whilst $R^9$ represents a mercapto-protecting group. Where $R^7$ and $R^8$ $R^8$ $R^9$ occur within the same compound, the protecting groups are preferably selected from different classes, so that one protecting group may be removed preferentially, without removing the other.

Examples of classes of hydroxy-protecting groups which may be employed include: di- and tri-arylmethyl groups, for example the diphenylmethyl ( = benzhydryl), triphenylmethyl, α-naphthyldiphenylmethyl, p-methoxyphenyldiphenylmethyl and p-(p-bromophenacyloxy)phenyldiphenylmethyl groups; optionally substituted tetrahydropyranyl groups, such as the tetrahydropyran-2-yl, 3-bromotetrahydropyran-2-yl, 4-methoxytetrahydropyran-4-yl, tetrahydrothiopyran-2-yl and 4-methoxytetrahydrothiopyran-2-yl groups; trialkylsilyl groups, in which each alkyl part is preferably $C_1$-$C_4$, for example the trimethylsilyl, triethylsilyl, isopropyldimethylsilyl, t-butyldimethylsilyl, methyldiisopropylsilyl, methyldi-t-butylsilyl and triisopropylsilyl groups; and optionally substituted benzyl groups, for example the benzyl, p-methoxybenzyl, o-nitrobenzyl, p-nitrobenzyl, p-halobenzyl (e. g. p-chlorobenzyl or p-bromobenzyl) and p-cyanobenzyl groups.

Preferred examples of mercaptoprotecting groups which may be represented by $R^9$ include the aforementioned benzyl groups and di- and tri-arylmethyl groups; also included amongst the preferred mercaptoprotecting groups are lower (e. g. $C_2$-$C_5$) aliphatic acyl groups, such as the acetyl group.

We particularly prefer that $R^7$ should be one of the aforementioned optionally substituted benzyl groups, $R^8$ should be one of the aforementioned optionally substituted tetrahydropyranyl groups and $R^9$ should be one of the lower aliphatic acyl groups. More preferably, $R^7$ is a benzyl group, $R^8$ is a tetrahydropyran-2-yl group and $R^9$ is an acetyl group.

Since many of the reactions involved in the above reaction schemes are repeated, they may be summarized as follows:

Reaction 1

In this reaction, the compound of formula (IX) is subjected to hydroxymethylation, to prepare the compound of formula (X) by the method of A. Lebouc et al. [A. Lebouc et al., Synthesis, 610 (1979)].

Reaction 2

In this reaction, the hydroxy group of the compound of formula (X) may be protected with any one of the appropriate protecting groups mentioned above to prepare the compound of formula (XI). This reaction may be carried out by conventional means for introduction of protecting groups, preferably benzylation using a benzyl halide.

Reaction 3

In this reaction, the starting material, for example the compound of formula (XI), is subjected to a hydroboration reaction to introduce a trans-hydroxy group onto the double bond and give, for example, the compound of formula (XII). By employing an optically active borane, such as isopinocamphenylborane, in this hydroboration reaction, it is possible to prepare an optically active trans-hydroxy derivative. Otherwise, borane itself, preferably employed as a complex with an organic solvent (e. g. borane-tetrahydrofuran), may conveniently be used.

Reaction 4

In this reaction, a hydroxy group, for example that of the compound of formula (XII) or (XV), or a mercapto group, for example that of the compound of formula (XVIII) or, after deprotection, (XX), is carbamoylated using an alkyl isocyanate in which the alkyl group corresponds to the group $R^3$ to be introduced, for example heptadecyl isocyanate. The isocyanate employed may be prepared by reacting a carboxylic acid with diphenylphosphoryl azide and then heating the reaction mixture. The resulting isocyanate may then be reacted, with or without isolation, with the appropriate hydroxy or mercapto compound.

Reaction 5

In this reaction, a hydroxy-protecting group is removed, for example the hydroxy-protecting group is removed from the compound of formula (XII) after carbamoylation. The nature of the removal reaction depends upon the particular class of hydroxy-protecting group involved and the nature of such reactions is well-known to those skilled in the art. For example, if the hydroxy-protecting group is one of the aforementioned di- or tri-arylmethyl groups, it may be removed by treatment with an acid such as trifluoroacetic acid, acetic acid or hydrochloric acid. If the hydroxyprotecting group is one of the aforementioned optionally substituted benzyl groups, it may be removed by reduction, preferably catalytic reduction, using palladium-on-carbon as the catalyst. If the hydroxyprotecting group is a trialkylsilyl group, it may be removed by treatment with a compound generating fluoride anions, preferably tetrabutylammonium fluoride. If the hydroxy-protecting group is one of the aforementioned optionally substituted tetrahydropyranyl groups, it may be removed by treatment with an acid, for example acetic acid or p-toluenesulphonic acid.

As is well known, optionally substituted benzyl groups which protect a hydroxy group are usually removed by reduction as described above. However, in the presence of a mercapto or protected mercapto group in the same molecule, the reducing agents used for deprotecting these protected hydroxy groups become rather inactive, adversely affected by the mercapto or protected mercapto group. Consequently, where the compound contains both a mercapto-protecting group (preferably an acyl group, such as an acetyl group) and a hydroxy-protecting group and it is desired to remove the hydroxy-protecting group, then reaction with aluminium chloride and sodium or potassium iodide is employed if the hydroxy-protecting group is one of the optionally substituted benzyl groups, whilst the above-mentioned reaction with an acid is employed if the hydroxy-protecting group is one of the di- or tri-arylmethyl groups.

Reaction 6

In this reaction, the hydroxy group of the compound of formula (XII) is oxidized to a carbonyl group by means of Jones' reagent, using chromic acid, or pyridinium chlorochromate, to prepare the compound of formula (XIV).

Reaction 7

In this reaction, the carbonyl group of the compound of formula (XIV) is reduced stereoselectively, using L-selectride, to form a cis-hydroxy group and give the compound of formula (XV).

Reaction 8

In this reaction, a hydroxy group, for example a hydroxy group of the compound of formula (XII), can be acylated to introduce an acyl group, for example a methanesulphonyl p-toluenesulphonyl, trifluoromethanesulphonyl or trifluoroacetyl group, to form an ester, and then the resulting acyloxy group can be converted to a protected mercapto group whose configuration is inverted, compared to the original hydroxy group, using, for example thioacetic acid. This reaction may be employed in a number of steps in the above reaction schemes.

Reaction 9

In this reaction a free hydroxy group, of a compound already containing one protected hydroxy group, for example the free hydroxy group of the compound of formula (XII), may be protected by a different hydroxyprotecting group in order to prepare the compound of formula (XXII). In this case, the preferred protecting group is one selected from the class of optionally substituted tetrahydropyranyl groups.

Reaction 10

In this reaction, the mercapto-protecting group $R^9$, for example of the compound of formula (XVII), is removed to give a free mercapto group, for example as in the compound of formula (XVIII). The mercapto-protecting group, preferably acetyl group, may be removed by treatment with a base, such as methanolic sodium methoxide, aqueous ammonia, aqueous sodium hydroxide or aqueous potassium hydroxide, preferably a 10 - 30 % w/w methanolic solution of sodium methoxide.

The following Table 13 shows the reactions which may be employed for each of the steps of the reaction schemes given above.

Table 7

| Step | Reaction used | Step | Reaction used | Step | Reaction used |
|---|---|---|---|---|---|
| 4 | 1 | 17 | 5 | 30 | 4,10 |
| 5 | 2 | 18 | 4,5 | 31 | 8,10 |
| 6 | 3 | 19 | 9 | 32 | 8 |
| 7 | 4,5 | 20 | 5 | 33 | 10,4,5 |
| 8 | 6 | 21 | 4,5 | 34 | 5 |
| 9 | 7 | 22 | 5 | 35 | 4,10 |
| 10 | 4,5 | 23 | 4,10 | 36 | 8,10 |
| 11 | 8 | 24 | 5 | 37 | 8,10 |
| 12 | 10 | 25 | 4,10 | 38 | 8,10 |
| 13 | 4,5 | 26 | 8 | | |
| 14 | 8+ | 27 | 10 | | |
| 15 | 10,4,5 | 28 | 4,5 | | |
| 16 | 9 | 29 | 5 | | |

Where it is desired to employ an optically active compound as the starting material of formula (IIIa), this may be prepared, for example, in the case where m is 3 as illustrated by the following reaction scheme:

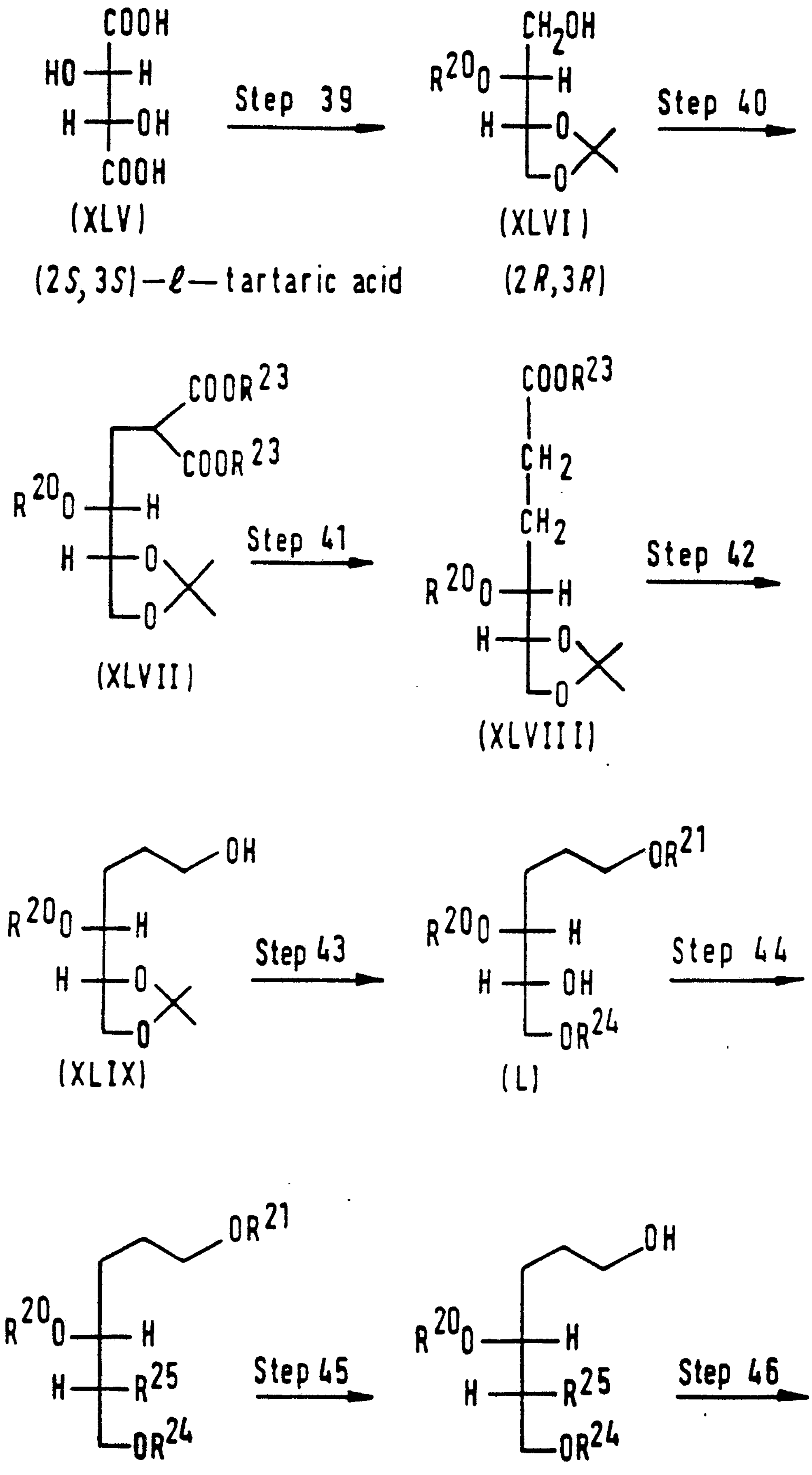


(XLV) (2*S*, 3*S*)–ℓ–tartaric acid

(XLVI) (2*R*,3*R*)

(XLVII)

(XLVIII)

(XLIX)

(L)

(LI)

(LII)

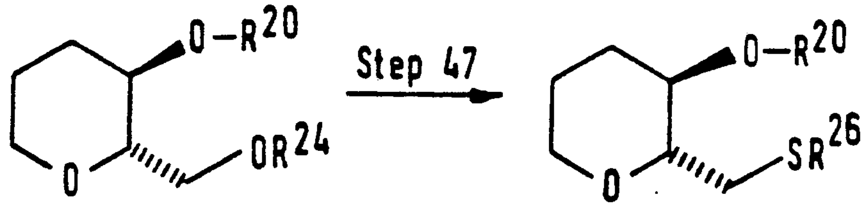


(LIII) (LIV)

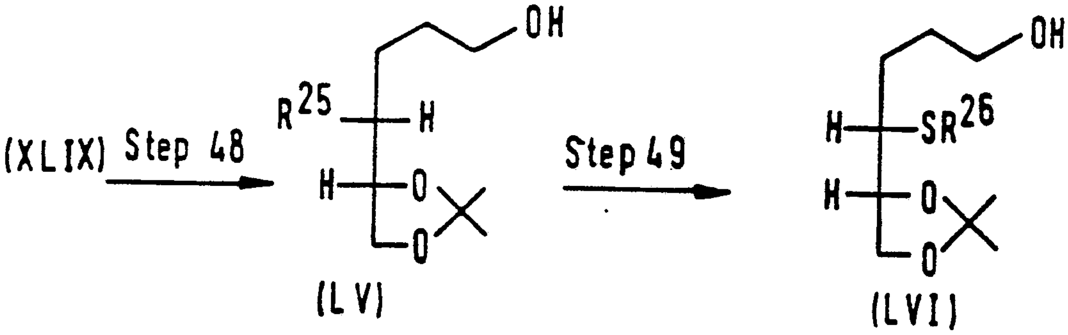


(XLIX) (LV) (LVI)

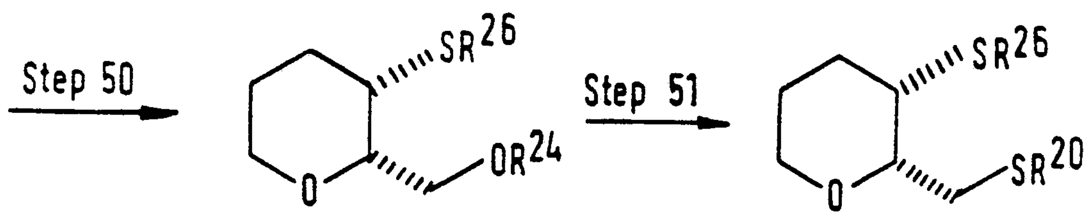


(LVII) (LVIII)

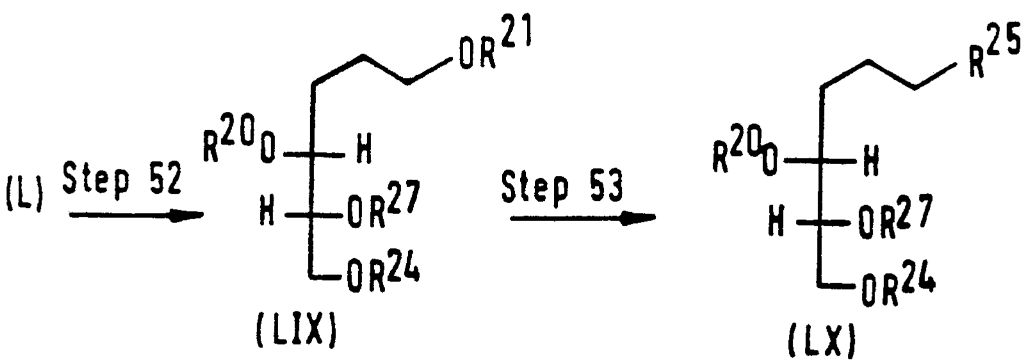


(L) (LIX) (LX)

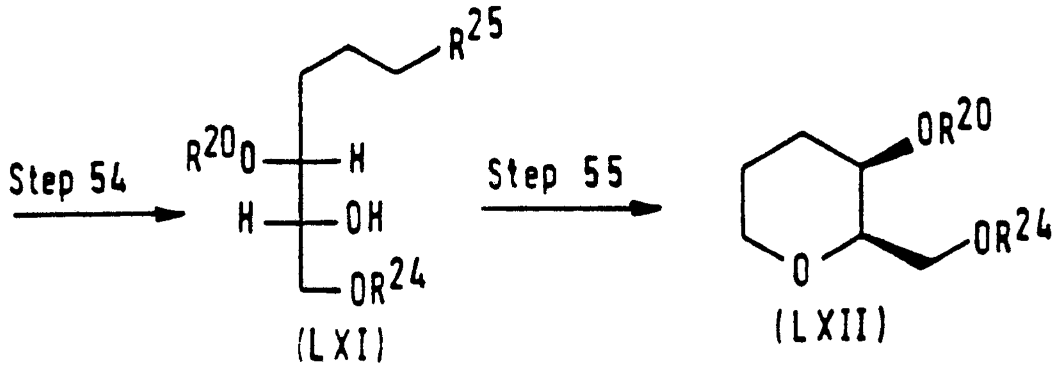


(LXI) (LXII)

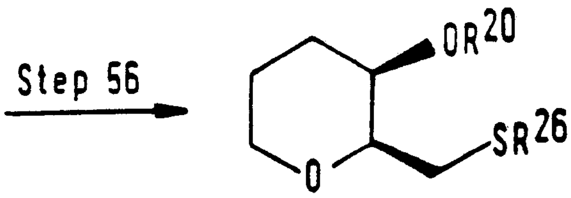


(LXIII)

(LVI)

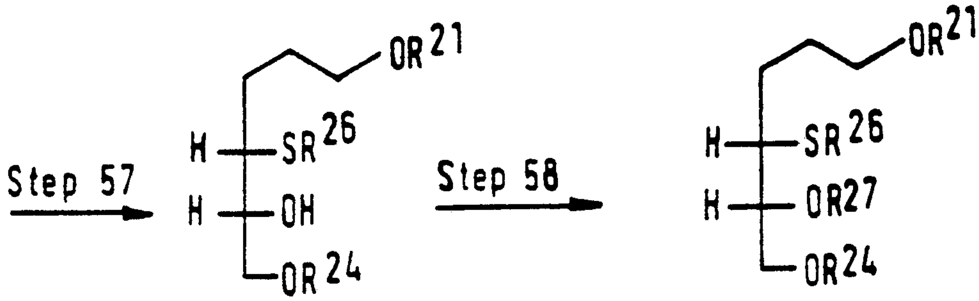


(LXIV) (LXV)

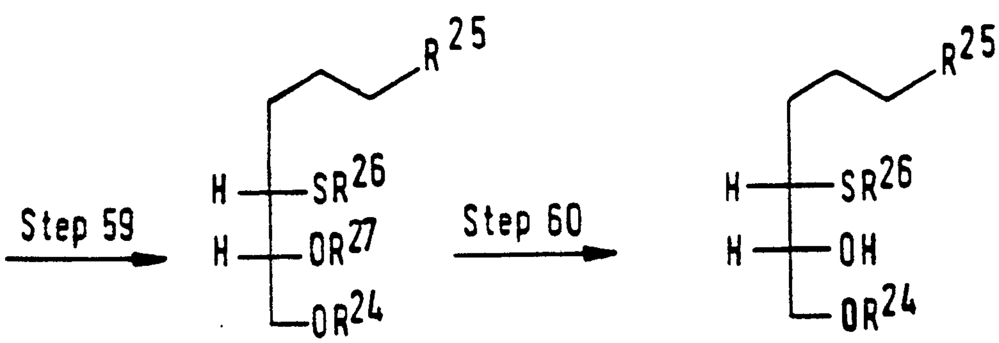


(LXVI) (LXVII)

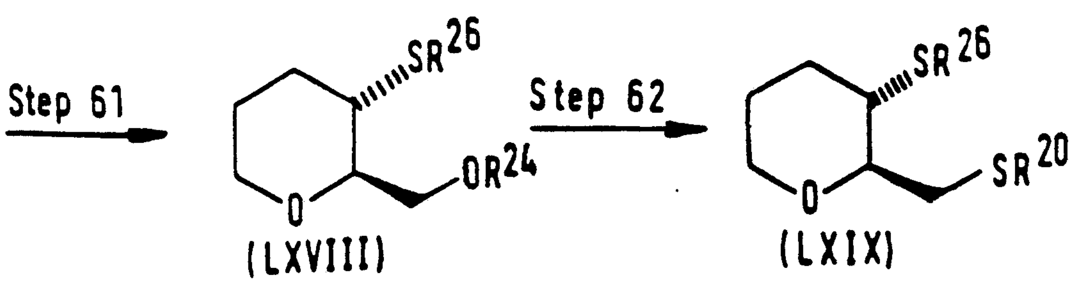


(LXVIII) (LXIX)

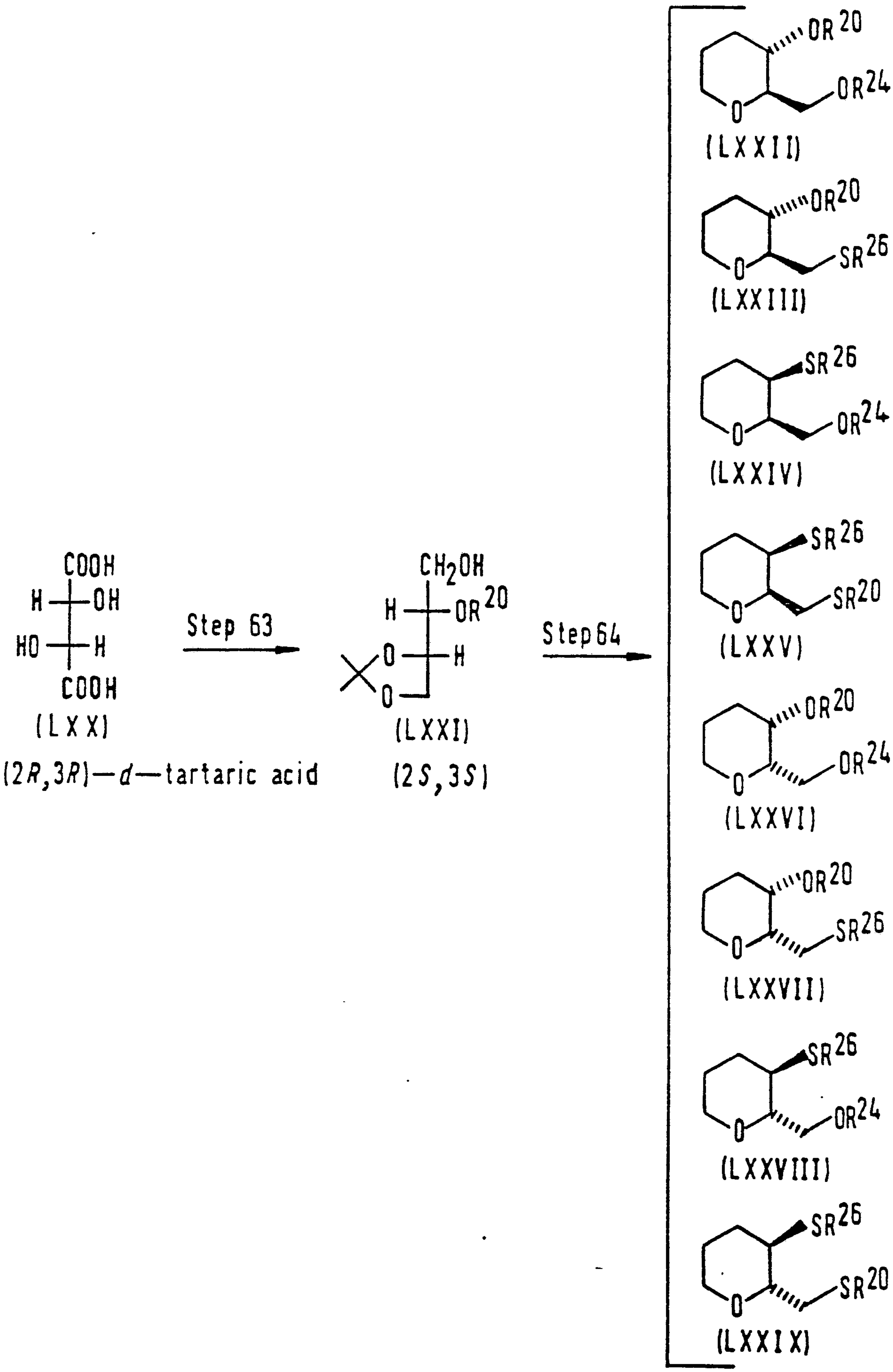


(LXX)
(2*R*,3*R*)—*d*—tartaric acid

(LXXI)
(2*S*,3*S*)

(LXXII)

(LXXIII)

(LXXIV)

(LXXV)

(LXXVI)

(LXXVII)

(LXXVIII)

(LXXIX)

In the above formulae, $R^{20}$, $R^{21}$, $R^{24}$, $R^{26}$ and $R^{27}$ are the same or different and each represents a hydroxy-protecting group or mercapto-protecting group, examples of which are given above in relation to the groups defined as $R^7$, $R^8$ and $R^9$. Where any molecule contains two or more of the groups $R^{20}$, $R^{21}$, $R^{24}$, $R^{26}$ and $R^{27}$, it is generally preferred that the two or more groups should be chosen from different classes of protecting group, so that each protecting group can be removed separately without removing the other or others, $R^{23}$ represents a lower alkyl group, e. g. a $C_1$-$C_6$, preferably $C_1$-$C_4$, alkyl group, such as a methyl, ethyl, propyl, isopropyl, butyl, isobutyl, sec-butyl, t-butyl, pentyl or hexyl group. $R^{25}$ represents a $C_1$-$C_6$ alkylsulphonyloxy group or an arylsulphonyloxy group, for example a methanesulphonyloxy, ethanesulphonyloxy, benzenesulphonyloxy, p-toluenesulphonyloxy, naphthalenesulphonyloxy or camphorsulphonyloxy group.

We particularly prefer that the protecting group represented by $R^{20}$ should be a benzyl or substituted benzyl group, $R^{21}$ should be a substituted silyl group, $R^{24}$ should be a di- or triarylmethyl group, $R^{26}$ should be a lower alkyl group and $R^{27}$ should be a tetrahydropyranyl or substituted tetrahydropyranyl group.

The starting material for these reactions can be l- or d-tartaric acid of formula (XLV) or (LXX), l-tartaric acid being (2S, 3S) and d-tartaric acid being (2R, 3R).

In Step 39 of the reaction scheme, the compound of formula (XLVI) is prepared from l-tartaric acid (XLV) according to the method described by ohno et al [Tetrahedron Letters 23, 3507 (1982)].

In Step 40, the remaining primary hydroxy group of this compound of formula (XLVI) is acylated (for example as described in relation to the first part of Reaction 8), and then the acyloxy group is replaced by an iodine atom and finally the resulting iodide is reacted with a dialkyl malonate in the presence of a base (such as sodium hydride) and a solvent (such as dimethylformamide), in order to give the compound of formula (XLVII).

In Step 41, this compound of formula (XLVII) is converted to a compound of formula (XLVIII) having two more carbon atoms than the original tartaric acid by decarboxylation, by heating the compound in a dimethyl sulphoxide solution of, for example, sodium chloride, in the presence of a trace of water.

In Step 42, the carboxy group of the compound of formula (XLVIII) is reduced to a hydroxymethyl group by reduction with a suitable reducing agent, for example lithium aluminium hydride in a solvent, for example an ether.

In Step 43 the resulting compound of formula (XLIX) is converted to the compound of formula (L) by protecting the free primary hydroxy group with a protecting group $R^{21}$ (a diphenyltbutylsulphonyl group is preferred), removing the isopropylidine group and protecting the primary hydroxy groups thus generated with another hydroxyprotecting group $R^{24}$ (the triphenylmethyl group is preferred). In Step 44, the free secondary hydroxy group is then acylated in the same manner as described in Reaction 8 to introduce the sulphonyloxy group $R^{25}$.

In Step 45, the hydroxyprotecting group $R^{21}$ is removed to give the compound of formula (LII). Where $R^{21}$ is a substituted silyl group, its removal is preferably effected by means of a compound generating fluoride anions. In Step 46, this compound of formula (LII) is cyclized, with inversion at the 2-position, to give the optically active cyclic compound (LIII) by treating the compound of formula (LII) with a base, for example potassium t-butoxide in t-butanol.

If desired, the corresponding mercapto compound of formula (LIV) can be prepared, with retention of configuration at the 2-position, by following the procedure hereinbefore described in Reaction 8.

The compound of formula (LV) can be prepared from the compound of formula (XLIX) by protecting the primary hydroxy group with, for example, $R^{27}$, removing the protecting group $R^{20}$ from the secondary hydroxy group, acylating the secondary hydroxy group thus generated in the same way as described in the first step of Reaction 8, and finally removing the protecting group $R^{27}$ from the primary hydroxy group (Step 48). The protected thiol compound of formula (LVI) can be prepared by reacting the resulting compound (LV) in the same manner as described in the second step of Reaction 8. The optically active compound of formula (LVII) or its mercapto analogue (LVIII) can then be prepared by following the procedures described in Steps 43, 44, 45 and 46 (together Step 50) and, if desired, Step 47 (Step 51). The compound of formula (LVII) can also be prepared from a compound of formula (LV) in which the primary hydroxy group is protected (prepared by the third reaction of Step 48) by following the procedures described in Steps 49 and 50.

As a further alternative, in Step 52, the secondary hydroxy group of the compound of formula (L) can be protected by a protecting group $R^{27}$, to give a compound (LIX), and then the protecting group $R^{21}$ can be selectively removed and the resulting hydroxy group acylated as described in Reaction 8 (Step 53). The resulting compound of formula (LX) can then be treated by Steps 54, 55 and 56, following the same procedures as described in Steps 45, 46 and 47, to give the compounds of formula (LXII) or (LXIII).

The compounds (LXVIII) and (LXIX) can be prepared from the compound (LVI) via Steps 57, 58, 59, 60, 61 and 62, involving the same reactions as described heretofore in Steps 43, 52, 53, 54, 55 and 47, respectively.

Instead of employing l-tartaric acid as the starting material, as illustrated in Steps 63 and 64, d-tartaric acid (LXX), the (2R, 3R) compound can be employed as the starting material to give the corresponding (2S, 3S) compound (LXXI) in Step 63, which corresponds to Step 39 described above, and then, in Step 64, this can be converted to any one of compounds (LXXII) - (LXXIX), following the appropriate ones of Steps 40 - 62.

The corresponding compounds where m is 2, that is to say optically active 5-membered ring compounds, can be prepared from a compound of formula (LXXX) and its enantiomer obtained as illustrated in step 65 by reacting the compound of formula (XLVI) or (LXXI) with an alkali metal cyanide, subjecting the resulting compound to alcoholysis to yield the ester and then reducing this ester to the corresponding hydroxy compound (LXXX), e. g.:

(XLVI) Step 65 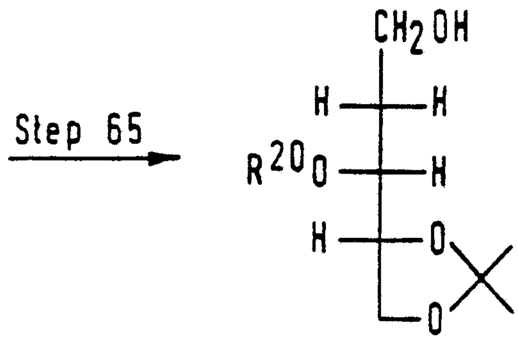

(LXXX)

This compound of formula (LXXX) and its enantiomer may then be subjected to any appropriate combination of Steps 43 - 62 and 64 to give the corresponding optically active 5-membered ring compounds.

7-Membered ring compounds, in which m is 4, can be prepared by treating the compound of formula (LXXX) and its enantiomer by any appropriate combination of Steps 40 - 62 and 64.

After completion of the foregoing reactions, the resulting compounds may be separated from the reaction mixture by conventional means and may, if desired, be further purified by various conventional techniques, for example recrystallization and/or the various chromatography techniques, including column chromatography, thin layer chromatography and liquid chromatography.

The compounds of the present invention have shown excellent PAF antagonistic activity and anti-inflammatory activity, in terms of the duration of the effect and/or biological utilization. They are, accordingly, useful as a new type of anti-shock agent, anti-thrombotic agent, anti-asthmatic agent, anti-allergic agent and anti-inflammatory agent.

The compounds of the invention may be administered orally or parenterally as required and may, if desired, be formulated into appropriate pharmaceutical formulations, depending upon the desired route of administration. For example, for oral administration, the compounds may be formulated as tablets, capsules, granules, powders or syrups. For parenteral administration, they may be formulated as injectible solutions or suspensions or as suppositories. Although the preferred dose will vary, depending upon the nature of the disorder, the symptoms, age, condition and body weight of the patient and the route of administration, a preferred dose for an adult human patient would normally be expected to be from 0.1 to 200 mg/kg body weight per day, and this could be administered in a single dose or in divided doses.

The invention is further illustrated by the following non-limiting Examples. Preparation of certain of the starting materials employed in these Examples is illustrated by the subsequent preparations. The biological activities of certain of the compounds of the invention are then illustrated in the subsequent Experiments.

**Exemple 1**

dl-[trans-3-(N-Heptadecylcarbamoyloxy)tetrahydropyran-2-yl]methyl 2-thiazolioethyl phosphate (inner salt)

A solution of 2.307 g of 2-bromoethyl phosphorodichloridate in 20 ml of methylene chloride was added dropwise, whilst ice-cooling, to 40 ml of methylene chloride in which was dissolved 2.630 g of dl-(trans-2-hydroxymethyltetrahydropyran-3-yl) N-heptadecylcarbamate (prepared as described in Preparation 4) and 1.51 ml of triethylamine. The mixture was stirred for 3 hours at room temperature. 5 ml of pyridine and 2.5 ml of water were then added to the reaction mixture. The mixture was stirred for 14 hours at the same temperature and then concentrated by evaporation under reduced pressure to give a residue. Ethyl acetate and water were added to the residue and the aqueous layer was acidif ied by the addition of a 10 % w/v aqueous solution of hydrochloric acid. The organic layer was separated and the aqueous layer was extracted with ethyl acetate. The combined organic layer and extract were washed with water, dried and concentrated by evaporation under reduced pressure. The residue was subjected to column chromatography through 80 g of silica gel. Fractions eluted with methylene chloride were discarded as an impure material. Fractions eluted with mixtures of methylene chloride and methanol ranging from 50 : 1 to 2 : 1 by volume were worked up to give 2.998 g of dl-[trans-3-(N-heptadecylcarbamoyl-oxy)tetrahydropyran-2-yl]methyl 2-bromoethyl phosphate as an oil.

Infrared Absorption Spectrum ($CHCl_3$) $\nu_{max}cm^{-1}$:
3450 (>NH), 3350 (-OH), 1710 (-O-CO-).

1.586 g of the above compound and 1.87 ml of thiazole were dissolved in 2 ml of toluene, and the solution was heated on an oil bath kept at 70° for 6 days, whilst stirring. The solution was cooled and then the solvent was distilled off. The residue was dissolved in 15 ml of a 95 : 5 by volume mixture of tetrahydrofuran and water. The solution was passed through a column of Amberlite (trade mark) MB-3 resin, and the resulting solution was passed through the same column again. This operation was repeated a total of 6 times, and finally the column

was washed with the same solvent. The solution was combined with the washings, and then the solvent was stripped off by evaporation under reduced pressure, and the residue was subjected to column chromatography through 20 g of silica gel, to give 0.422 g of the title compound as a white powder from the fractions eluted with a 32 : 7 : 1 by volume mixture of methylene chloride, methanol and water.

Nuclear Magnetic Resonance Spectrum ($CD_3OD$) δ ppm:
0.8 - 2.4 (37H, multiplet);
3.05 (2H, multiplet);
3.2 - 4.9 (10H, multiplet);
8.27 (1H, multiplet);
8.53 (1H, doublet, J = 3Hz);
10.17 (1H, multiplet).

Infrared Absorption Spectrum (KBr) $\nu_{max}cm^{-1}$:
1700 (-O-CO-).

Elemental Analysis:

| | |
|---|---|
| Calculated for | $C_{29}H_{53}N_2O_7PS.1/2H_2O$: |
| | C, 56.75 %; H, 8.87 %; N, 4.56 %. |
| Found: | C, 56.91 %; H, 8.80 %; N, 4.51 %. |

**Example 2**

dl-[trans-3-(N-Heptadecylcarbamoyloxy)tetrahydropyran-2-yl]methyl 2(trimethylammonio)ethyl phosphate (inner salt)

1.402 g of dl-[trans-3-(N-heptadecylcarbamoyloxy)tetrahydropyran-2-yl]methyl 2-bromoethyl phosphate (prepared as described in Example 1) was dissolved in a 3 : 5 : 5 by volume mixture of chloroform, isopropanol and dimethylformamide, and 7.0 g of gaseous trimethylamine were introduced into the solution. The mixture was heated, under a nitrogen atmosphere, on an oil bath kept at 50° for 6 hours, whilst stirring, and was then cooled, after which 0.483 g of silver carbonate was added. The mixture was heated under reflux for 1 hour, and then the solvent was distilled off. Methanol was added to the residue and insolubles were separated by filtration. The solvent was stripped from the resulting solution by evaporation under reduced pressure. The residue was purified by liquid chromatography [using 30 g of silica gel and a Lobar column (size B)], giving 0.930 g of the title compound as a white powder from the fractions eluted with a 32 : 7 : 1 by volume mixture of methylene chloride, methanol and water.

Nuclear Magnetic Resonance Spectrum ($CD_3OD$) δ ppm:
0.8 - 2.4 (37H, multiplet);
3.23 (9H, singlet);
2.9 - 4.8 (13H, multiplet).

Infrared Absorption Spectrum (KBr) $\nu_{max}cm^{-1}$:
1700 (-O-CO-).

Elemental Analysis:

| | |
|---|---|
| Calculated for | $C_{29}H_{59}N_2O_7P.H_2O$: |
| | C, 58.37 %; H, 10.30 %; N, 4.69 %; P, 5.19 %. |
| Found: | C, 58.23 %; H, 10.23 %; N, 4.72 %; P, 5.19 %. |

**Example 3**

dl-[trans-3-(N-Octadecylcarbamoyloxy)tetrahydropyran-2-yl]methyl 2-thiazolioethyl phosphate (inner salt)

0.594 g of dl-(trans-2-hydroxymethyltetrahydropyran-3-yl) N-octadecylcarbamate (prepared as described in Preparation 5) was treated in the same way as described in Example 1, to give 0.284 g of the title compound as a white powder.

Nuclear Magnetic Resonance Spectrum ($CD_3OD$) δ ppm:
0.8 - 2.5 (39H, multiplet);
2.9 - 5.1 (13H, multiplet);

8.30 (1H, multiplet);
8.53 (1H, doublet, J = 3Hz);
10.20 (1H, multiplet).

Infrared Absorption Spectrum (KBr) $\nu_{max}cm^{-1}$:
1700 (-O-CO-).

Elemental Analysis:

| | | |
|---|---|---|
| Calculated for | $C_{30}H_{55}N_2O_7PS.H_2O$: | |
| | C, 56.58 %; H, 9.02 %; N, 4.40 %; P, 4.86 %. | |
| Found: | C, 56.71 %; H, 8.65 %; N, 4.38 %; P, 4.41 %. | |

**Exemple 4**

dl-[cis-3-(N-Octadecylcarbamoyloxy)tetrahydropyran-2-yl]methyl 2-thiazolioethyl phosphate (inner salt)

0.690 g of dl-(cis-2-hydroxymethyltetrahydropyran-3-yl) N-octadecylcarbamate (prepared as described in Preparation 7) was treated as described in Example 1, except that the reaction mixture was heated on an oil bath at 80° C for 64 hours (in place of heating on an oil bath for 6 days at a temperature of 70° C), to give 0.180 g of the title compound as a white powder.

Nuclear Magnetic Resonance Spectrum ($CD_3OD$) δ ppm:
0.7 - 2.2 (39H, multiplet);
2.9 - 4.9 (11H, multiplet);
8.28 (1H, doublet, J = 3Hz);
8.51 (1H, doublet, J = 3Hz);
10.18 (1H, multiplet).

Infrared Absorption Spectrum (KBr) $\nu_{max}cm^{-1}$:
1695 (-O-CO-).

Elemental Analysis:

| | |
|---|---|
| Calculated for | $C_{30}H_{55}N_2O_7PS.3/2H_2O$: |
| | C, 55.79 %; H, 9.05 %; N, 4.34 %; P, 4.80 %. |
| Found: | C, 55.90 %; H, 8.65 %; N, 4.38 %; P, 4.78 %. |

**Example 5**

dl-[trans-2-(N-Octadecylcarbamoyloxymethyl)tetrahydropyran-3-yl] 2-thiazolioethyl phosphate (inner salt)

1.711 g of dl-(trans-3-hydroxytetrahydropyran-2-yl)methyl N-octadecylcarbamate (prepared as described in Preparation 10) was reacted with 2-bromoethyl phosphorodichloridate in a manner similar to that described in Example 1, to afford 1.381 g of dl-[trans-2-(N-octadecylcarbamoyloxymethyl)tetrahydropyran-3-yl] 2-bromoethyl phosphate, 0.888 g of this compound was dissolved in 3 ml of toluene, and the solution was mixed with 1.02 ml of thiazole. The mixture was then heated on an oil bath kept at 80°C for 60 hours, whilst stirring. The resulting reaction mixture was then worked up as described in Example 1, to give 0.178 g of the title compound as a powder.

Nuclear Magnetic Resonance Spectrum ($CD_3OD$) δ ppm:
0.7 - 2.4 (39H, multiplet);
2.9 - 5.0 (13H, multiplet);
8.27 (1H, doublet, J = 4Hz);
8.52 (1H, doublet J = 4Hz);
10.20 (1H, multiplet).
Elemental Analysis:

Calculated for

$C_{30}H_{55}N_2O_7PS.3/2H_2O$:
C, 55.79 %; H, 9.05 %; N, 4.34 %;

P.4.79 %.
Found: C, 55.60 %; H, 9.05 %; N, 4.21 %; P, 4.31 %.

**Example 6**

dl-[cis-3-(N-Octadecylcarbamoylthio)tetrahydropyran-2-yl]methyl 2-thiazolioethyl phosphate (inner salt)

1.300 g of dl-S-(cis-2-hydroxymethyltetrahydropyran-3-yl) N-(octadecyl)thiocarbamate (prepared as described in Preparation 14) was reacted with 2-bromoethyl phosphorodichloridate in a manner similar to that described in Example 1, to give 1.673 g of dl-[cis-3-(N-octadecylcarbamoylthio)tetrahydropyran-2-yl]methyl 2-bromoethyl phosphate. 1.000 g of this compound was dissolved in 1.0 ml of toluene, and the resulting solution was mixed with 1.12 ml of thiazole. The mixture was heated on an oil bath kept at 80°C for 109 hours, whilst stirring. It was then worked up as described in Example 1, to give 0.500 g of the title compound as a powder.

Nuclear Magnetic Resonance Spectrum ($CD_3OD$) δ ppm:
0.7 - 2.2 (39H, multiplet);
3.0 - 4.9 (13H, multiplet);
8.30 (1H, multiplet);
8.53 (1H, doublet, J = 3Hz);
10.20 (1H, multiplet).

Elemental Analysis:
Calculated for $C_{30}H_{55}N_2O_6PS_2 1/2H_2O$:
C, 55.96 %; H, 8.77 %; N, 4.35 %; P, 4.81 %.
Found: C, 55.96 %; H, 8.34 %; N, 4.33 %; P, 4.77 %.

**Exemple 7**

dl-(trans-3-Heptadecylcarbamoyloxytetrahydrofuran-2-yl)methyl 2-thiazolioethyl phosphate (inner salt)

0.421 g of the title compound, as a viscous oil, was prepared from 1.527 g of dl-trans-2-hydroxymethyltetrahydrofuran-3-yl N-heptadecylcarbamate (prepared as described in preparation 19) by treating it in a manner similar to that described in Example 1.

Nuclear Magnetic Resonance Spectrum (270MHz, $CD_3OD$) δ ppm:
0.89 (3H, triplet J = 7.0Hz);
1.2 - 1.6 (30H, multiplet);
1.96 (1H, multiplet);
2.20 (1H, multiplet);
3.07 (2H, triplet, J = 7.0Hz);
3.75 - 4.10 (4H, multiplet);
4.20 - 4.40 (2H, multiplet);
5.05 (1H, doublet of multiplets, J = 6.2Hz);
8.26 (1H, doublet of doublets, J = 3.5 & 2.2Hz);
8.50 (1H, doublet of doublets, 3 = 3.7 & 1.1Hz);
10.14 (1H, multiplet).

**Exemple 8**

dl-[trans-3-(N-Heptadecylcarbamoyloxy)tetrahydropyran-2-yl]methyl 6-thiazoliohexyl phosphate (inner salt)

1.000 g of dl-trans-2-hydroxymethyltetrahydropyran-3-yl N-heptadecylcarbamate (prepared as described in preparation 4) was reacted with 1.441 g of 6-bromohexyl phosphorodichloridate in a manner similar to that described in Example 1, to give 1.76 g of dl-[trans-3-(N-heptadecylcarbamoyloxy)tetrahydropyran-2-yl]methyl 6-bromohexyl phosphate, 0.88 g of this compound was dissolved in 2.0 ml of toluene and 1.72 ml of thiazole were added thereto. The resulting mixture was heated on an oil bath at 80°C for 86 hours. It was then worked up as described in Example 1, to give 0.344 g of the title compound as a pale yellow powder.

Nuclear Magnetic Resonance Spectrum ($CD_3OD$) δ ppm:

0.7 - 2.4 (45H, multiplet);
2.8 - 4.9 (13H, multiplet);
4.63 (2H, triplet, J = 7.5Hz);
8.30 (1H, doublet, J = 4Hz);
8.54 (1H, doublet, J = 4Hz).
Elemental Analysis:

| | |
|---|---|
| Calculated for | $C_{33}H_{61}N_2O_7PS.H_2O$: |
| | C, 58.38 %; H, 9.35 %; N, 4.13 %; |
| | P, 4.56 %; S, 4.72 %. |
| Found: | C, 58.09 %; H, 9.31 %; N, 4.15 %; |
| | P, 4.66 %; S, 4.94 %. |

**Exemple 9**

dl-[trans-3-(N-Heptadecylcarbamoyloxy)tetrahydropyran-2-yl]methyl 6-[5-(2-hydroxyethyl)-4-methylthiazolio]hexyl phosphate (inner salt)

0.88 g of dl-[trans-3-(N-heptadecylcarbamoyloxy)tetrahydropyran-2-yl]methyl 6-bromohexyl phosphate (prepared as described in the first step of Example 8) was dissolved in 2.0 ml of toluene. The resulting solution was mixed with 2.88 ml of 5-(2-hydroxyethyl)-4-methylthiazole. The mixture was then heated on an oil bath at 80°C for 90 hours, whilst stirring. It was then worked up as described in Example 1, to give 0.460 g of the title compound as a powder.

**Exemple 10**

dl-[cis-3-(N-Heptadecylcarbamoylthio)tetrahydropyran-2-yl]methyl 4-thiazoliobutyl phosphate (inner salt)

0.500 g of dl-S-(cis-2-hydroxymethyltetrahydropyran-3-yl) N-(heptadecyl)thiocarbamate (prepared as described in Preparation 21) was reacted with 0.942 g of 4-bromobutyl phosphorodichloridate in a manner similar to that described in Example 1, to give 0.75 g of dl-[cis-3- (N-heptadecylcarbamoylthio)tetrahydropyran-2-yl]methyl 4-bromobutyl phosphate. The whole of this compound was then dissolved in 2.0 ml of toluene, and the resulting solution was mixed with 1.65 ml of thiazole. The mixture was heated on an oil bath kept at 80°C for 67 hours, whilst stirring, and was then worked up as described in Example 1, to give 0.309 g of the title compound as a pale yellow powder.

Nuclear Magnetic Resonance Spectrum ($CD_3OD$) δ ppm:
0.7 - 2.4 (4H, multiplet);
2.9 - 4.2 (11H, multiplet);
4.71 (2H, triplet, J = 7.5Hz);
8.29 (1H, doublet, J = 4Hz);
8.55 (1H, doublet, J = 4Hz).

**Exemple 11**

dl-[cis-3-(N-Heptadecylcarbamoylthio)tetrahydropyran-2-yl]methyl 5-thiazoliopentyl phosphate (inner salt)

0.500 g of dl-S-(cis-2-hydroxymethyltetrahydropyran-3-yl) N-(heptadecyl)thiocarbamate (prepared as described in preparation 21) was reacted with 1.322 g of 5-bromopentyl phosphorodichloridate in a manner similar to that described In Example 1, to give 0.85 g of dl-[cis-3-(N-heptadecylcarbamoylthio)tetrahydropyran-2-yl]methyl 5-bromopentyl phosphate. The whole of this compound was dissolved in 2.0 ml of toluene and the resulting solution was mixed with 1.65 ml of thiazole. The mixture was heated on an oil bath at 80°C for 88 hours, whilst stirring. It was then worked up as described in Example 1, to give 0.340 g of the title compound as a powder.

Nuclear Magnetic Resonance Spectrum ($CD_3OD$) δ ppm:
0.7 - 2.3 (43H, multiplet);
3.0 - 4.2 (11H, multiplet);
4.63 (2H, triplet, J = 7.5Hz);
8.29 (1H, doublet J = 4Hz);
8.54 (1H, doublet, J = 4Hz).

Elemental Analysis:
Calculated for $C_{32}H_{59}N_2O_6PS_2.H_2O$:
C, 56.44 %; H, 9.03 %; N, 4.11 %;
P, 4.55 %; S, 9.42 %.
Found: C, 56.48 %; H, 9.07 %; N, 4.14 %;
P, 4.75 %; S, 9.64 %.

**Exemple 12**

dl-[cis-3-(N-Heptadecylcarbamoylthio)tetrahydropyran-2-yl]methyl 6-thiazoliohexyl phosphate (inner salt)

0.500 g of dl-S-(cis-2-hydroxymethyltetrahydropyran-3-yl) N-(heptadecyl)thiocarbamate (prepared as described in Preparation 21) was reacted with 1.040 g of 6-bromohexyl phosphorodichloridate in a manner similar to that described in Example 1, to give 0.86 g of dl-[cis-3-(N-heptadecylcarbamoylthio)tetrahydropyran-2-yl]methyl 6-bromohexyl phosphate. The whole of this compound was dissolved in 2.0 ml of toluene and the resulting solution was mixed with 1.65 ml of thiazole. The mixture was heated on an oil bath at 80°C for 87 hours, whilst stirring. It was then worked up as described in Example 1, to give 0.305 g of the title compound as a powder.

Nuclear Magnetic Resonance Spectrum ($CD_3OD$) δ ppm:
0.7 - 2.4 (45H, multiplet);
3.0 - 4.2 (11H, multiplet);
4.61 (2H, triplet, J = 7.5Hz);
8.28 (1H, doublet, J = 4Hz);
8.52 (1H, doublet, J = 4Hz).

Elemental Analysis:

Calculated for $C_{33}H_{61}N_2O_6PS_2.H_2O$:
C, 57.03 %; H, 9.14 %; N, 4.03 %;
P, 4.46 %; S, 9.23 %.
Found: C, 57.06 %; H, 9.18 %; N, 3.97 %;
P, 4.30 %; S, 9.59 %.

**Preparation 1**

6-Benzyloxymethyl-3,4-dihydro-2H-pyran

A solution of 5.71 g of 6-hydroxymethyl-3,4-dihydro-2H-pyran in 100 ml of dimethylformamide was added dropwise to a suspension of 2.18 g of sodium hydride (in the form of a 55 % w/w dispersion in mineral oil) in 100 ml of dimethylformamide, whilst ice-cooling. The mixture was stirred for 1 hour at room temperature, and then 6.33 g of benzyl chloride were added. After this, the reaction mixture was stirred for 16 hours, poured into 1 litre of water and then extracted twice with ethyl acetate. The extracts were washed with water, dried and then concentrated by evaporation under reduced pressure, to give 13 g of an oily residue. This residue was subjected to column chromatography through 200 g of silica gel. Those fractions eluted with mixtures of diethyl ether and hexane ranging from 4 : 100 to 5 : 100 by volume were worked up to give 9.40 g of the title compound as an oily substance, boiling at 125 - 130°C (bath temperature)/l mmHg (133 Pa).

Nuclear Magnetic Resonance Spectrum ($CDCl_3$) δ ppm:
1.65 - 2.2 (4H, multiplet);
3.87 (2H, singlet);
4.03 (2H, multiplet);
4.57 (2H, singlet);
4.80 (1H, triplet J = 3.5Hz);
7.2 - 7.6 (5H, multiplet).

Elemental Analysis:
Calculated for $C_{13}H_{16}O_2$:
C, 76.44 %; H, 7.90 %.
Found: C, 76.36 %; H, 7.90 %.

**Preparation 2**

dl-trans-2-Benzyloxymethyltetrahydropyran-3-ol

29.3 ml of a 1M tetrahydrofuran solution of borane were added dropwise to a solution of 9.00 g of 6-benzyloxymethyl-3,4-dihydro-2H-pyran (prepared as described in Preparation 1) dissolved in 30 ml of tetrahydrofuran, at a temperature in the range from -5°C to 0°C. The mixture was stirred at room temperature for 3 hours, and then a 10 % w/v aqueous solution of sodium hydroxide was added dropwise. 10.8 ml of a 30 % v/v aqueous solution of hydrogen peroxide were then added dropwise, maintaining the temperature of the reaction mixture between 32 and 40°C. The reaction mixture was then stirred at room temperature for a further 1 hour. At the end of this time, the organic layer was separated, washed with water, dried and concentrated by evaporation under reduced pressure, to give 10.5 g of an oily residue. This residue was subjected to column chromatography through 250 g of silica gel. Those fractions eluted with a 1 : 20 by volume mixture of ethyl acetate and methylene chloride were worked up to give 8.82 g of the title compound, boiling at a bath temperature of 130 - 135°C/l mmHg (133 Pa).

Nuclear Magnetic Resonance Spectrum ($CDCl_3$) δ ppm:
1.15 - 2.25 (4H, multiplet);
2.83 (1H, doublet, J = 3Hz);
3.1 - 3.6 (3H, multiplet);
3.68 (2H, doublet, J = 5Hz);
3.75 - 4.05 (1H, multiplet);
4.58 (2H, singlet);
7.2 - 7.5 (5H, multiplet).

Mass spectrum (m/e): 222 (M+).

Elemental Analysis:

| | |
|---|---|
| Calculated for | $C_{13}H_{18}O_2$: |
| | C, 70.24 %; H, 8.16 %. |
| Found: | C, 70.07 %; H, 8.04 %. |

**Preparation 3**

dl-trans-2-Benzyloxymethyltetrahydropyran-3-yl N-heptadecylcarbamate

A solution of 6.082 g of stearic acid, 3.84 ml of diphenylphosphoryl azide and 2.48 ml of triethylamine in 200 ml of benzene was heated under reflux for 3 hours. At the end of this time, the reaction mixture was cooled to room temperature and then washed, in turn, with an aqueous solution of sodium bicarbonate and then with an aqueous solution of sodium chloride. It was then dried and concentrated by evaporation under reduced pressure. The resulting residue was dissolved in 160 ml of benzene 1.980 g of dl-trans-2-benzyloxymethyltetrahydropyran-3-ol (prepared as described in Preparation 2) were then added to the resulting solution, and the mixture was heated under reflux for 38 hours under a nitrogen atmosphere. The reaction mixture was cooled to room temperature and then washed, in turn, with an aqueous solution of sodium bicarbonate and then with water. It was then dried and concentrated by evaporation under reduced pressure. The resulting residue was subjected to column chromatography through 60 g of silica gel. Fractions eluted with a 6 : 3 : 1 by volume mixture of hexane, methylene chloride and diethyl ether were worked up to afford 3.904 g of the title compound as a white solid, melting at 61 - 63°C.

Infrared Absorption Spectrum ($CHCl_3$) $\nu_{max}cm^{-1}$:
3460 (-NH-), 1720 (-O-CO-).
Mass spectrum (m/e): 503 (M+), 412 (M+-$C_7H_7$).

Elemental Analysis:

| | |
|---|---|
| Calculated for | $C_{31}H_{53}NO_4$: |
| | C, 73.91 %; H, 10.60 %; N, 2.78 %. |
| Found: | C, 74.27 %; H, 10.70 %; N, 2.71 %. |

**Preparation 4**

dl-trans-2-Hydroxymethyltetrahydropyran-3-yl N-heptadecylcarbamate

3.800 g of dl-trans-2-benzyloxymethyltetrahydropyran-3-yl N-heptadecylcarbamate (prepared as described in preparation 3) were dissolved in 120 ml of methanol and were then hydrogenated at room temperature for 8

hours in the presence of 1.90 g of a 10 % w/w palladium-on-carbon catalyst and hydrogen at an initial pressure of 4 atmospheres (about 4 bars) in a Paar's apparatus. At the end of this time, the catalyst was filtered off and then the solvent was distilled off, to give the desired compound, which was recrystallized from diethyl ether, yielding 2.729 g of the title compound as a white solid melting at 84 - 86° C.

Infrared Absorption Spectrum ($CHCl_3$) $\nu_{max}cm^{-1}$:
3570 (-OH), 3450 (-NH), 1710 (-O-CO-).
Mass spectrum (m/e): 413 (M+), 382 (M+-$CH_2OH$).

Elemental Analysis:

| | | |
|---|---|---|
| Calculated for | $C_{24}H_{47}NO_4$: | |
| | C, 69.69 %; H, 11.45 %; N, 3.39 %. | |
| Found: | C, 69.38 %; H, 11.35 %; N, 3.52 %. | |

**Preparation 5**

dl-trans-2-Hydroxymethyltetrahydropyran-3-yl N-octadecylcarbamate

Following a similar procedure to that described in Preparation 3, 5.405 g of nonadecanoic acid were reacted with 1.118 g of dl-2-trans-2-benzyloxymethyltetrahydropyran-3-ol (prepared as described in Preparation 2), and the reaction product was purified by chromatography, to afford 1.417 g of dl-trans-2-benzyloxymethyltetrahydropyran-3-yl N-octadecylcarbamate as a solid.

1.395 g of this solid was dissolved, without further purification, in 30 ml of tetrahydrofuran and was hydrogenated at room temperature for 7 hours in the presence of 700 mg of a 10 % w/w palladium-on-carbon catalyst and of hydrogen at an initial pressure of 4 atmospheres (about 4 bars). The catalyst was then removed by filtration and the solvent was distilled off, to give the title compound. This was recrystallized from diethyl ether, to give 1.128 g of the title compound as a white solid melting at 84 - 86° C.

Nuclear Magnetic Resonance Spectrum ($CDCl_3$) δ ppm:
0.7 - 2.4 (39H, multiplet);
2.80 (1H, multiplet);
3.0 - 4.2 (7H, multiplet);
4.5 - 4.9 (2H, multiplet).

Infrared Absorption Spectrum ($CHCl_3$) $\nu_{max}cm^{-1}$:
3600 (-OH), 3460 (-NH-), 1710 (-O-CO-).
Mass spectrum (m/e): 427 (M+), 396 (M+-$CH_2OH$).

Elemental Analysis:

| | | |
|---|---|---|
| Calculated for | $C_{25}H_{49}NO_4$: | |
| | C, 70.21 %; H, 11.55 %; N, 3.28 %. | |
| Found: | C, 69.91 %; H, 11.55 %; N, 3.19 %. | |

**Preparation 6**

dl-cis-2-Benzyloxymethyltetrahydropyran-3-ol

2.22 g of dl-trans-2-benzyloxymethyltetrahydropyran-3-ol (prepared as described in Preparation 2) were dissolved in 20 ml of acetone, and were then oxidized with 6 ml of Jones' reagent (containing 1.60 g of chromic anhydride), whilst ice-cooling. The reaction mixture was then stirred at room temperature for 1 hour, after which it was poured into water and extracted twice with ethyl acetate. The combined extracts were washed with water, dried and concentrated by evaporation under reduced pressure, to give 2.08 g of a crude oily ketonic compound. The whole of this ketonic compound was dissolved, without further purification, in 10 ml of tetrahydrofuran and was then reduced at a temperature between 0 and 5° C with 12 ml of a 1M tetrahydrofuran solution of L-selectride. The solution was then stirred for 30 minutes whilst ice-cooling and for 2 hours at room temperature. 6 ml of a 10 % w/v aqueous solution of sodium hydroxide were then added dropwise at 5 - 15° C, and then 6 ml of 35 % v/v aqueous hydrogen peroxide were added dropwise to the mixture at 15 - 30° C. The organic layer was then separated, dried and concentrated by evaporation under reduced pressure, to give an oily residue. This was purified by column chromatography through 60 g of silica gel. Those fractions eluted with mixtures of hexane and ethyl acetate ranging from 100 : 15 to 2 : 1 by volume were worked up to give 1.135 g of the title compound as a colourless liquid boiling at a bath temperature of 130 - 140° C/l mmHg (about 133 Pa).

Nuclear Magnetic Resonance Spectrum ($CDCl_3$) δ ppm:
1.25 - 2.30 (4H, multiplet);
2.68 (1H, doublet, J = 6Hz);
3.3 - 3.7 (4H, multiplet);
3.80 (1H, multiplet);
4.03 (1H, multiplet);
4.59 (2H, singlet);
7.2 - 7.5 (5H, multiplet).

Mass spectrum (m/e): 222 (M+).

**Preparation 7**

dl-cis-2-Hydroxymethyltetrahydropyran-3-yl N-octadecylcarbamate

2.627 g of nonadecanoic acid were reacted, in a similar manner to that described in Preparation 3, with 0.815 g of dl-cis-2-benyloxymethyltetrahydropyran-3-ol (prepared as described in Preparation 6). The resulting crude product was purified by column chromatography through 70 g of silica gel. The fractions eluted with a 1 : 5 by volume mixture of ethyl acetate and hexane afforded 1.05 g of dl-cis-2-benzyloxymethyltetrahydropyran-3-yl N-octadecylcarbamate.

The whole of this product was dissolved in 30 ml of a 2 : 1 by volume mixture of tetrahydrofuran and methanol, and was then hydrogenated at room temperature for 8 hours in the presence of 0.50 g of a 10 % w/w palladium-on-carbon catalyst and hydrogen at an initial pressure of 4 atmospheres (about 4 bars) in a Paar's apparatus. The catalyst was removed by filtration, and then the solvent was distilled off under reduced pressure. The residue was subjected to column chromatography through 20 g of silica gel. Those fractions eluted with mixtures of ethyl acetate and hexane ranging from 1 : 5 to 1 : 2 by volume were worked up and recrystallized from a mixture of diethyl ether and hexane, to give 0.732 g of the title compound as crystals melting at 85 - 86°C.

Nuclear Magnetic Resonance Spectrum ($CDCl_3$) δ ppm:
0.7 - 2.2 (39H, multiplet);
2.9 - 3.8 (7H, multiplet);
3.9 - 4.2 (1H, multiplet);
4.65 - 5.1 (2H, multiplet).

Infrared Absorption Spectrum ($CHCl_3$) $\nu_{max}cm^{-1}$:
3600 (-OH), 3450 (-NH-), 1700 (-O-CO-).
Mass spectrum (m/e): 427 (M+), 396 ($M^+-CH_2OH$).
Elemental Analysis:

| | |
|---|---|
| Calculated for | $C_{25}H_{49}NO_4$: |
| | C,70.21 %; H, 11.55 %; N, 3.28 %. |
| Found: | C, 70.27 %; H, 11.73 %; N, 3.28 %. |

**Preparation 8**

dl-trans-2-Benzyloxymethyl-3-(tetrahydropyran-2-yloxy)tetrahydropyran

2.22 g of dl-trans-2-benzyloxymethyltetrahydropyran-3-ol (prepared as described in preparation 2), 2.65 ml of dihydropyran and 0.05 g of pyridinium p-toluenesulphonate were dissolved in 40 ml of methylene chloride, and the mixture was stirred at room temperature for 4 hours. The solvent was then distilled off to give a residue, which was subjected to column chromatography through 80 g of silica gel. Those fractions eluted with mixtures of hexane and diethyl ether ranging from 6 : 1 to 5 : 1 by volume were worked up, to afford 2.93 g of the title compound as a colourless oil.

Nuclear Magnetic Resonance Spectrum ($CDCl_3$) δ ppm:
1.1 - 2.4 (10H, multiplet);
3.15 - 4.15 (8H, multiplet);
4.57 (2H, AB-quartet, J = 12Hz);
4.78 (1H, multiplet);
7.35 (5H, multiplet).

Mass spectrum (m/e): 306 (M+).

Elemental Analysis:

| | |
|---|---|
| Calculated for | $C_{18}H_{26}O_4$: |
| | C, 70.56 %; H, 8.55 %. |
| Found: | C, 70.65 %; H, 8.45 %. |

**Preparation 9**

dl-trans-3-(Tetrahydropyran-2-yloxy)tetrahydropyran-2-ylmethanol

2.93 g of dl-trans-2-benzyloxymethyl-3-(tetrahydropyran-2-yloxy)tetrahydropyran (prepared as described in Preparation 8) were dissolved in 130 ml of tetrahydrofuran and were then hydrogenated at room temperature for 8 hours in the presence of 1.30 g of a 10 % w/w palladium-on-carbon catalyst and of hydrogen at an initial pressure of 4 atmospheres (about 4 bars). The catalyst was removed by filtration, and then the solvent was distilled off. The resulting residue was purified by column chromatography through 50 g of silica gel. Those fractions eluted with mixtures of hexane and diethyl ether ranging from 2 : 1 to 1 : 1 by volume were worked up to give 1.87 g of the title compound as a colourless oil.

Nuclear Magnetic Resonance Spectrum ($CDCl_3$) δ ppm:
2.2 - 2.4 (10H, multiplet);
2.77 (1H, triplet, J = 7Hz);
3.1 - 4.1 (8H, multiplet);
4.70 (1H, multiplet).

Infrared Absorption Spectrum ($CDCl_3$) $\nu_{max}cm^{-1}$:
3600, 3480 (-OH).
Elemental Analysis:

| | |
|---|---|
| Calculated for | $C_{11}H_{20}O_4$: |
| | C, 61.12 %; H, 9.33 %. |
| Found: | 60.75 %; H, 9.29 %. |

**Preparation 10**

dl-(trans-3-Hydroxytetrahydropyran-2-yl)methyl N-octadecylcarbamate

6.247 g of nonadecanoic acid were reacted with 1.809 g of dl-trans-3-(tetrahydropyran-2-yloxy)tetrahydropyran-2-ylmethanol (prepared as described in Preparation 9) on an oil bath at 85°C for 24 hours, in a manner similar to that described in preparation 3. The reaction mixture was cooled to room temperature, and then solids were filtered off and the solvent was removed by evaporation under reduced pressure. The residue was purified by column chromatography through 100 g of silica gel. The fractions eluted with a 1 : 5 : 5 by volume mixture of diethyl ether, hexane and methylene chloride were worked up to give 4.20 g of dl-[trans-3-(tetrahydropyran-2-yloxy)tetrahydropyran-2-yl]methyl N-octadecylcarbamate as a solid.

The whole of this compound was dissolved in 30 ml of a 1 : 2 by volume mixture of tetrahydrofuran and methanol, and then 0.20 g of camphorsulphonic acid was added to the solution. The reaction mixture was then stirred at room temperature for 45 minutes, after whicha saturated aqueous solution of sodium bicarbonate was added. The solvent was removed by evaporation under reduced pressure, and diethyl ether was added to the residue. The ethereal layer was separated, washed with water and dried and then the ether was evaporated off under reduced pressure. The residue was subjected to column chromatography through 70 g of silica gel. Those fractions eluted with a 1 : 5 : 5 by volume mixture of diethyl ether, hexane and methylene chloride were worked up and then recrystallized from a mixture of diethyl ether and hexane, to give 3.289 g of the title compound as white crystals melting at 55 - 56° C.

Nuclear Magnetic Resonance Spectrum ($CDCl_3$) δ ppm:
0.75 - 2.30 (39H, multiplet);
3.00 - 3.60 (5H, multiplet);
3.69 (1H, doublet, J = 4Hz);
3.85 - 4.20 (2H, multiplet);
4.76 (1H, doublet of doublets, $J_1$ = 13Hz, $J_2$ = 3Hz);
4.95 (1H, multiplet).

Infrared Absorption Spectrum ($CHCl_3$) $\nu_{max}cm^{-1}$:
3450 (-NH-, -OH), 1700 (-O-CO-).

Mass spectrum (m/e): 427 (M+), 409 (M+-$H_2O$).

Elemental Analysis:

| | |
|---|---|
| Calculated for | $C_{25}H_{49}NO_4$: |
| | C, 70.21 %; H, 11.55 %; N, 3.28 %. |
| Found: | C, 70.31 %; H, 11.42 %; N, 3.27 %. |

**Preparation 11**

dl-S-(cis-2-Benzyloxymethyltetrahydropyran-3-yl) thioacetate

1.04 ml of methanesulphonyl chloride were added dropwise, whilst ice-cooling, to a solution of 2.00 g of dl-trans-2-benzyloxymethyltetrahydropyran-3-ol (prepared as described in preparation 2) and 2.51 ml of triethylamine in 40 ml of benzene. The reaction mixture was then stirred at room temperature for 1 hour, after which it was washed with water and dried. The solvent was then evaporated off under reduced pressure, to give a crude methanesulphonate as an oil, which was dissolved, without further purification, in 10 ml of dimethylformamide.

Meanwhile, a solution of 0.77 ml of thioacetic acid in 5 ml of dimethylformamide was added dropwise to a suspension of 0.47 g of sodium hydride (as a 55 % w/w dispersion in mineral oil) in 5 ml of dimethylformamide, whilst cooling; the mixture was then stirred at room temperature for 1 hour. To this mixture was then added the solution of the crude methanesulphonate prepared as described above, and the mixture was heated for 16 hours at 80°C and then for 10 hours at 100°C, whilst stirring. The mixture was cooled to room temperature, poured into water and then extracted with ethyl acetate. The extract was washed with water and dried, and then the solvent was evaporated off under reduced pressure, to give an oily residue. This residue was purified by column chromatography through 50 g of silica gel. Fractions eluted with mixtures of diethyl ether and hexane ranging from 3 : 97 to 10 : 90 by volume were worked up, to give 1.448 g of the title compound as an oil.

Nuclear Magnetic Resonance Spectrum ($CDCl_3$) δ ppm:
1.10 - 2.20 (4H, multiplet);
2.30 (3H, singlet);
3.20 - 4.20 (6H, multiplet);
4.52 (2H, AB-quartet, J = 12Hz);
7.20 - 7.50 (5H, multiplet).

Infrared Absorption Spectrum ($CHCl_3$) $\nu_{max}cm^{-1}$:
1685 (-S-$CO_2$)
Mass spectrum (m/e): 280 (M+).

Elemental Analysis:

| | |
|---|---|
| Calculated for | $C_{15}H_{20}O_3S$: |
| | C, 64.26 %; H, 7.19 %; S, 11.44 %. |
| Found: | C, 64.19 %; H, 6.96 %; S, 11.67 %. |

**Preparation 12**

dl-cis-2-Benzyloxymethyltetrahydropyran-3-thiol

1.04 ml of an approximately 28 % w/v methanolic solution of sodium methoxide were added dropwise at -10°C to 1.422 g of dl-S-(cis-2-benzyloxymethyltetrahydropyran-3-yl) thioacetate (prepared as described in Preparation 11) in 30 ml of methanol. The reaction mixture was stirred at -10 to 0°C for 2 hours, and then 0.33 ml of methanesulphonic acid was added. The reaction mixture was poured into water and extracted with ethyl acetate. The extracts were washed with water, dried and concentrated by evaporation under reduced pressure to give an oily residue. The residue was subjected to column chromatography through 30 g of silica gel. Those fractions eluted with mixtures of diethyl ether and hexane ranging from 3 : 97 to 95 : 5 by volume were worked up, to give 1.146 g of the title compound as an oil.

Nuclear Magnetic Resonance Spectrum ($CDCl_3$) δ ppm:
1.10 - 2.40 (4H, multiplet);
1.66 (1H, doublet J = 10Hz);
2.95 - 3.25 (1H, multiplet);
3.25 - 3.85 (4H, multiplet);
3.85 - 4.20 (1H, multiplet);
4.55 (2H, AB-quartet, J = 12Hz);

7.10 - 7.50 (5H, multiplet).

Infrared Absorption Spectrum ($CHCl_3$) $\nu_{max}cm^{-1}$:
2580 (-SH).
Mass spectrum (m/e): 238 (M+).

Elemental Analysis:

| | |
|---|---|
| Calculated for | $C_{13}H_{18}O_2S$: |
| | C, 65.51 %; H, 7.61 %; S, 13.45 %. |
| Found: | C, 65.62 %; H, 7.83 %; S, 13.19 %. |

**Preparation 13**

dl-S-(cis-2-Benzyloxymethyltetrahydropyran-3-yl) N-(octadecyl)thiocarbamate

3.38 g of nonadecanoic acid were allowed to react with 1.124 g of dl-cis-2-benzyloxymethyltetrahydropyran-3-thiol (prepared as described in Preparation 12) in a similar manner to that described in Preparation 3 to give a crude product. This product was purified by column chromatography through 80 g of silica gel. Those fractions eluted with mixtures of diethyl ether and hexane ranging from 1 : 5 to 1 : 2 were worked up, to give 2.283 g of the title compound as white crystals melting at 85 - 86°C.

Nuclear Magnetic Resonance Spectrum ($CDCl_3$) δ ppm:
0.8 - 2.2 (39H, multiplet);
3.1 - 4.2 (8H, multiplet);
4.57 (2H, AB-quartet, $J_{AB}$ = 12Hz);
5.33 (1H, multiplet);
7.35 (5H, multiplet).

Infrared Absorption Spectrum ($CHCl_3$) $\nu_{max}cm^{-1}$:
3450 (-NH-), 1675 (-S-CO-).

Elemental Analysis:

| | |
|---|---|
| Calculated for | $C_{32}H_{55}NO_3S$: |
| | C, 71.99 %; H, 10.38 %; N, 2.62 %; S, 6.01 %. |
| Found: | C, 71.87 %; H, 10.30 %; N, 2.62 %; S, 6.17 %. |

**Preparation 14**

dl-S-(cis-2-Hydroxymethyltetrahydropyran-3-yl) N-(octadecyl)thiocarbamate

2.32 g of aluminium chloride and 2.61 g of sodium iodide were added succesively to a mixture of 70 ml of acetonitrile and 35 ml of methylene chloride, whilst ice-cooling. A methylene chloride solution containing 1.859 g of dl-S-(cis-2-benzyloxymethyltetrahydropyran-3-yl) N-(octadecyl)thiocarbamate (prepared as described in Preparation 13) was then added, and the reaction mixture was stirred at room temperature for 7 hours. It was then diluted with water and purified by filtration with a celite (trade mark) filter aid. The filtrate was mixed with methylene chloride. The methylene chloride layer was separated and the water layer was extracted with methylene chloride. The combined extract and methylene chloride layer were washed successively with an aqueous solution of sodium thiosulphate and then with water, dried and concentrated by evaporation under reduced pressure. The residue was purified by column chromatography through 45 g of silica gel. Those fractions eluted with mixtures of ethyl acetate and hexane ranging from 1 : 5 to 1 : 2 by volume were worked up, to give 1.200 g of the title compound as white crystals, melting at 93 - 94°C.

Nuclear Magnetic Resonance Spectrum ($CDCl_3$) δ ppm:
0.7 - 2.3 (40H, multiplet);
3.15 - 4.15 (8H, multiplet);
5.53 (1H, multiplet).

Infrared Absorption Spectrum ($CHCl_3$) $\nu_{max}cm^{-1}$:
3450 (-NH-, -OH), 1650 (-S-CO-).

Elemental Analysis:

| | |
|---|---|
| Calculated for | $C_{25}H_{49}NO_3S$: C, 67.67 %; H, 11.13 %; N, 3.16 %; S, 7.23 %. |
| Found: | C, 67.65 %; H, 11.24 %; N, 2.96 %; S, 7.51 %. |

**Preparation 15**

4,5-Dihydrofurfuryl alcohol

358 g of a 15.08 % w/w solution of butyllithium in hexane were added dropwise to 58.7 g of dihydrofuran in 350 ml of anhydrous tetrahydrofuran over a period of 30 minutes at 5 - 10°C, whilst ice-cooling. The reaction mixture was then heated at 50°C for 2 hours, whilst stirring. The mixture was then cooled to 0°C in an ice-bath. 25.0 g of paraformaldehyde were added all at once to the reaction mixture, which was then heated for 2 hours at 50°C. The reaction mixture was cooled to room temperature and then washed with 500 ml of ice-water; the aqueous layer was then extracted five times with methylene chloride. The organic layer and the extracts were combined, dried and concentrated by evaporation under reduced pressure to give 14 g of a residue. The residue was then distilled under reduced pressure to give 8.97 g of the title compound as a colourless oil boiling at 66 - 67°C/7mmHg (about 933Pa). The title compound is preferably used as a reagent in the next step (e. g. Preparation 16) immediately after distillation because it is easily dimerized.

Nuclear Magnetic Resonance Spectrum ($C_6D_6$) δ ppm:

2.21 (2H, broad triplet, J = 9Hz);
2.98 (1H, broad triplet, J = 6Hz);
3.98 (2H, doublet, J = 6Hz);
4.00 (2H, triplet, J = 9Hz);
4.68 (1H, multiplet).

Mass spectrum (m/e): 200 (M+x2), 101 (M+ +1), 100 (M+).

**Preparation 16**

2-Benzyloxymethyl-4,5-dihydrofuran

7.69 g of sodium hydride (as a 55 % w/w suspension in mineral oil) were suspended in 150 ml of dimethylformamide, and 17.64 g of 4,5-dihydrofurfuryl alcohol (prepared as described in preparation 15) in 30 ml of dimethylformamide were added dropwise at 5 - 10°C over 30 minutes, whilst ice-cooling. The mixture was stirred at room temperature for 1 hour, and then 20.93 ml of benzyl bromide were added dropwise thereto at 10 - 15°C over a further 30 minutes, whilst ice-cooling. The reaction mixture was stirred at room temperature for 1 hour, poured into 2 litres of water and extracted twice with ethyl acetate. The extracts were washed with water, dried and evaporated to dryness under reduced pressure, to give 17.8 g of a residue. This residue was purified by column chromatography through 400 g of silica gel. Those fractions eluted with a 7 : 100 by volume mixture of hexane and diethyl ether afforded 3.71 g of the title compound as a colourless oil.

Nuclear Magnetic Resonance Spectrum ($CDCl_3$) δ ppm:
2.65 (2H, triplet of multiplets, $J_1$ = 10Hz);
3.58 (2H, singlet);
4.03 (2H, multiplet);
4.39 (2H, triplet J = 10Hz);
4.93 (1H, multiplet);
7.35 (5H, multiplet).

Mass spectrum (m/e): 190 (M+).

**Preparation 17**

dl-trans-2-Benzyloxymethyltetrahydrofuran-3-ol

3.389 g of 2-benzyloxymethyl-4,5-dihydrofuran (prepared as described in Preparation 16) were hydroborated in a similar manner to that described in Preparation 2. 3.93 g of the crude product were purified by column chromatography through 120 g of silica gel. Those fractions eluted with a 1 : 2 by volume mixture of hexane

and ethyl acetate were condensed by evaporation under reduced pressure, to afford 2.012 g of the title compound as a colourless oil.

Nuclear Magnetic Resonance Spectrum ($CDCl_3$) δ ppm:
1.6 - 2.4 (2H, multiplet);
2.28 (1H, multiplet);
3.43 (1H, doublet of doublets, $J_1$ = 10Hz & $J_2$ = 6Hz);
3.60 (1H, doublet of doublets, $J_1$ = 10Hz & $J_2$ = 4,5Hz).
3.75 - 4.10 (3H, multiplet);
4.27 (1H, multiplet);
4.58 (2H, singlet);
7.30 (5H, singlet).

Mass spectrum (m/e): 208 (M+).

**Preparation 18**

dl-trans-2-Benzyloxymethyltetrahydrofuran-3-yl N-heptadecylcarbamate

1.967 g of dl-trans-2-benzyloxymethyltetrahydrofuran-3-ol (prepared as described in Preparation 17) were treated in a manner similar to that described in Preparation 3 to give 3.711 g of the title compound as white crystals melting at 54 - 56° C, after recrystallization from hexane.

Nuclear Magnetic Resonance Spectrum (270MHz, $CDCl_3$) δ ppm:
0.8 - 1.7 (33H, multiplet);
1.90 - 2.30 (2H, multiplet);
3.15 (2H, doublet of triplets, $J_1$ = $J_2$ = 6.6Hz);
3.59 (2H, doublet, J = 4.4Hz);
3.88 (1H, multiplet);
4.05 (2H, multiplet);
4.56 (2H, singlet);
4.67 (1H, multiplet);
5.10 (1H, multiplet);
7.32 (5H, multiplet).

Infrared Absorption Spectrum ($CHCl_3$) $\nu_{max}cm^{-1}$:
3450 (-NH-), 1720 (-OCONH).

Elemental Analysis:

| | |
|---|---|
| Calculated for | $C_{30}H_{51}NO_4$: |
| | C, 73.57 %; H, 10.50 %; N, 2.86 %. |
| Found: | C, 73.09 %; H, 10.33 %; N, 2.87 %. |

**Preparation 19**

dl-trans-2-Hydroxymethyltetrahydrofuran-3-yl N-heptadecylcarbamate

1.142 g of dl-trans-2-benzyloxymethyltetrahydrofuran-3-yl N-heptadecylcarbamate (prepared as described in Preparation 18) were subjected to de-benzylation in a similar manner to that described in Preparation 4, to give 0.841 g of the title compound as white crystals melting at 77 - 78° C, after recrystallization from a mixture of diethyl ether and hexane.

Nuclear Magnetic Resonance Spectrum (270MHz, $CDCl_3$) δ ppm:
0.8 - 1.7 (33H, multiplet);
1.95 - 2.25 (2H, multiplet);
2.41 (1H, triplet, J = 6.2Hz);
3.16 (2H, doublet of triplets, $J_1$ = $J_2$ = 6.6Hz);
3.70 (2H, multiplet);
3.80 - 4.10 (3H, multiplet);
4.72 (1H, multiplet);
5.01 (1H, multiplet).

Infrared Absorption Spectrum ($CHCl_3$) $\nu_{max}cm^{-1}$:

3600 (-OH), 3450 (-NH-), 1710 (-OCONH-).

Elemental Analysis:

| | |
|---|---|
| Calculated for | $C_{23}H_{45}NO_4$: |
| | C, 69.13 %; H, 11.35 %; N, 3.50 %. |
| Found: | C, 68.98 %; H, 11.22 %; N, 3.70 %. |

Mass spectrum (m/e): 400 (M+ +1), 399 (M+), 368 (M+ -$OCH_3$).

**Preparation 20**

dl-S-(cis-2-Benzyloxymethyltetrahydropyran-3-yl) N-(heptadecyl)thiocarbamate

A similar reaction and treatment procedure to that described in Preparation 13 was repeated, but using 7.75 g of stearic acid in place of the nonadecanoic acid, to give 5.29 g of the title compound as white crystals melting at 80 - 81°C, after recrystallization from a mixture of diethyl ether and hexane.

Nuclear Magnetic Resonance Spectrum ($CDCl_3$) δ ppm:
0.8 - 2.2 (37H, multiplet);
3.1 - 4.2 (8H, multiplet);
4.57 (2H, AB-quartet, $J_{AB}$ = 12Hz);
5.31 (1H, multiplet);
7.35 (5H, multiplet).

Infrared Absorption Spectrum ($CHCl_3$) $\nu_{max}cm^{-1}$:
3430 (-NH-), 1670 (-SCO-)

Elemental Analysis:

| | |
|---|---|
| Calculated for | $C_{31}H_{53}NO_3S$: |
| | C, 71.63 %; H, 10.28 %; N, 2.69 %; S, 6.17 %. |
| Found: | C, 71.73 %; H, 10.19 %; N, 2.64 %; S, 6.43 %. |

**Preparation 21**

dl-S-(cis-2-Hydroxymethyltetrahydropyran3-yl) N-(heptadecyl)thiocarbamate

5.20 g of dl-S-(cis-2-benzyloxymethyltetrahydropyran-3-yl) N-(heptadecyl)thiocarbamate (prepared as described in Preparation 20) were subjected to similar reactions and treatment to those described in Preparation 14, to give 4.04 g of the title compound as white crystals melting at 90 - 91°C, after recrystallization from hexane.

Nuclear Magnetic Resonance Spectrum ($CDCl_3$) δ ppm:
0.7 - 2.3 (38H, multiplet);
3.15 - 4.15 (8H, multiplet);
5.53 (1H, multiplet).

Infrared Absorption Spectrum ($CHCl_3$) $\nu_{max}cm^{-1}$:
3430 (-NH-, OH), 1650 (-SCO-).

Elemental Analysis:

| | |
|---|---|
| Calculated for | $C_{24}H_{47}NO_3S$: |
| | C, 67.08 %; H, 11.02 %; N, 3.26 %; S, 7.46 %. |
| Found: | C, 67.04 %; H, 10.98 %; N, 3.31 %; S, 7.63 %. |

**Experiment 1**

Inhibition of PAF-induced hypotension

The test animals employed were rats of the Wistar-Imamichi strain, each weighing between 350 and 450 g.

Under Inactin anaesthesia (90 mg/kg intraperitoneally) the left femoral artery and vein of each test animal were cannulated to enable the arterial blood pressure to be monitored continuously and for drug administration, respectively. At intervals of about 5 minutes, each animal was given by intravenous injection 10 ng/kg of synthetic 1-$C_{16:0}$ PAF until a steady hypotensive response was achieved. At this time, the drug to be tested was administered by intravenous injection in doses increasing cumulatively by a factor of 3. Within 1 minute of this injection, a further 10 ng/kg of the 1-$C_{16:0}$ PAF was administered. The hypotensive response to PAF was inhibited by the test drug in a dose-related manner.

The PAF was administered in the form of a solution in physiological saline containing 0.25 % w/v bovine serum albumin. The test drugs were dissolved in physiological saline containing either 0.25 % w/v BSA or 20 % v/v ethanol.

The 50 % inhibitory dose ($ID_{50}$) was calculated from the dose-response curve constructed for the inhibition of PAF-induced hypotension.

This test was carried out using compounds of the invention, as well as the prior art compound CV-3988, disclosed in US Patent No. 4 408 052 and represented by the foregoing formula (B). The results are shown in Table 14.

**Experiment 2**

Inhibition of PAF-induced platelet aggregation in vitro

Blood was drawn from a rabbit and immediately mixed with one nineth of its volume of a 3.8 % w/v aqueous solution of sodium citrate. A platelet-rich plasma (PRP) was obtained as a supernatant by centrifugation of the blood at 150 x g for 15 minutes at room temperature. The precipitated fraction was centrifuged for a further 15 minutes at 1000 x g to obtain a platelet-poor plasma (PPP) as a supernatant. Appropriate proportions of this PRP and PPP were mixed to obtain a plasma having a platelet count of $6 \times 10^5$/µl.

Platelet aggregation was determined by the method of Born et al. [G. V. R. Born et al., J. Physiol., 62, 67 - 68 (1962)] where an increase in light transmission is measured by the aggregometer.

25 µl of a saline solution containing the compound to be tested at an appropriate concentration was added to 250 µl of the above plasma. One minute thereafter, 25 µl of a saline solution of synthetic $C_{16:0}$ PAF (at a concentration of $1 \times 10^{-8}$ - $3 \times 10^{-8}$M) was added and aggregation was observed for 5 minutes. The aggregation resulting from the addition of PAF alone, without the prior addition of the test compound, was taken as 100 %.

The $IC_{50}$ values (i. e. concentrations to inhibit aggregation by 50 %) were calculated from dose-response curves and are shown in Table 14.

Table 14

| Test compound | Inhibition of Hypotension $ID_{50}$ (mg/kg) | Inhibition of Platelet aggregation $IC_{50}$ (M) |
|---|---|---|
| Cpd. of Ex. 1 | 0.57 | $1.5 \times 10^{-6}$ |
| Cpd. of Ex. 3 | 0.46 | $4.3 \times 10^{-6}$ |
| Cpd. of Ex. 4 | 0.09 | $8.4 \times 10^{-6}$ |
| Cpd. of Ex. 6 | 0.08 | $1.8 \times 10^{-5}$ |
| CV - 3988 | 0.42 | $9.8 \times 10^{-6}$ |

**Experiment 3**

Reversal of endotoxin-induced hypotension (Anti-endotoxin shock)

The test animals used were male rats of the Sprague-Dawley strain with a body weight of 250 - 350 g. They were anaesthetized by the intraperitoneal administration of 60 mg/kg of pentobarbital sodium. The mean arterial blood pressure was recorded via a cannula inserted into the femoral artery, whilst endotoxin and the test compounds were administered through a cannula inserted into the femoral vein.

Endotoxin (5 mg/kg) was administered to each rat. When the maximum reduction in blood pressure was observed (after about 4 minutes), the test compound was administered.

The anti-endotoxin shock activity of the test compound at each dose was calculated from:

$$[(C - B)/(A - B)] \times 100 \%$$

where:
A = blood pressure just before injection of endotoxin;
B = blood pressure 4 minutes after injection of endotoxin; and
C = blood pressure 1 minute after injection of test compound.

The experiment was repeated for each test compound at several doses and $ED_{50}$ values were calculated from the dose-response curves. The results are shown in Table 15.

Endotoxin (Lipopolysaccharide W E. coli 0127 : B8) and the test compounds were dissolved in physiological saline just before use.

Table 15

| Test compound | $ED_{50}$ (mg/kg, i. v.) |
|---|---|
| Cpd. of Ex. 1 | 0.14 |
| Cpd. of Ex. 6 | 0.058 |
| CV-3988 | 0.24 |

**Experiment 4**

Anti-inflammatory effect

This test was carried out by the carrageenin oedema method [C. A. Winter, E. A. Risley, G. W. Ness, J. Pharmacol. Exp. Therap., 141, 369 (1963)] using rats of the Wistar strain and employing the compound of Example 2 at a dose of 50 mg/kg per os. The inhibition rate was 65.9 %.

**Claims** for the Contracting States: BE, CH, DE, FR, GB, IT, LI, LU, NL, SE

1. Compounds of formula (I):

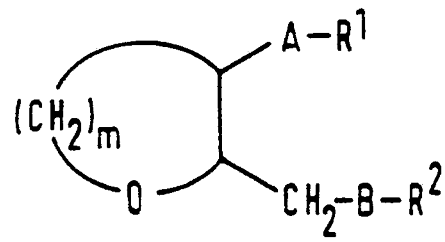

(I)

in which:
m is an integer of from 2 to 4;
A and a are the same or different and each represents an oxygen atom or a sulphur atom; and
one of $R^1$ and $R^2$ represents a group of formula $-CONH-R^3$, where $R^3$ represents a $C_{10}$-$C_{22}$ alkyl group, and the other represents a group of formula (II):

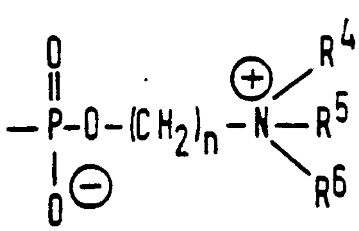

(II)

in which:
n represents an integer of from 2 to 10; and
$R^4$, $R^5$ and $R^6$ are the same or different and each represents a hydrogen atom or a $C_1$-$C_6$ alkyl group, or $R^4$ and $R^5$ or $R^4$, $R^5$ and $R^6$ together with the nitrogen atom to which they are attached, form a heterocyclic ring; and pharmaceutically acceptable salts thereof.

2. Compounds as claimed in Claim 1, wherein m is 2 or 3.

3. Compounds as claimed in Claim 1 or claim 2, wherein n is an integer from 2 to 6.

4. Compounds as claimed in Claim 1 or Claim 2, wherein n is 2 or 3.

5. Compounds as claimed in any one of Claims 1 to 4, wherein $R^4$, $R^5$ and $R^6$ are the same or different and each represents a hydrogen atom or a $C_1$-$C_6$ alkyl group.

6. Compounds as claimed in any one of Claims 1 to 4, wherein $R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group.

7. Compounds as claimed in any one of Claims 1 to 4, wherein $R^4$ and $R^5$, $R^6$ together with the nitrogen atom to which they are attached, represent an aromatic heterocyclic group having from 5 to 10 ring atoms, said heterocyclic group being unsubstituted or having at least one $C_1$-$C_5$ alkyl, $C_1$-$C_5$ alkoxy, $C_1$-$C_5$ hydroxyalkyl, carbamoyl or halogen substituent.

8. Compounds as claimed in any one of Claims 1 to 7, wherein $R^1$ represents said group of formula $-CONHR^3$ and $R^2$ represents said group of formula (II).

9. Compounds as claimed in any one of Claims 1 to 8, wherein $R^3$ represents an alkyl group having from 16 to 18 carbon atoms.

10. Compounds as claimed in Claim 1, wherein:
$R^1$ represents said group of formula $-CONHR^3$;
$R^2$ represents said group of formula (II);
$R^4$, $R^5$ and $R^6$ are the same or different and each represents a hydrogen atom or a $C_1$-$C_6$ alkyl group;
$\underline{n}$ is an integer from 2 to 6; and
$\underline{m}$ is 2 or 3.

11. Compounds as claimed in Claim 1, wherein:
$R^1$ represents said group of formula $-CONHR^3$;
$R^2$ represents said group of formula (II);
$R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group;
$\underline{n}$ is an integer from 2 to 6; and
$\underline{m}$ is 2 or 3.

12. Compounds as claimed in Claim 1, wherein:
$R^1$ represents said group of formula $-CONHR^3$;
$R^2$ represents said group of formula (II);
$R^4$, $R^5$ and $R^6$, together with the nitrogen atom to which they are attached, represent an aromatic heterocyclic group having from 5 to 10 ring atoms, said group being unsubstituted or having at least one $C_1$-$C_5$ alkyl, $C_1$-$C_5$ alkoxy, $C_1$-$C_5$ hydroxyalkyl, carbamoyl or halogen substituent;
$\underline{n}$ is an integer from 2 to 6; and
$\underline{m}$ is 2 or 3.

13. Compounds as claimed in Claim 1, wherein:
$R^1$ represents a group of formula $-CONHR^3$, where $R^3$ represents a $C_{16}$-$C_{18}$ alkyl group;
$R^2$ represents said group of formula (II);
$R^4$, $R^5$ and $R^6$ are the same or different and each represents a $C_1$-$C_6$ alkyl group;
$\underline{n}$ is an integer from 2 to 6; and
$\underline{m}$ is 2 or 3.

14. Compounds as claimed in Claim 1, wherein:
$R^1$ represents a group of formula $-CONHR^3$, where $R^3$ represents a $C_{16}$-$C_{18}$ alkyl group;
$R^2$ represents said group of formula (II);
$R^4$ and $R^5$, together with the nitrogen atom to which they are attached, represent a non-aromatic heterocyclic group having from 5 to 7 ring atoms, and
$R^6$ represents a hydrogen atom or a $C_1$-$C_6$ alkyl group;
$\underline{n}$ is an integer from 2 to 6; and
$\underline{m}$ is 2 or 3.

15. Compounds as claimed in Claim 1, wherein:
$R^1$ represents a group of formula $-CONHR^3$, where $R^3$ represents a $C_{16}$-$C_{18}$ alkyl group;
$R^2$ represents said group of formula (II);
$R^4$ and $R^5$, $R^6$ together with the nitrogen atom to which they are attached, represent an aromatic heterocyclic group having from 5 to 7 ring atoms, said group being unsubstituted or having at least one $C_1$-$C_5$ alkyl or $C_1$-$C_5$ hydroxyalkyl substituent;
$\underline{n}$ is an integer from 2 to 6; and
$\underline{m}$ is 2 or 3.

16. Compounds as claimed in Claim 1, wherein:
$R^1$ represents a group of formula $-CONHR^3$, where $R^3$ represents a $C_{16}$-$C_{18}$ alkyl group;
$R^2$ represents said group of formula (II);
$R^4$ and $R^5$, together with the nitrogen atom to which they are attached, represent a piperidino or 1-pyrrolidinyl group, and
$R^6$ represents a hydrogen atom or a $C_1$-$C_6$ alkyl group;
$\underline{n}$ is an integer from 2 to 6; and
$\underline{m}$ is 2 or 3.

17. Compounds as claimed in Claim 1, wherein:

$R^1$ represents a group of formula $-CONHR^3$, where $R^3$ represents a $C_{16}$-$C_{18}$ alkyl group;
$R^2$ represents said group of formula (II);
$R^4$, $R^5$ and $R^6$, together with the nitrogen atom to which they are attached, represent a 1-pyridyl or 1-thiazolyl group which is unsubstituted or has at least one $C_1$-$C_5$ alkyl or $C_1$-$C_5$ hydroxyalkyl substituent;
$\underline{n}$ is an integer from 2 to 6; and
$\underline{m}$ is 2 or 3.

18. A compound of formula:

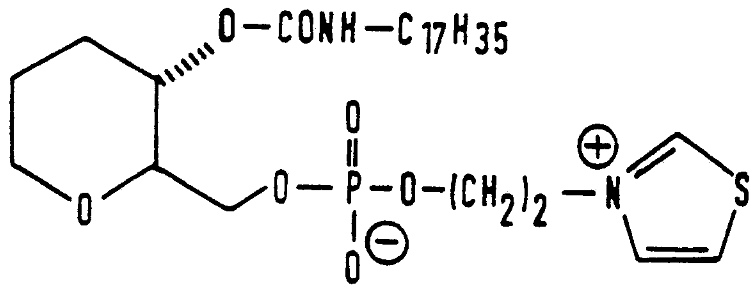

(b)

its mirror image and pharmaceutically acceptable salts of said compound and of its mirror image.

19. A compound of formula:

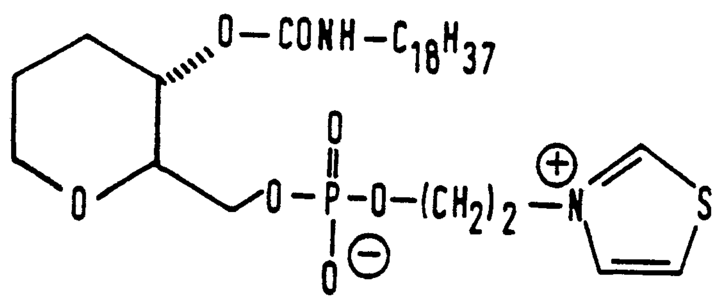

(c)

its mirror image and pharmaceutically acceptable salts of said compound and of its mirror image.

20. A compound of formula:

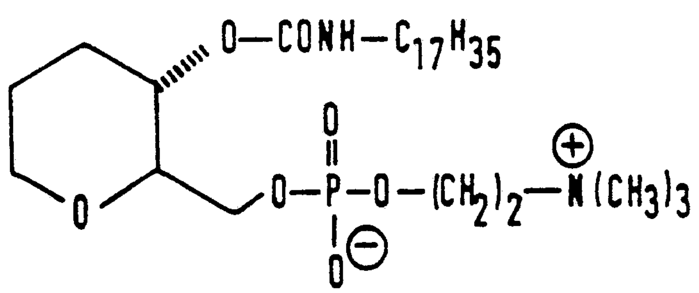

(d)

its mirror image and pharmaceutically acceptable salts of said compound and of its mirror image.

21. A compound of formula:

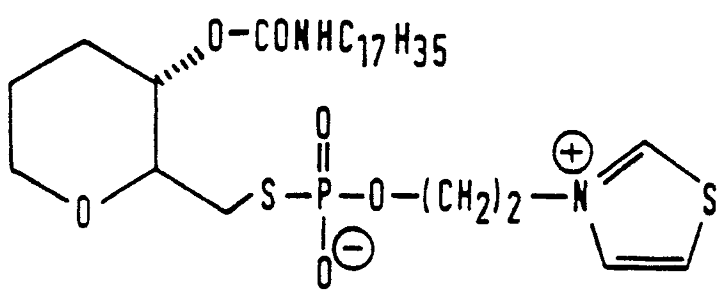

(g)

its mirror image and pharmaceutically acceptable salts of said compound and of its mirror image.

22. A compound of formula:

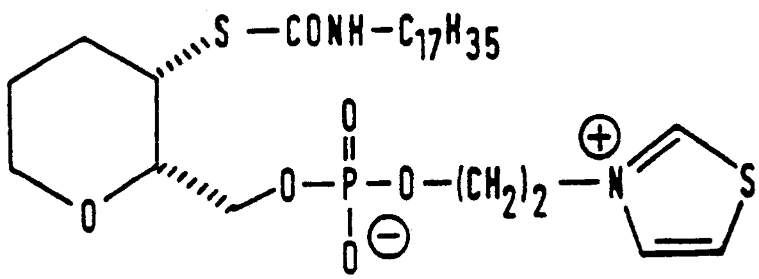

(j)

its mirror image and pharmaceutically acceptable salts of said compound and of its mirror image.

23. A compound of formula:

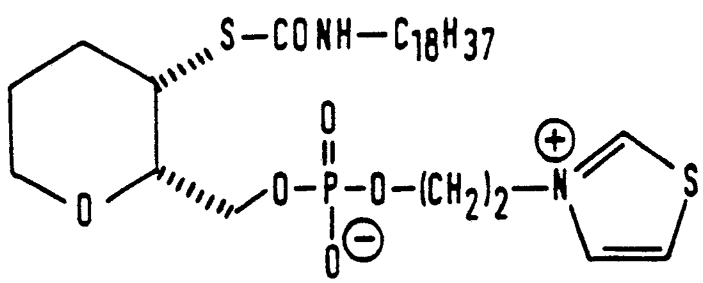

(k)

its mirror image and pharmaceutically acceptable salts of said compound and of its mirror image.

24. A compound of formula:

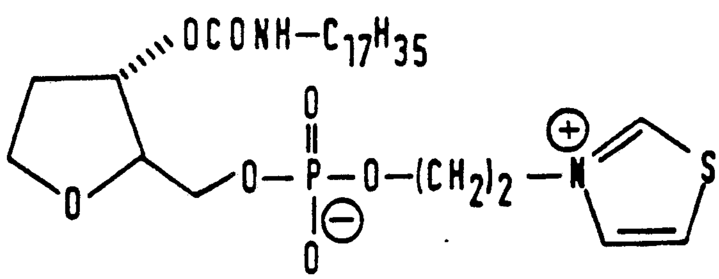

(p)

its mirror image and pharmaceutically acceptable salts of said compound and of its mirror image.

25. A compound of formula:

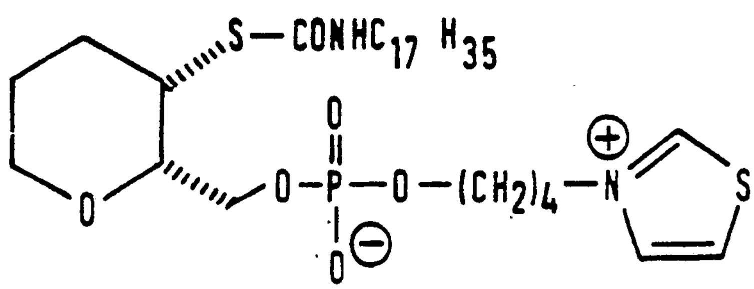

(u)

its mirror image and pharmaceutically acceptable salts of said compound and of its mirror image.

26. A compound of formula:

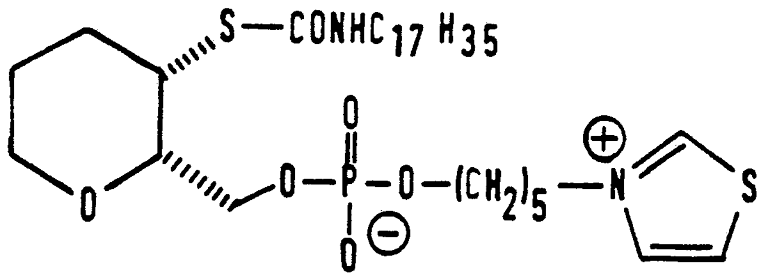

(v)

its mirror image and pharmaceutically acceptable salts of said compound and of its mirror image.

27. A compound of formula:

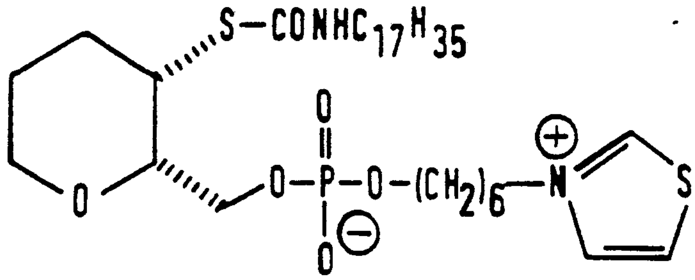

(w)

its mirror image and pharmaceutically acceptable salts of said compound and of its mirror image.

28. A compound of formula:

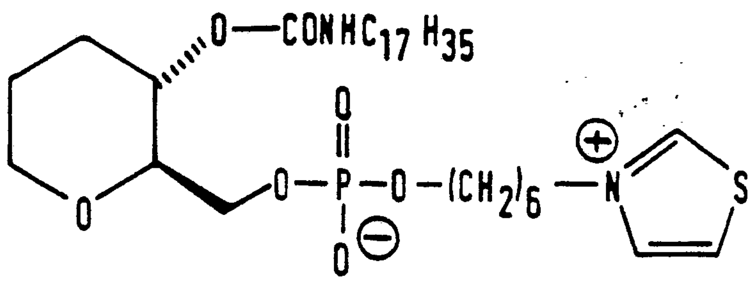

(x)

its mirror image and pharmaceutically acceptable salts of said compound and of its mirror image.

29. A compound of formula:

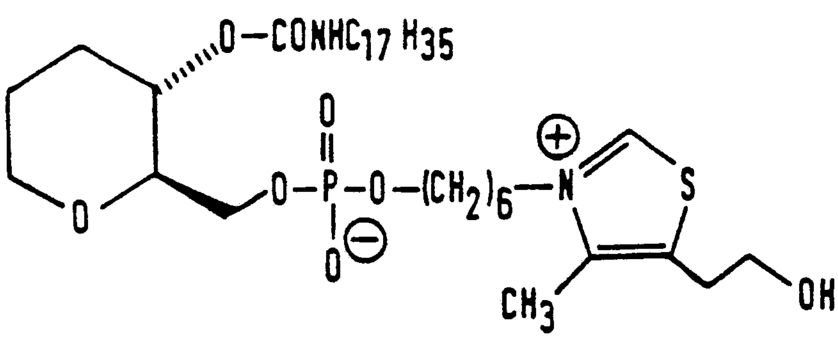

(y)

its mirror image and pharmaceutically acceptable salts of said compound and of its mirror image.

30. A pharmaceutical composition for the treatment of asthma, inflammation or shock comprising a PAF antagonist in combination with a pharmaceutically acceptable carrier or diluent, wherein the PAF antagonist is at least one compound as claimed in any one of the preceding Claims.

31. The use for the manufacture of a medicament for the treatment of asthma, inflammation or shock of a compound as claimed in any one of Claims 1 to 29.

**Claims** for the Contracting State: AT

1. A process for preparing compounds of formula (I):

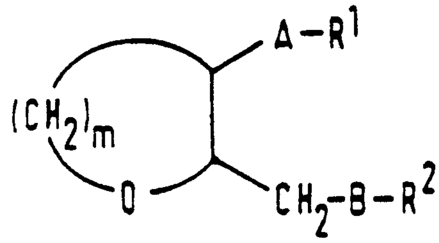

(I)

[in which:
m is an integer of from 2 to 4;
A and B are the same or different and each represents an oxygen atom or a sulphur atom; and
one of $R^1$ and $R^2$ represents a group of formula $-CONH-R^3$, where $R^3$ represents a $C_{10}$-$C_{22}$ alkyl group, and the other represents a group of formula (II):

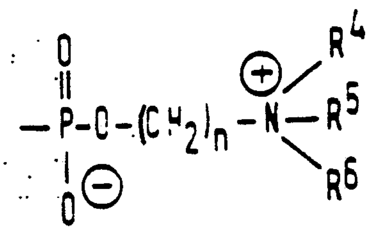

(II)

in which:
n represents an integer of from 2 to 10; and
$R^4$, $R^5$ and $R^6$ are the same or different and each represents a hydrogen atom or a $C_1$-$C_6$ alkyl group, or $R^4$ and $R^5$ or $R^4$, $R^5$ and $R^6$, together with the nitrogen atom to which they are attached, form a heterocyclic ring;]
and pharmaceutically acceptable salts thereof, by reacting a compound of formula (III):

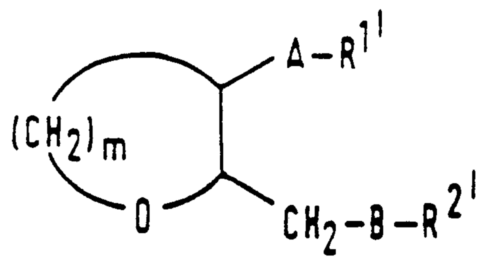

(III)

in which m A and B are as defined above; and
one of $R^{1'}$ and $R^{2'}$ represents said group of formula $-CONH-R^3$, defined above, and the other represents a group of formula (IV):

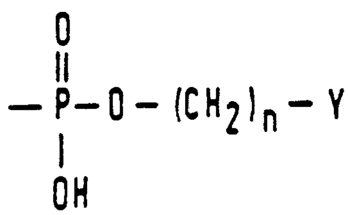

(IV)

(in which n is as defined above and Y represents a halogen atom) or a group of formula (V):

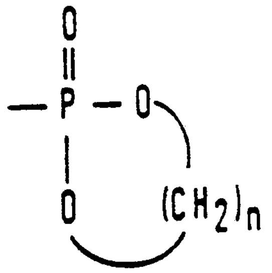

(V)

(in which n is as defined above) with an amine compound of formula (VI):

$R^4NR^5R^6$ (VI)

(in which $R^4$, $R^5$ and $R^6$ are as defined above), and optionally salifying the product.

2. A process as claimed in Claim 1, wherein in said compound of formula (III) m is 2 or 3.

3. A process as claimed in Claim 1 or Claim 2, wherein in said group of formula (IV) or (V) n is an integer from 2 to 6.

4. A process as claimed in Claim 1 or Claim 2, wherein in said group of formula (IV) or (V) n is 2 or 3.

5. A process as claimed in any one of the preceding Claims, wherein in said compound of formula (VI) $R^4$, $R^5$ and $R^6$ are the same or different and each represents a hydrogen atom or a $C_1$-$C_6$ alkyl group.

6. A process as claimed in any one of Claims 1 to 4, wherein in said compound of formula (VI) $R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group.

7. A process as claimed in any one of Claims 1 to 4, wherein in said compound of formula (VI) $R^4$, $R^5$ and $R^6$, together with the nitrogen atom to which they are attached, represent an aromatic heterocyclic group having from 5 to 10 ring atoms, said heterocyclic group being unsubstituted or having at least one $C_1$-$C_5$ alkyl, $C_1$-$C_5$ alkoxy, $C_1$-$C_5$ hydroxyalkyl, carbamoyl or halogen substituent.

8. A process as claimed in any one of the preceding Claims, wherein $R^{1'}$ represents said group of formula $-CONHR^3$ and $R^{2'}$ represents said group of formula (IV) or (V).

9. A process as claimed in any one of the preceding Claims, wherein $R^3$ in said compound of formula (III) represents an alkyl group having from 16 to 18 carbon atoms.

10. A process as claimed in Claim 1, wherein:

$R^{1'}$ represents said group of formula $-CONHR^3$;

$R^{2'}$ represents said group of formula (IV) or (V);

$R^4$, $R^5$ and $R^6$ in said compound of formula (VI) are the same or different and each represents a hydrogen atom or a $C_1$-$C_6$ alkyl group;

n in said group of formula (IV) or (V) is an integer from 2 to 6; and

m in said compound of formula (III) is 2 or 3.

11. A process as claimed in Claim 1, wherein:

$R^{1'}$ represents said group of formula $-CONHR^3$;

$R^{2'}$ represents said group of formula (IV) or (V);

in said compound of formula (VI) $R^4$ and $R^5$, together with the nitrogen atom to which they are attached, represent a non-aromatic heterocyclic group having from 5 to 7 ring atoms, and $R^6$ represents a hydrogen atom or a $C_1$-$C_6$ alkyl group;

n in said group of formula (IV) or (V) is an integer from 2 to 6; and

m in said compound of formula (III) is 2 or 3.

12. A process as claimed in Claim 1, wherein:

$R^{1'}$ represents said group of formula $-CONHR^3$;

$R^{2'}$ represents said group of formula (IV) or (V);

in said compound of formula (VI) $R^4$, $R^5$ and $R^6$, together with the nitrogen atom to which they are attached, represent an aromatic heterocyclic group having from 5 to 10 ring atoms, said group being unsubstituted or having at least one $C_1$-$C_5$ alkyl, $C_1$-$C_5$ alkoxy, $C_1$-$C_5$ hydroxyalkyl, carbamoyl or halogen substituent;

n in said group of formula (IV) or (V) is an integer from 2 to 6; and

m in said compound of formula (III) is 2 or 3.

13. A process as claimed in Claim 1, wherein:

$R^{1'}$ represents a group of formula $-CONHR^3$, where $R^3$ represents a $C_{16}$-$C_{18}$ alkyl group;

$R^2$ represents said group of formula (IV) or (V);

in said compound of formula (VI) $R^4$, $R^5$ and $R^6$ are the same or different and each represents a $C_1$-$C_6$ alkyl group;

n in said group of formula (IV) or (V) is an integer from 2 to 6; and

m in said compound of formula (III) is 2 or 3.

14. A process as claimed in Claim 1, wherein:
$R^{1'}$ represents a group of formula $-CONHR^3$, where $R^3$ represents a $C_{16}$-$C_{18}$ alkyl group;
$R^{2'}$ represents said group of formula (IV) or (V);
in said compound of formula (VI) $R^4$ and $R^5$, together with the nitrogen atom to which they are attached, represent a non-aromatic heterocyclic group having from 5 to 7 ring atoms, and $R^6$ represents a hydrogen atom or a $C_1C_6$ alkyl group;
n in said group of formula (IV) or (V) is an integer from 2 to 6; and
m in said compound of formula (III) is 2 or 3.

15. A process as claimed in Claim 1, wherein:
$R^{1'}$ represents a group of formula $-CONHR^3$, where $R^3$ represents a $C_{16}$-$C_{18}$ alkyl group;
$R^{2'}$ represents said group of formula (IV) or (V);
in said compound of formula (VI) $R^4$, $R^5$ and $R^6$, together with the nitrogen atom to which they are attached, represent an aromatic heterocyclic group having from 5 to 7 ring atoms, said group being unsubstituted or having at least one $C_1$-$C_5$ alkyl or $C_1$-$C_5$ hydroxyalkyl substituent;
n in said group of formula (IV) or (V) is an integer from 2 to 6; and
m in said compound of formula (III) is 2 or 3.

16. A process as claimed in Claim 1, wherein:
$R^1$ represents a group of formula $-CONHR^3$ where $R^3$ represents a $C_{16}$-$C_{18}$ alkyl group;
$R^{2'}$ represents said group of formula (IV) or (V);
in said compound of formula (VI) $R^4$ and $R^5$, together with the nitrogen atom to which they are attached, represent a piperidino or 1-pyrrolidinyl group, and $R^6$ represents a hydrogen atom or a $C_1$-$C_6$ alkyl group;
n in said group of formula (IV) or (V) is an integer from 2 to 6; and
m in said compound of formula (III) is 2 or 3.

17. A process as claimed in Claim 1, wherein:
$R^{1'}$ represents a group of formula $-CONHR^3$, where $R^3$ represents a $C_{16}$-$C_{18}$ alkyl group;
$R^{2'}$ represents said group of formula (IV) or (V);
in said compound of formula (VI) $R^4$, $R^5$ and $R^6$, together with the nitrogen atom to which they are attached, represent a 1-pyridyl or 1-thiazolyl group which is unsubstituted or has at least one $C_1$-$C_5$ alkyl or $C_1$-$C_5$ hydroxyalkyl substituent;
n in said group of formula (IV) or (V) is an integer from 2 to 6; and
m in said compound of formula (III) is 2 or 3.

18. A process as claimed in Claim 1, wherein the reagents and reaction conditions are so selected as to produce a compound of formula:

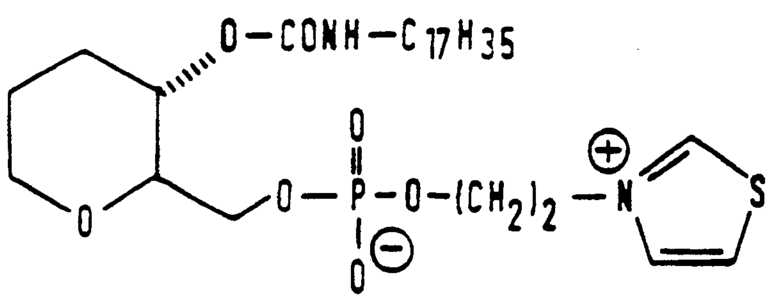

(b)

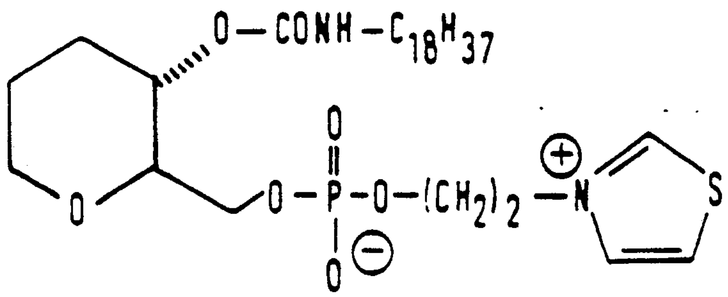

(c)

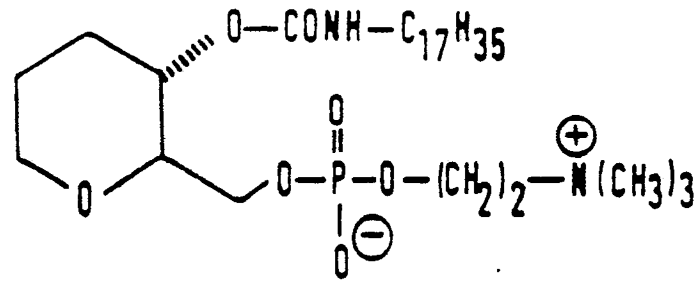

(d)

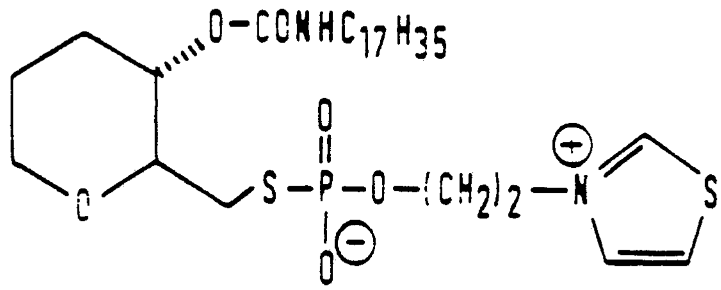

(g)

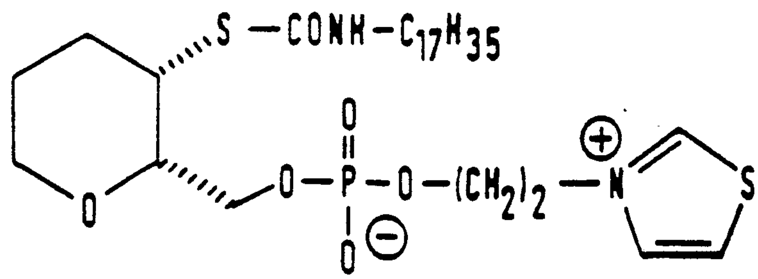

(j)

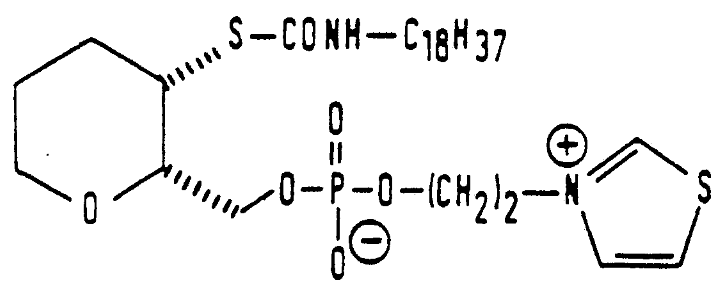

(k)

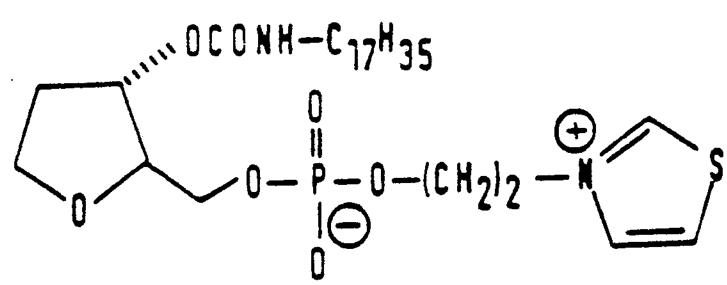

(p)

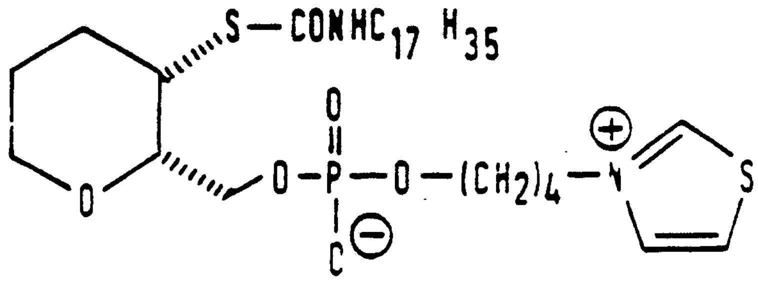

(u)

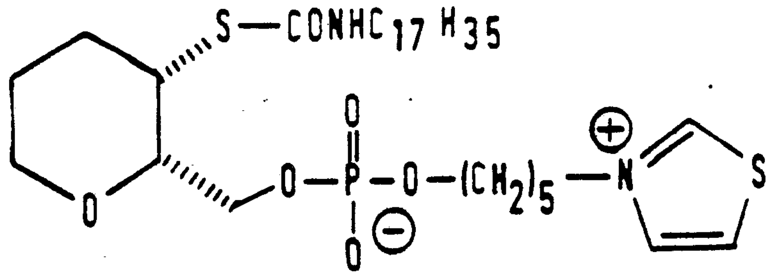

(v)

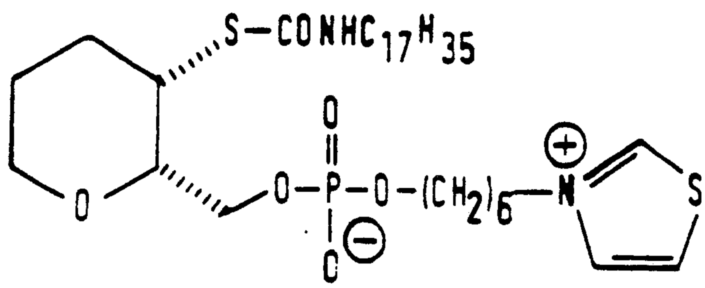

(w)

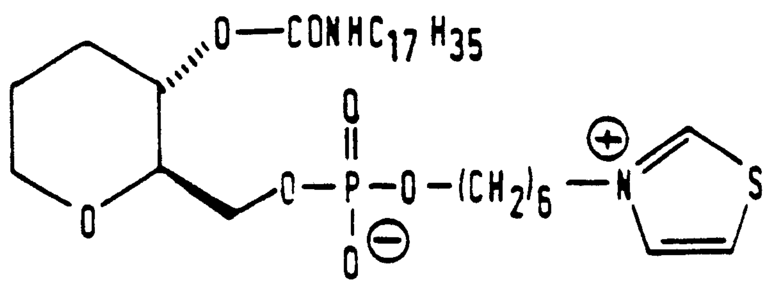

(x)

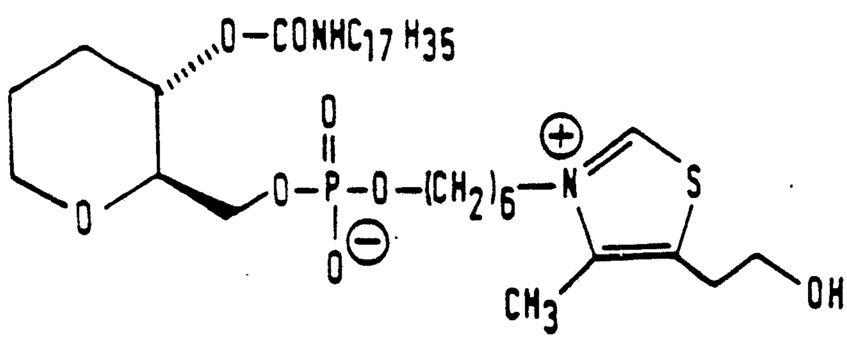

(y)

or the mirror image of said compounds or pharmaceutically acceptable salts of said compounds or of their mirror images.

19. The use for the manufacture of a medicament for the treatment of asthma, inflammation or shock of a compound of formula (I) or a pharmaceutically acceptable salt thereof, as defined in Claim 1.

20. The use for the manufacture of a medicament for the treatment of asthma, inflammation or shock of a compound of formula (b), (c), (d), (g), (j), (k), (p), (u), (v), (w), (x) or (y), as shown in claim 18, or of a mirror image thereof or of a pharmaceutically acceptable salt of said compound or of its mirror image.

**Patentansprüche** für die Vertragsstaaten: BE, CH, DE, FR, GB, IT, LI, LU, NL, SE

1. Verbindungen der Formel (I):

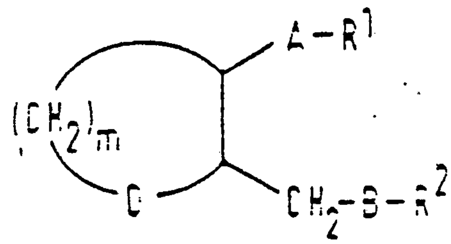

(I)

in welcher:

m eine ganze Zahl von 2 bis 4 bedeutet;

A und B untereinander gleich oder voneinander verschieden sind und je für sich ein Sauerstoffatom oder ein Schwefelatom bedeuten; und

einer der Reste $R^1$ und $R^2$ für eine Gruppe der Formel $-CONH-R^3$ mit der Bedeutung einer $C_{10}$-$C_{22}$-Alkylgruppe für $R^3$ steht und der andere dieser Reste eine Gruppe der Formel (II):

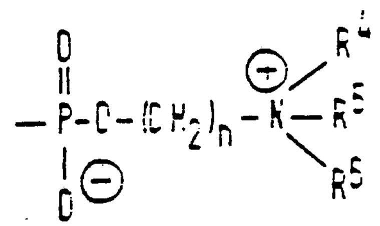

(II)

darstellt, in welcher:

n eine ganze Zahl von 2 bis 10 bedeutet; und

$R^4$, $R^5$ und $R^6$ untereinander gleich oder voneinander verschieden sind und je für sich ein Wasserstoffatom oder eine $C_1$-$C_6$-Alkylgruppe bedeuten oder $R^4$ und $R^5$ oder $R^4$, $R^5$ und $R^6$ zusammen mit dem diese Reste tragenden Stickstoffatom einen heterocyclischen Ring bilden;

und pharmazeutisch annehmbare Salze hievon.

2. Verbindungen nach Anspruch 1, worin m gleich ist 2 oder 3.

3. Verbindungen nach Anspruch 1 oder Anspruch 2, worin n eine ganze Zahl von 2 bis 6 bedeutet.

4. Verbindungen nach Anspruch 1 oder Anspruch 2, worin n gleich ist 2 oder 3.

5. Verbindungen nach irgendeinem der Ansprüche 1 bis 4, worin $R^4$, $R^5$ und $R^6$ untereinander gleich oder voneinander verschieden sind und je für sich ein Wasserstoffatom oder eine $C_1$-$C_6$-Alkylgruppe bedeuten.

6. Verbindungen nach irgendeinem der Ansprüche 1 bis 4, worin $R^4$ und $R^5$ zusammen mit dem diese Reste tragenden Stickstoffatom eine nicht-aromatische heterocyclische Gruppe mit 5 bis 7 Ringatomen bedeuten und $R^6$ ein Wasserstoffatom oder eine $C_1$-$C_6$-Alkylgruppe bedeutet.

7. Verbindungen nach irgendeinem der Ansprüche 1 bis 4, worin $R^4$, $R^5$ und $R^6$ zusammen mit dem diese Reste tragenden Stickstoffatom eine aromatische heterocyclische Gruppe mit 5 bis 10 Ringatomen bedeuten, wobei diese heterocyclische Gruppe unsubstituiert ist oder zumindest eine $C_1$-$C_5$-Alkyl-, $C_1$-$C_5$-Alkoxy-, $C_1$-$C_5$-Hydroxyalkyl-Carbamoyl- oder Halogensubstituenten aufweist.

8. Verbindungen nach irgendeinem der Ansprüche 1 bis 7, worin $R^1$ die erwähnte Gruppe der Formel $-CONHR^3$ und $R^2$ die erwähnte Gruppe der Formel (II) bedeutet.

9. Verbindungen nach irgendeinem der Ansprüche 1 bis 8, worin $R^3$ eine Alkylgruppe mit 16 bis 18 Kohlenstoffatomen bedeutet.

10. Verbindungen nach Anspruch 1, worin:

$R^1$ die Gruppe der Formel $-CONHR^3$ bedeutet;

$R^2$ die Gruppe der Formel (II) darstellt;

$R^4$, $R^5$ und $R^6$ untereinander gleich oder voneinander verschieden sind und je für sich ein Wasserstoffatom oder eine $C_1$-$C_6$-Alkylgruppe bedeuten;

n eine ganze Zahl von 2 bis 6 bedeutet; und
m gleich ist 2 oder 3.

11. Verbindungen nach Anspruch 1, worin:
$R^1$ die Gruppe der Formel -CONHR$^3$ bedeutet;
$R^2$ die Gruppe der Formel (II) darstellt;
$R^4$ und $R^5$ zusammen mit dem diese Reste tragenden Stickstoffatom eine nicht-aromatische heterocyclische Gruppe mit 5 bis 7 Ringatomen darstellen und $R^6$ ein Wasserstoffatom oder eine $C_1$-$C_6$-Alkylgruppe bedeutet;
n eine ganze Zahl von 2 bis 6 bedeutet; und
m gleich ist 2 oder 3.

12. Verbindungen nach Anspruch 1, worin:
$R^1$ die Gruppe der Formel -CONHR$^3$ bedeutet;
$R^2$ die Gruppe der Formel (II) darstellt;
$R^4$, $R^5$ und $R^6$ zusammen mit dem diese Reste tragenden Stickstoffatom eine aromatische heterocyclische Gruppe mit 5 bis 10 Ringatomen bilden, wobei diese Gruppe unsubstituiert ist oder zumindest einen $C_1$-$C_5$-Alkyl-, $C_1$-$C_5$-Alkoxy-, $C_1$-$C_5$-Hydroxyalkyl-, Carbamoyl- oder Halogensubstituenten aufweist;
n eine ganze Zahl von 2 bis 6 bedeutet; und
m gleich ist 2 oder 3.

13. Verbindungen nach Anspruch 1, worin:
$R^1$ eine Gruppe der Formel -CONHR$^3$ mit der Bedeutung einer $C_{16}$-$C_{18}$-Alkylgruppe für $R^3$ darstellt;
$R^2$ die Gruppe der Formel (II) bedeutet;
$R^4$, $R^5$ und $R^6$ untereinander gleich oder voneinander verschieden sind und je für sich eine $C_1$-$C_6$-Alkylgruppe bedeuten;
n eine ganze Zahl von 2 bis 6 bedeutet; und
m gleich ist 2 oder 3.

14. Verbindungen nach Anspruch 1, worin:
$R^1$ eine Gruppe der Formel -CONHR$^3$ mit der Bedeutung einer $C_{16}$-$C_{18}$-Alkylgruppe für $R^3$ darstellt;
$R^2$ die Gruppe der Formel (II) bedeutet;
$R^4$ und $R^5$ zusammen mit dem diese Reste tragenden Stickstoffatom eine nicht-aromatische heterocyclische Gruppe mit 5 bis 7 Ringatomen bilden und $R^6$ ein Wasserstoffatom oder eine $C_1$-$C_6$-Alkylgruppe darstellt;
n eine ganze Zahl von 2 bis 6 bedeutet; und
m gleich ist 2 oder 3.

15. Verbindungen nach Anspruch 1, worin:
$R^1$ eine Gruppe der Formel -CONHR$^3$ mit der Bedeutung einer $C_{16}$-$C_{18}$-Alkylgruppe für $R^3$ darstellt;
$R^2$ die Gruppe der Formel (II) bedeutet;
$R^4$, $R^5$ und $R^6$ zusammen mit dem diese Reste tragenden Stickstoffatom eine aromatische heterocyclische Gruppe mit 5 bis 7 Ringatomen bilden, wobei diese Gruppe unsubstituiert ist oder zumindest einen $C_1$-$C_5$-Alkyl- oder $C_1$-$C_5$-Hydroxyalkyl-substituenten aufweist;
n eine ganze Zahl von 2 bis 6 bedeutet; und
m gleich ist 2 oder 3.

16. Verbindungen nach Anspruch 1, worin:
$R^1$ eine Gruppe der Formel -CONHR$^3$ mit der Bedeutung einer $C_{16}$-$C_{18}$-Alkylgruppe für $R^3$ darstellt;
$R^2$ eine Gruppe der Formel (II) bedeutet;
$R^4$ und $R^5$ zusammen mit dem diese Reste tragenden Stickstoffatom eine Piperidino- oder 1-Pyrrolidinylgruppe bilden und $R^6$ ein Wasserstoffatom oder eine $C_1$-$C_6$-Alkylgruppe bedeutet;
n eine ganze Zahl von 2 bis 6 bedeutet; und
m gleich ist 2 oder 3.

17. Verbindungen nach Anspruch 1, worin:
$R^1$ eine Gruppe der Formel -CONHR$^3$ mit der Bedeutung einer $C_{16}$-$C_{18}$-Alkylgruppe für $R^3$ darstellt;
$R^2$ eine Gruppe der Formel (II) bedeutet;
$R^4$, $R^5$ und $R^6$ zusammen mit dem diese Reste tragenden Stickstoffatom eine 1-Pyridyl- oder 1-Thiazolylgruppe bilden, welche unsubstituiert ist oder als Substituenten zumindest eine $C_1$-$C_5$-Alkyl- oder $C_1$-$C_5$-Hydroxyalkylgruppe aufweist;
n eine ganze Zahl von 2 bis 6 bedeutet; und
m gleich ist 2 oder 3.

18. Verbindung der Formel:

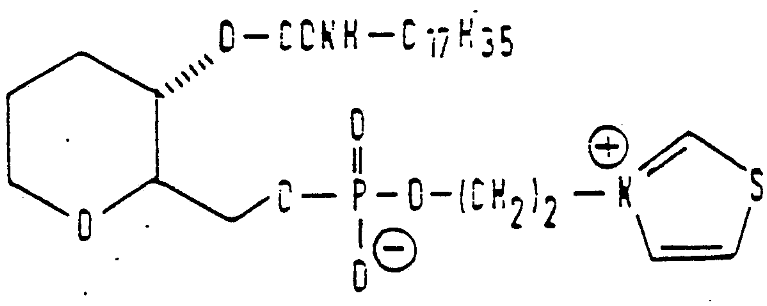

(b)

und ihr Spiegelbild und pharmazeutisch annehmbare Salze dieser Verbindung und ihres Spiegelbildes.

19. Verbindung der Formel:

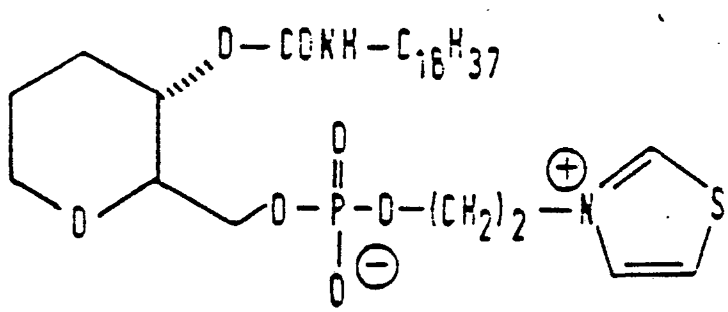

(c)

und ihr Spiegelbild und pharmazeutisch annehmbare Salze dieser Verbindung und ihres Spiegelbildes.

20. Verbindung der Formel:

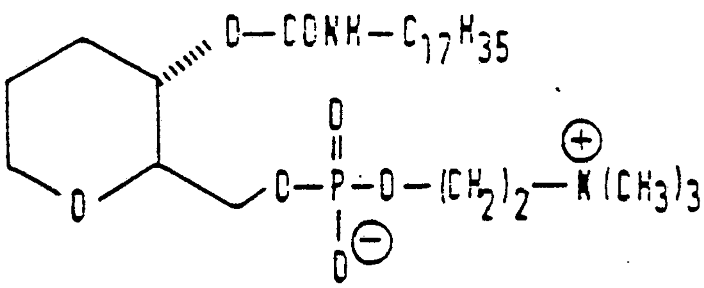

(d)

und ihr Spiegelbild und pharmazeutisch annehmbare Salze dieser Verbindung und ihres Spiegelbildes.

21. Verbindung der Formel:

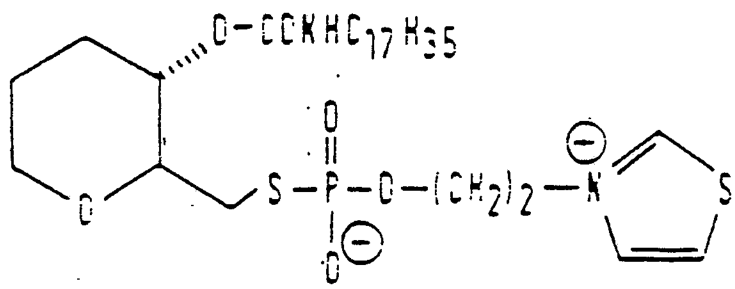

(g)

und ihr Spiegelbild und pharmazeutisch annehmbare Salze dieser Verbindung und ihres Spiegelbildes.

22. Verbindung der Formel:

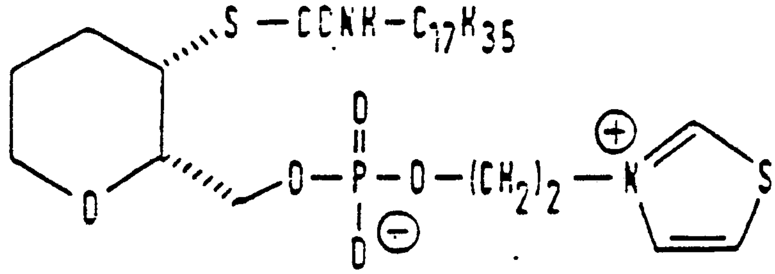

(j)

und ihr Spiegelbild und pharmazeutisch annehmbare Salze dieser Verbindung und ihres Spiegelbildes.

23. Verbindung der Formel:

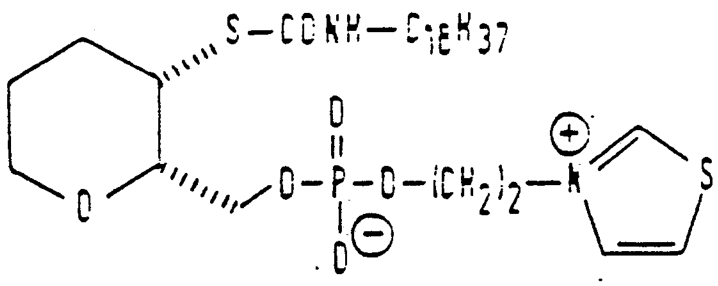

(k)

und ihr Spiegelbild und pharmazeutisch annehmbare Salze dieser Verbindung und ihres Spiegelbildes.

24. Verbindung der Formel:

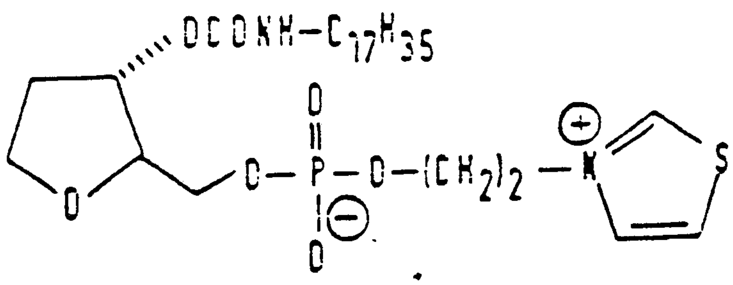

(r)

und ihr Spiegelbild und pharmazeutisch annehmbare Salze dieser Verbindung und ihres Spiegelbildes.

25. Verbindung der Formel:

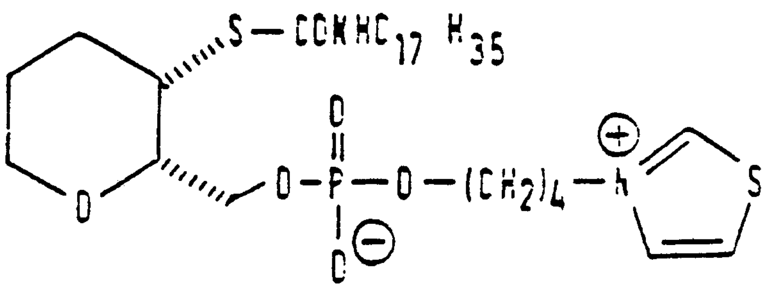

(u)

und ihr Spiegelbild und pharmazeutisch annehmbare Salze dieser Verbindung und ihres Spiegelbildes.

26. Verbindung der Formel:

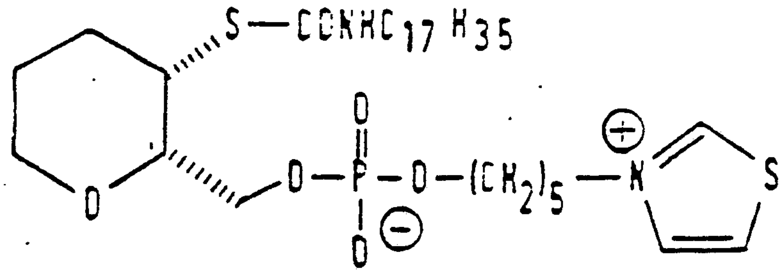

(V)

und ihr Spiegelbild und pharmazeutisch annehmbare Salze dieser Verbindung und ihres Spiegelbildes.

27. Verbindung der Formel:

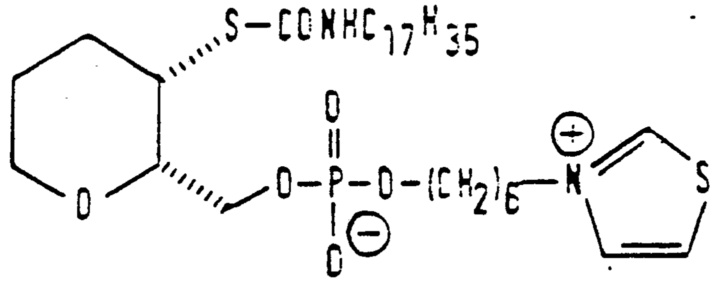

(W)

und ihr Spiegelbild und pharmazeutisch annehmbare Salze dieser Verbindung und ihres Spiegelbildes.

28. Verbindung der Formel:

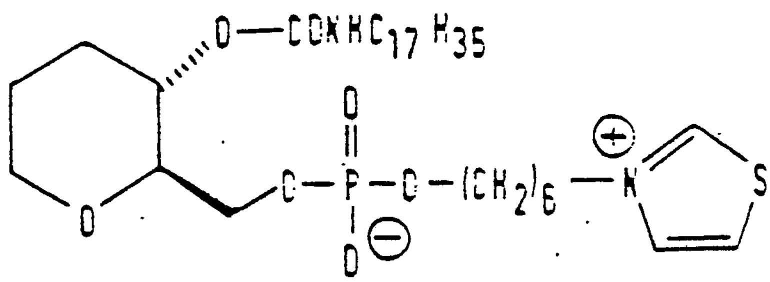

(X)

und ihr Spiegelbild und pharmazeutisch annehmbare Salze dieser Verbindung und ihres Spiegelbildes.

29. Verbindung der Formel:

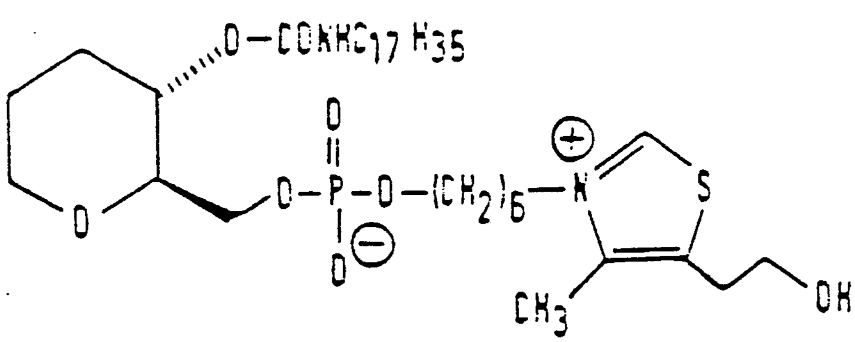

(Y)

und ihr Spiegelbild und pharmazeutisch annehmbare Salze dieser Verbindung und ihres Spiegelbildes.

30. Pharmazeutische Mischung für die Behandlung von Asthma, Entzündungen oder Schocks, welche einen PAF-Antagonisten in Kombination mit einem pharmazeutisch annehmbaren Träger oder Verdünnungsmittel enthält, worin der PAF-Antagonist zumindest eine in irgendeinem der vorhergehenden Ansprüche beanspruchte Verbindung ist.

31. Verwendung einer in irgendeinem der Ansprüche 1 bis 29 beanspruchten Verbindung für die Herstellung eines Medikaments zum Behandeln von Asthma, Entzündungen oder Schocks.

**Patentansprüche** für den Vertragsstaat: AT

1. Verfahren zum Herstellen von Verbindungen der Formel (I):

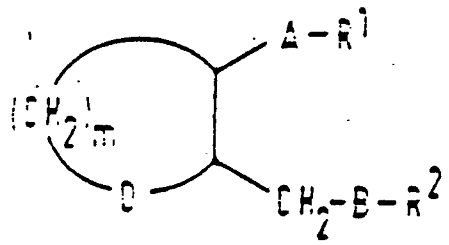

(I)

[in welcher:
m eine ganze Zahl von 2 bis 4 bedeutet;
A und B untereinander gleich oder voneinander verschieden sind und je für sich ein Sauerstoffatom oder ein Schwefelatom bedeuten; und
einer der Reste $R^1$ und $R^2$ für eine Gruppe der Formel -CONH-$R^3$ mit der Bedeutung einer $C_{10}$-$C_{22}$-Alkylgruppe für $R^3$ steht und der andere dieser Reste eine Gruppe der Formel (II):

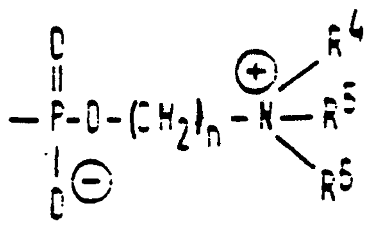

(II)

darstellt, in welcher:
n eine ganze Zahl von 2 bis 10 bedeutet; und
$R^4$, $R^5$ und $R^6$ untereinander gleich oder voneinander verschieden sind und je für sich ein Wasserstoffatom oder eine $C_1$-$C_6$-Alkylgruppe bedeuten oder $R^4$ und $R^5$ oder $R^4$, $R^5$ und $R^6$ zusammen mit dem diese Reste tragenden Stickstoffatom einen heterocyclischen Ring bilden];
und pharmazeutisch annehmbare Salze hievon, durch Umsetzen einer Verbindung der Formel (III):

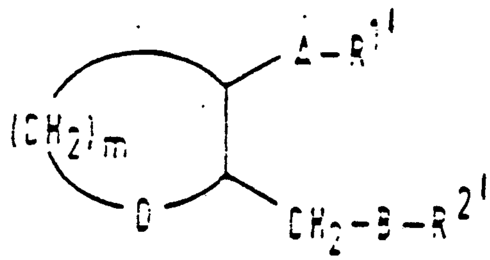

(III)

in welcher m, A und B die oben angegebene Bedeutung besitzen; und
einer der Reste $R^{1'}$ und $R^{2'}$ die oben angegegene Gruppe der Formel -CONH-$R^3$ bedeutet und der andere dieser Reste eine Gruppe der Formel (IV)

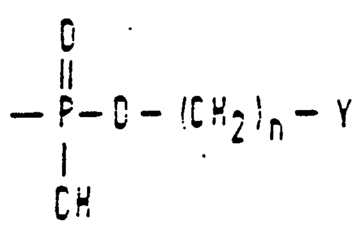

(IV)

(in welcher n die oben angegebene Bedeutung besitzt und Y ein Halogenatom darstellt) oder eine Gruppe der Formel (V):

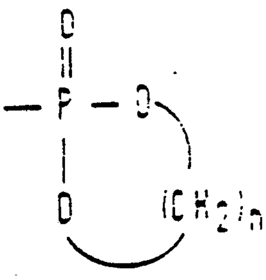

(V)

(in welcher n die oben angegebene Bedeutung besitzt) darstellt, mit einer Aminverbindung der Formel (VI):

$R^4NR^5R^6$ (VI)

(in welcher $R^4$, $R^5$ und $R^6$ die oben angegebene Bedeutung besitzen) umgesetzt wird und das Produkt gegebenenfalls in ein Salz übergeführt wird.

2. Verfahren nach Anspruch 1, worin in der Verbindung der Formel (III) m gleich ist 2 oder 3.

3. Verfahren nach Anspruch 1 oder Anspruch 2, worin in der Gruppe der Formel (IV) oder (V) n eine ganze Zahl von 2 bis 6 bedeutet.

4. Verfahren nach Anspruch 1 oder Anspruch 2, worin in der Gruppe der Formel (IV) oder (V) n gleich ist 2 oder 3.

5. Verfahren nach irgendeinem der vorhergehenden Ansprüche, worin in der Verbindung der Formel (VI) $R^4$, $R^5$ und $R^6$ untereinander gleich oder voneinander verschieden sind und je für sich ein Wasserstoffatom oder eine $C_1$-$C_6$-Alkylgruppe bedeuten.

6. Verfahren nach irgendeinem der Ansprüche 1 bis 4, worin in der Verbindung der Formel (VI) $R^4$ und $R^5$ zusammen mit dem diese Reste tragenden Stickstoffatom eine nicht-aromatische heterocyclische Gruppe mit 5 bis 7 Ringatomen bilden und $R^6$ ein Wasserstoffatom oder eine $C_1$-$C_6$-Alkylgruppe bedeutet.

7. Verfahren nach irgendeinem der Ansprüche 1 bis 4, worin in der Verbindung der Formel (VI) $R^4$, $R^5$ und $R^6$ zusammen mit dem diese Reste tragenden Stickstoffatom eine aromatische heterocyclische Gruppe mit 5 bis 10 Ringatomen bilden und diese heterocyclische Gruppe unsubstituiert ist oder zumindest einen $C_1$-$C_5$-Alkyl, $C_1$-$C_5$-Alkoxy-, $C_1$-$C_5$-Hydroxyalkyl-, Carbamoyl- oder Halogensubstituenten aufweist.

8. Verfahren nach irgendeinem der vorhergehenden Ansprüche, worin $R^{1'}$ die Gruppe der Formel $-CONHR^3$ und $R^{2'}$ die Gruppe der Formel (IV) oder (V) bedeutet.

9. Verfahren nach irgendeinem der vorhergehenden Ansprüche, worin $R^3$ in der Verbindung der Formel (III) eine Alkylgruppe mit 16 bis 18 Kohlenstoffatomen bedeutet.

10. Verfahren nach Anspruch 1, worin:
$R^{1'}$ die Gruppe der Formel $-CONHR^3$ bedeutet;
$R^2$ die Gruppe der Formel (IV) oder der Formel (V) darstellt;
$R^4$, $R^5$ und $R^6$ in der Verbindung der Formel (VI) untereinander gleich oder voneinander verschieden sind und je für sich ein Wasserstoffatom oder eine $C_1$-$C_6$-Alkylgruppe bedeuten;
n in der Gruppe der Formel (IV) oder der Formel (V) eine ganze Zahl von 2 bis 6 bedeutet; und
m in der Verbindung der Formel (III) gleich ist 2 oder 3.

11. Verfahren nach Anspruch 1 worin:
$R^{1'}$ die Gruppe der Formel $-CONHR^3$ bedeutet;
$R^{2'}$ die Gruppe der Formel (IV) oder der Formel (V) darstellt;
$R^4$ und $R^5$ in der Verbindung der Formel (VI) zusammen mit dem diese Reste tragenden Stickstoffatom eine nicht-aromatische heterocyclische Gruppe mit 5 bis 7 Ringatomen bilden und $R^6$ ein Wasserstoffatom oder eine $C_1$-$C_6$-Alkylgruppe darstellt;
n in der Gruppe der Formel (IV) oder (V) eine ganze Zahl von 2 bis 6 bedeutet; und
m in der Verbindung der Formel (III) gleich ist 2 oder 3.

12. Verfahren nach Anspruch 1, worin:
$R^{1'}$ die Gruppe der Formel $-CONHR^3$ bedeutet;
$R^{2'}$ die Gruppe der Formel (IV) oder der Formel (V) darstellt;
$R^4$, $R^5$ und $R^6$ in der Verbindung der Formel (VI) zusammen mit dem diese Reste tragenden Stickstoffatom eine aromatische heterocyclische Gruppe mit 5 bis 10 Ringatomen bilden und diese Gruppe unsubstituiert ist oder zumindest einen $C_1$-$C_5$-Alkyl-, $C_1$-$C_5$-Alkoxy-, $C_1$-$C_5$-Hydroxyalkyl-, Carbamoyl- oder Halogensubstituenten aufweist;
n in der Gruppe der Formel (IV) oder (V) eine ganze Zahl von 2 bis 6 bedeutet; und
m in der Verbindung der Formel (III) gleich ist 2 oder 3.

13. Verfahren nach Anspruch 1, worin:
$R^{1'}$ eine Gruppe der Formel $-CONHR^3$ mit der Bedeutung einer $C_{16}$-$C_{18}$-Alkylgruppe für $R^3$ darstellt;
$R^{2'}$ die Gruppe der Formel (IV) oder der Formel (V) bedeutet;
in der Verbindung der Formel (VI) $R^4$, $R^5$ und $R^6$ untereinander gleich oder voneinander verschieden sind und je für sich eine $C_1$-$C_6$-Alkylgruppe bedeuten;

n in der Gruppe der Formel (IV) oder (V) eine ganze Zahl von 2 bis 6 bedeutet; und
m in der Verbindung der Formel (III) gleich ist 2 oder 3.

14. Verfahren nach Anspruch 1, worin:
$R^{1'}$ eine Gruppe der Formel $-CONHR^3$ mit der Bedeutung einer $C_{16}$-$C_{18}$-Alkylgruppe für $R^3$ darstellt;
$R^{2'}$ die Gruppe der Formel (IV) oder der Formel (V) bedeutet;
in der Verbindung der Formel (VI) $R^4$ und $R^5$ zusammen mit dem diese Reste tragenden Stickstoffatom eine nicht-aromatische heterocyclische Gruppe mit 5 bis 7 Ringatomen bilden und $R^6$ ein Wasserstoffatom oder eine $C_1$-$C_6$-Alkylgruppe bedeutet;
n in der Gruppe der Formel (IV) oder (V) eine ganze Zahl von 2 bis 6 bedeutet; und
m in der Verbindung der Formel (III) gleich ist 2 oder 3.

15. Verfahren nach Anspruch 1, worin:
$R^{1'}$ eine Gruppe der Formel $-CONHR^3$ mit der Bedeutung einer $C_{16}$-$C_{18}$-Alkylgruppe für $R^3$ darstellt;
$R^{2'}$ die Gruppe der Formel (IV) oder der Formel (V) bedeutet;
in der Verbindung der Formel (VI) $R^4$, $R^5$ und $R^6$ zusammen mit dem diese Reste tragenden Stickstoffatom eine aromatische heterocyclische Gruppe mit 5 bis 7 Ringatomen bilden und diese Gruppe unsubstituiert ist oder zumindest einen $C_1$-$C_5$-Alkyl- oder $C_1$-$C_5$-Hydroxyalkylsubstituenten aufweist;
n in der Gruppe der Formel (IV) oder (V) eine ganze Zahl von 2 bis 6 bedeutet; und
m in der Verbindung der Formel (III) gleich ist 2 oder 3.

16. Verfahren nach Anspruch 1, worin:
$R^{1'}$ eine Gruppe der Formel $-CONHR^3$ mit der Bedeutung einer $C_{16}$-$C_{18}$-Alkylgruppe für $R^3$ darstellt;
$R^{2'}$ die Gruppe der Formel (IV) oder der Formel (V) bedeutet;
in der Verbindung der Formel (VI) $R^4$ und $R^5$ zusammen mit dem diese Reste tragenden Stickstoffatom eine Piperidino- oder 1-Pyrrolidinylgruppe bilden und $R^6$ ein Wasserstoffatom oder eine $C_1$-$C_6$-Alkylgruppe darstellt;
n in der Gruppe der Formel (IV) oder (V) eine ganze Zahl von 2 bis 6 bedeutet; und
m in der Verbindung der Formel (III) gleich ist 2 oder 3.

17. Verfahren nach Anspruch 1, worin:
$R^{1'}$ eine Gruppe der Formel $-CONHR^3$ mit der Bedeutung einer $C_{16}$-$C_{18}$-Alkylgruppe für $R^3$ darstellt;
$R^{2'}$ die Gruppe der Formel (IV) oder (V) bedeutet,
in der Verbindung der Formel (VI) $R^4$, $R^5$ und $R^6$ zusammen mit dem diese Reste tragenden Stickstoffatom eine 1-Pyridyl- oder 1-Thiazolylgruppe bilden, die unsubstituiert ist oder zumindest einen $C_1$-$C_5$-Alkyl- oder $C_1$-$C_5$-Hydroxyalkylsubstituenten aufweist;
n in der Gruppe der Formel (IV) oder (V) eine ganze Zahl von 2 bis 6 bedeutet; und
m in der Verbindung der Formel (III) gleich ist 2 oder 3.

18. Verfahren nach Anspruch 1, worin die Reagerzien und Reaktionsbedingungen so gewählt werden, daß eine Verbindung der Formel:

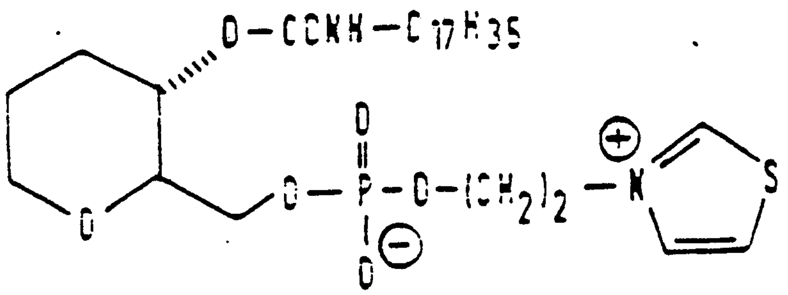

(b)

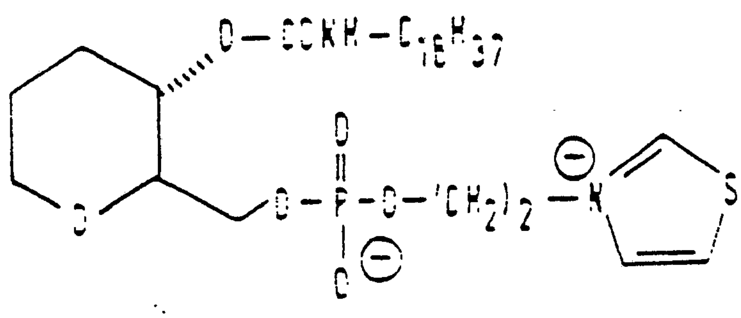

(c)

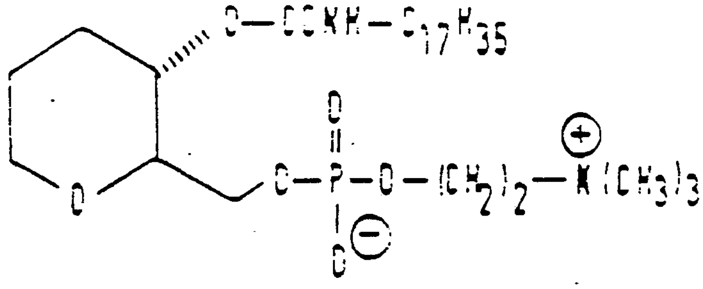

(d)

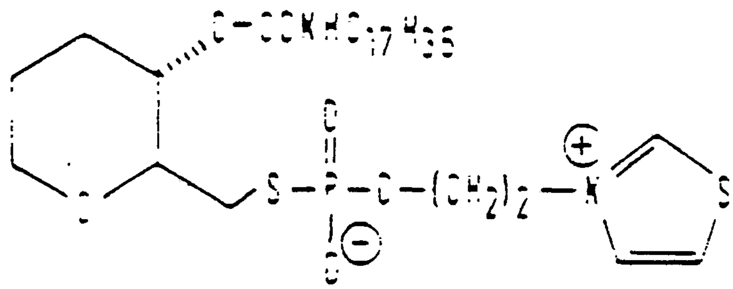

(g)

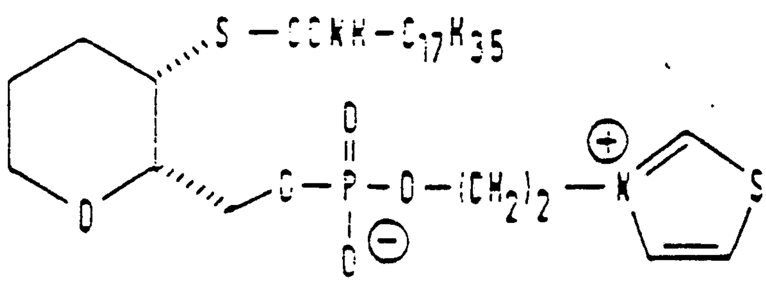

(j)

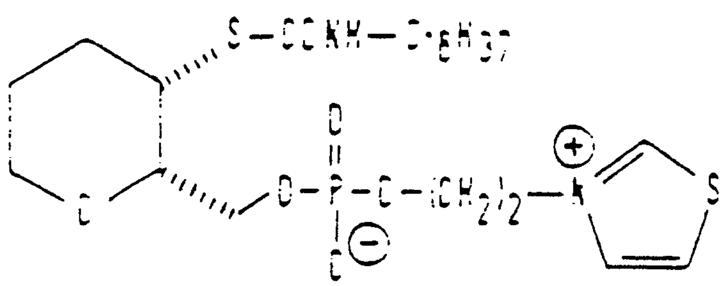

(k)

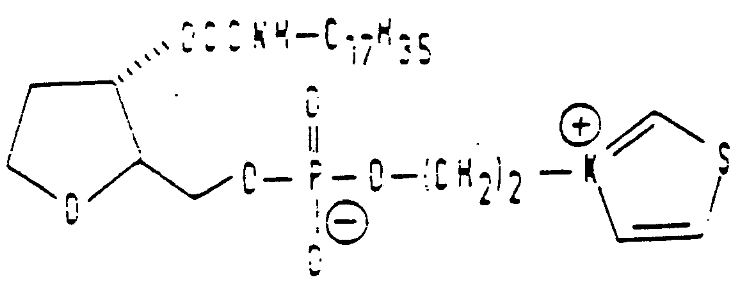

(p)

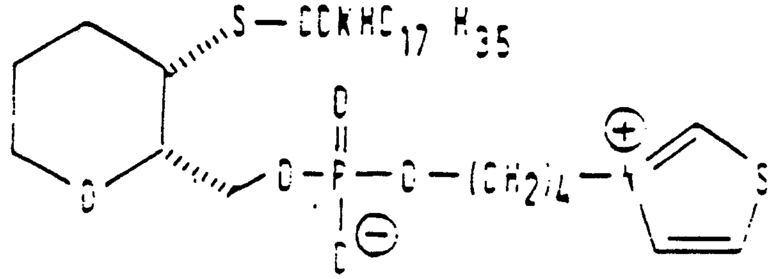

(u)

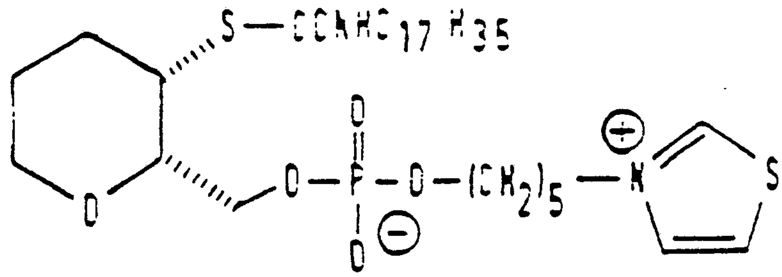

(v)

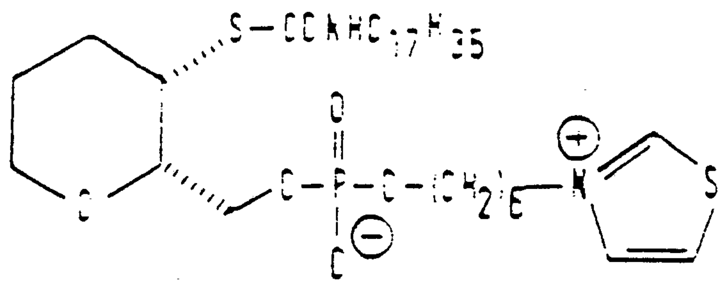

(w)

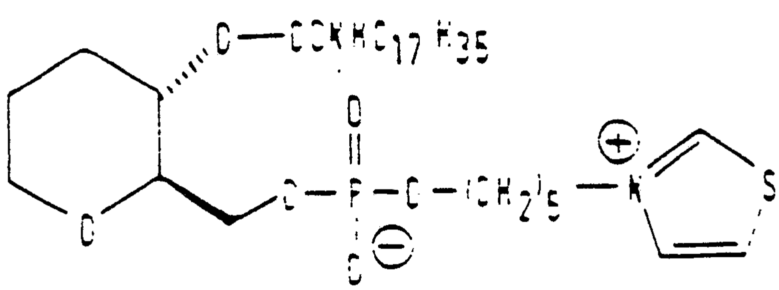

(x)

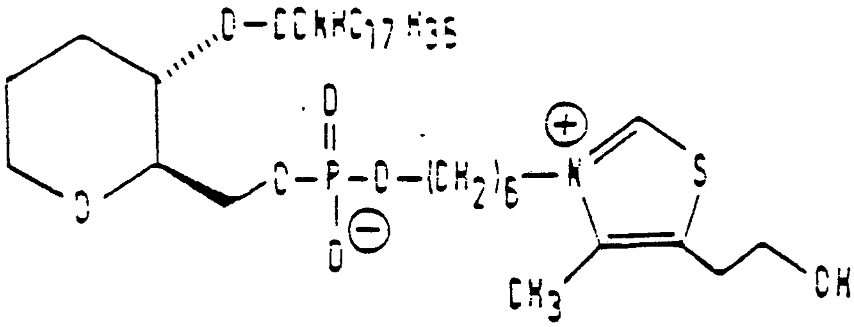

(y)

oder das Spiegelbild dieser Verbindungen oder pharmazeutisch annehmbare Salze dieser Verbindungen oder ihrer Spiegelbilder hergestellt werden.

19. Verwendung einer in Anspruch 1 definierten Verbindung der Formel (I) oder eines pharmazeutisch annehmbaren Salzes hievon zum Herstellen eines Heilmittels für die Behandlung von Asthma, Entzündungen oder Schocks.

20. Verwendung einer in Anspruch 18 gezeigten Verbindung der Formel (b), (c), (d), (g), (j), (k), (p), (u), (v), (w), (x) oder (y) oder eines Spiegelbildes hievon oder eines pharmazeutisch annehmbaren Salzes dieser Verbindung oder ihres Spiegelbildes zum Herstellen eines Heilmittels für die Behandlung von Asthma, Entzündungen oder Schocks.

**Revendications** pour les Etats Contractants: BE, CH, DE, FR, GB, IT, LI, LU, NL, SE.

1. Composés de formule I:

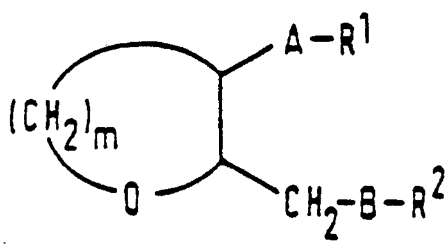

(I)

dans laquelle:

m est un entier de 2 à 4;

A et B sont semblables ou différents et représentent chacun un atome d'oxygène ou un atome de soufre; et un de $R^1$ et $R^2$ représente un groupe de formule $-CONH-R^3$, dans laquelle $R^3$ représente un groupe alkyle en $C_{10}$-$C_{22}$ et l'autre représente un groupe de formule (II):

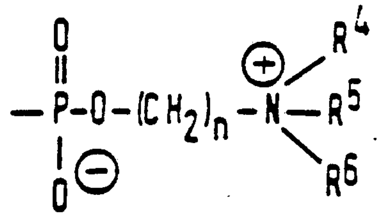

(II)

dans laquelle:

n représente un entier de 2 à 10; et

$R^4$, $R^5$ et $R^6$ sont semblables ou différents et représentent chacun un atome d'hydrogène ou un groupe alkyle en $C_1$-$C_6$, ou $R^4$ et $R^5$ ou $R^4$, $R^5$ et $R^6$, avec l'atome d'azote auquel ils sont fixés, forment un hétérocycle;

et leurs sels pharmaceutiquement acceptables.

2. Composés selon la revendication 1, où m est 2 ou 3.

3. Composés selon la revendication 1 ou la revendication 2, où n est un entier de 2 à 6.

4. Composés selon la revendication 1 ou la revendication 2, où n est 2 ou 3.

5. Composés selon l'une quelconque des revendications 1 à 4, où $R^4$, $R^5$ et $R^6$ sont semblables ou différents et représentent chacun un atome d'hydrogène ou un groupe alkyle en $C_1$-$C_6$.

6. Composés selon l'une quelconque des revendications 1 à 4, où $R^4$ et $R^5$, avec l'atome d'azote auquel ils

sont fixés représentent un groupe hétérocyclique non aromatique ayant 5 à 7 atomes nucléaires et $R^6$ représente un atome d'hydrogène ou un groupe alkyle en $C_1$-$C_6$.

7. Composés selon l'une quelconque des revendications 1 à 4, où $R^4$, $R^5$ et $R^6$, avec l'atome d'azote auquel ils sont fixés, représentent un groupe hétérocyclique aromatique ayant 5 à 10 atomes nucléaires, ledit groupe hétérocyclique étant non substitué ou ayant au moins un substituant alkyle en $C_1$-$C_5$, alcoxy en $C_1$-$C_5$, hydroxyalkyle en $C_1$-$C_5$, carbamoyle ou halogène.

8. Composés selon l'une quelconque des revendications 1 à 7, où $R^1$ représente ledit groupe de formule $-CONHR^3$ et $R^2$ représente ledit groupe de formule (II).

9. Composés selon l'une quelconque des revendications 1 à 8; où $R^3$ représente un groupe alkyle ayant 16 à 18 atomes de carbone.

10. Composés selon la revendication 1,
$R^1$ représente ledit groupe de formule $-CONHR^3$;
$R^2$ représente ledit groupe de formule (II);
$R^4$, $R^5$ et $R^6$ sont semblables ou différents et représentent chacun un atome d'hydrogène ou un groupe alkyle en $C_1$-$C_6$;
n est un entier de 2 à 6 et
m est 2 ou 3.

11. Composés selon la revendication 1, où:
$R^1$ représente ledit groupe de formule $-CONHR^3$;
$R^2$ représente ledit groupe de formule (II);
$R^4$ et $R^5$, avec l'atome d'azote auquel ils sont fixés, représentent un groupe hétérocyclique non aromatique ayant 5 à 7 atomes nucléaires et $R^6$ représente un atome d'hydrogène ou un groupe alkyle en $C_1$-$C_6$;
n est un entier de 2 à 6; et
m est 2 ou 3.

12. Composés selon la revendication 1, où:
$R^1$ représente ledit groupe de formule $-CONHR^3$;
$R^2$ représente ledit groupe de formule (II);
$R^4$, $R^5$ et $R^6$, avec l'atome d'azote au quel ils sont fixés, représentent un groupe hétérocyclique aromatique ayant 5 à 10 atomes nucléaires, ledit groupe étant non substitué ou ayant au moins un substituant alkyle en $C_1$-$C_5$, alcoxy en $C_1$-$C_5$, hydroxyalkyle en $C_1$-$C_5$, carbamoyle ou halogène;
n est un entier de 2 à 6; et
m est 2 ou 3.

13. Composés selon la revendication 1, où:
$R^1$ représente un groupe de formule $-CONHR^3$ où $R^3$ représente un groupe alkyle en $C_{16}$-$C_{18}$;
$R^2$ représente ledit groupe de formule (II);
$R^4$, $R^5$ et $R^6$ sont semblables ou différents et représentent chacun un groupe alkyle en $C_1$-$C_6$;
n est un entier de 2 à 6; et
m est 2 ou 3.

14. Composés selon la revendication 1, où:
$R^1$ représente un groupe de formule $-CONHR^3$ où $R^3$ représente un groupe alkyle en $C_{16}$-$C_{18}$;
$R^2$ représente ledit groupe de formule (II);
$R^4$ et $R^5$, avec l'atome d'azote auquel ils sont fixés, représentent un groupe hétérocyclique non aromatique ayant 5 à 7 atomes nucléaires et $R^6$ représente un atome d'hydrogène ou un groupe alkyle en $C_1$-$C_6$;
n est un entier de 2 à 6; et
m est 2 ou 3.

15. Composés selon la revendication 1, où:
$R^1$ représente un groupe de formule $-CONHR^3$ où $R^3$ représente un groupe alkyle en $C_{16}$-$C_{18}$;
$R^2$ représente ledit groupe de formule (II);
$R^4$, $R^5$ et $R^6$, avec l'atome d'azote auquel ils sont fixés, représentent un groupe hétérocyclique aromatique ayant 5 à 7 atomes nucléaires, ledit groupe étant non substitué ou ayant au moins un substituant alkyle en $C_1$-$C_5$ ou hydroxyalkyle en $C_1$-$C_5$;
n est un entier de 2 à 6; et
m est 2 ou 3.

16. Composés selon la revendication 1, où:
$R^1$ représente un groupe de formule $-CONHR^3$ où $R^3$ représente un groupe alkyle en $C_{16}$-$C_{18}$;
$R^2$ représente ledit groupe de formule (II);
$R^4$ et $R^5$, avec l'atome d'azote auquel ils sont fixés, représentent un groupe pipéridino ou 1-pyrrolidinyle et $R^6$ représente un atome d'hydrogène ou un groupe alkyle en $C_1$-$C_6$;
n est un entier de 2 à 6; et
m est 2 ou 3.

17. Composés selon la revendication 1, où:
$R^1$ représente un groupe de formule $-CONHR^3$ où $R^3$ représente un groupe alkyle en $C_{16}$-$C_{18}$;
$R^2$ représente ledit groupe de formule (II);
$R^4$, $R^5$ et $R^6$, avec l'atome d'azote auquel ils sont fixés, représentent un groupe 1-pyridyle ou 1-thiazolyle qui est non substitué ou qui a au moins un substituant alkyle en $C_1$-$C_5$ ou hydroxyalkyle en $C_1$-$C_5$;

n est un entier de 2 à 6; et
m est 2 ou 3.
18. Composé de formule:

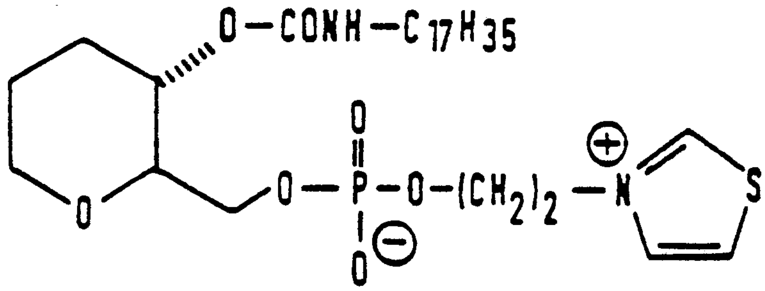

(b)

son image spéculaire et les sels pharmaceutiquement acceptables dudit composé et de son image spéculaire.
19. Composé de formule:

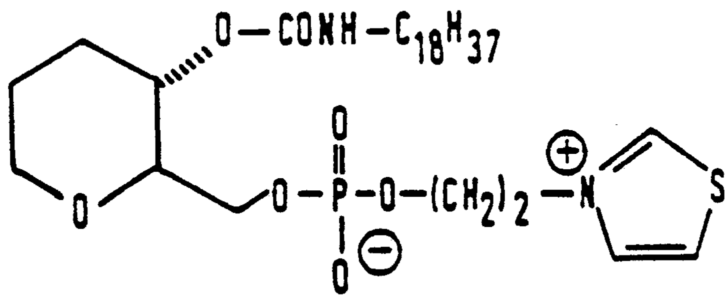

(c)

son image spéculaire et les sels pharmaceutiquement acceptables dudit composé et de son image spéculaire.
20. Composé de formule:

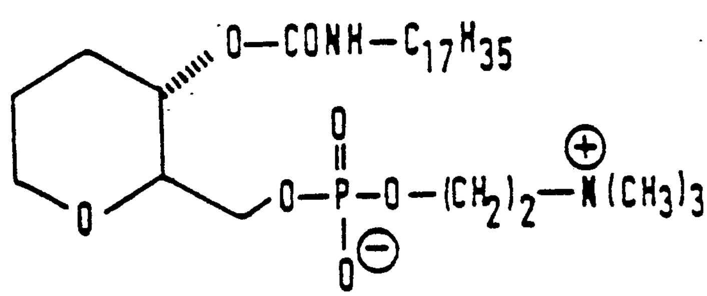

(d)

son image spéculaire et les sels pharmaceutiquement acceptables dudit composé et de son image spéculaire.
21. Composé de formule:

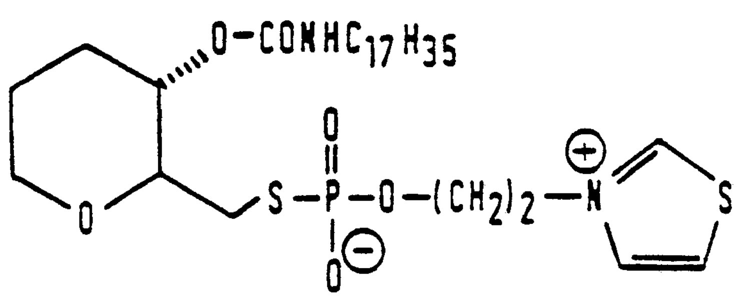

(g)

son image spéculaire et les sels pharmaceutiquement acceptables dudit composé et de son image spéculaire.

22. Composé de formule:

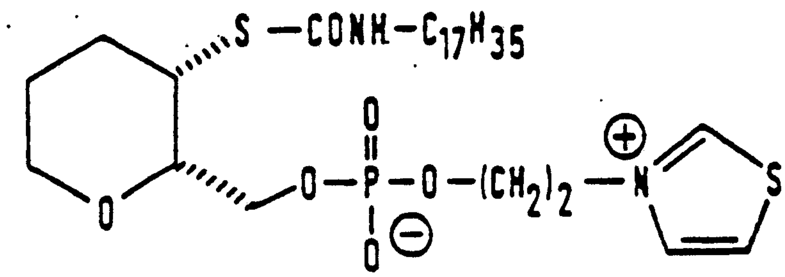

(j)

son image spéculaire et les sels pharmaceutiquement acceptables dudit composé et de son image spéculaire.

23. Composé de formule:

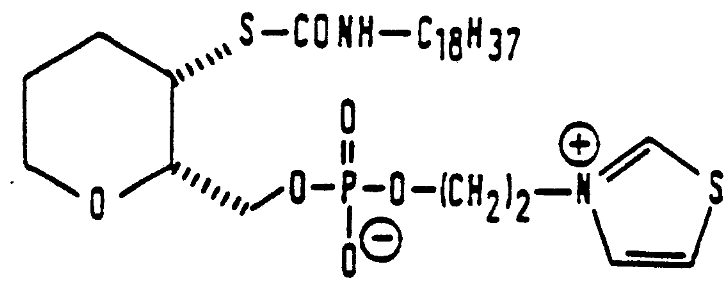

(k)

son image spéculaire et les sels pharmaceutiquement acceptables dudit composé et de son image spéculaire.

24. Composé de formule:

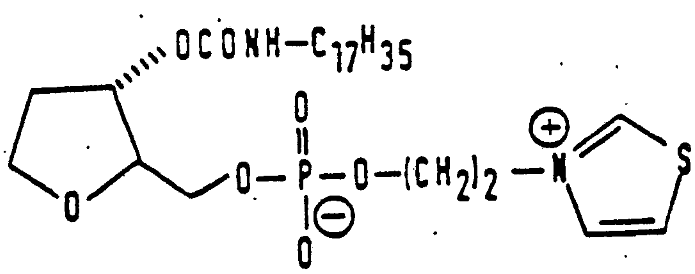

(p)

son image spéculaire et les sels pharmaceutiquement acceptables dudit composé et de son image spéculaire.

25. Composé de formule:

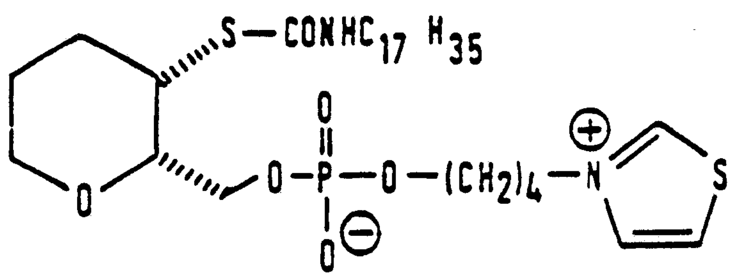

(u)

son image spéculaire et les sels pharmaceutiquement acceptables dudit composé et de son image spéculaire.

26. Composé de formule:

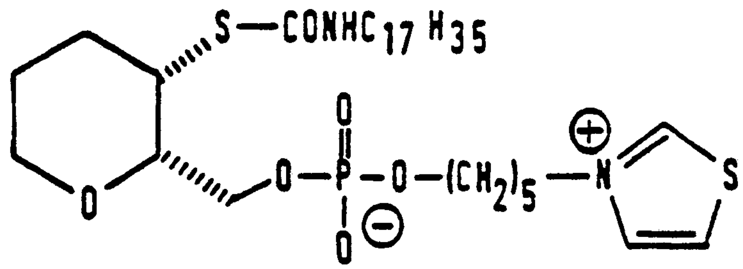

(v)

son image spéculaire et les sels pharmaceutiquement acceptables dudit composé et de son image spéculaire.

27. Composé de formule:

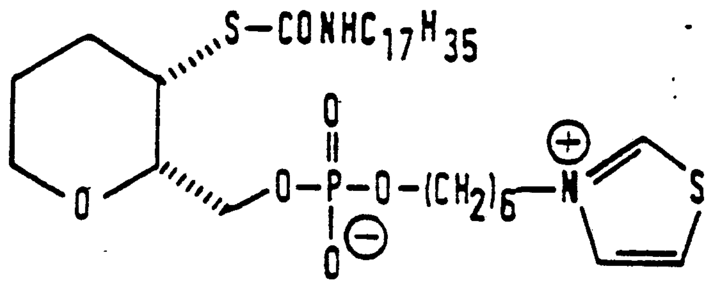

(w)

son image spéculaire et les sels pharmaceutiquement acceptables dudit composé et de son image spéculaire.

28. Composé de formule:

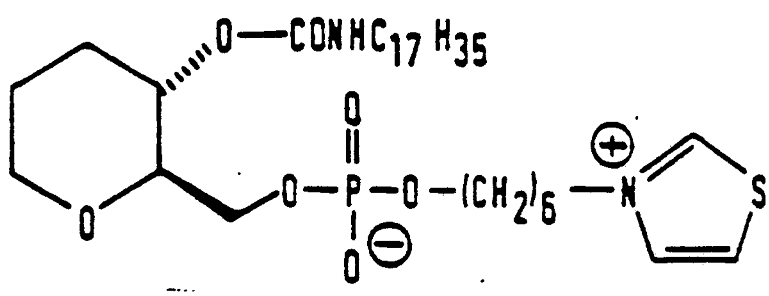

(x)

son image spéculaire et les sels pharmaceutiquement acceptables dudit composé et de son image spéculaire.

29. Composé de formule:

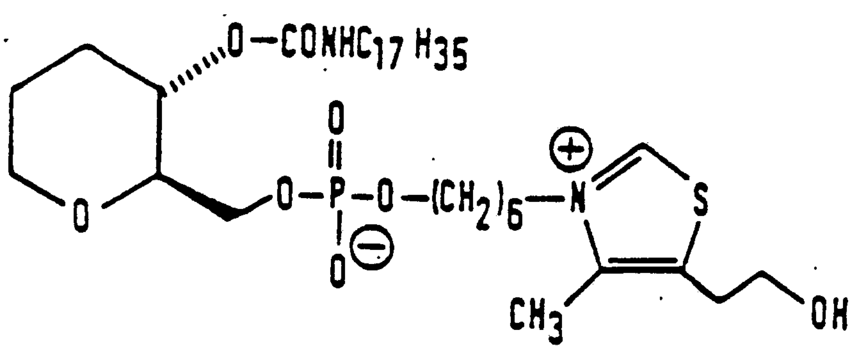

(y)

son image spéculaire et les sels pharmaceutiquement acceptables dudit composé et de son image spéculaire.

30. Composition pharmaceutique pour le traitement de l'asthme, de l'inflammation ou du choc, comprenant un antagoniste du PAF en combinaison avec un véhicule ou diluant pharmaceutiquement acceptable, dans laquelle l'antagoniste du PAF est au moins un composé selon l'une quelconque des revendications précédentes.

31. Utilisation pour la fabrication d'un médicament pour le traitement de l'asthme, de l'inflammation ou du choc, d'un composé selon l'une quelconque des revendications 1 à 29.

**Revendications** l'Etat Contractant: AT.

1. Procédé pour préparer les composés de formule (II):

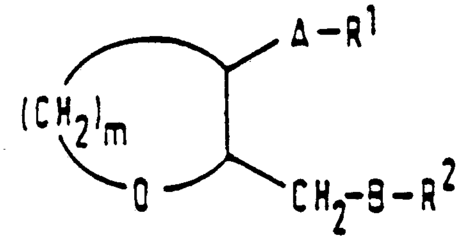

(I)

[dans laquelle:
m est un entier de 2 à 4;
A et B sont semblables ou différents et représentent chacun un atome d'oxygène ou un atome de soufre; et un de $R^1$ et $R^2$ représente un groupe de formule -CONH-$R^3$, dans laquelle $R^3$ représente un groupe alkyle en $C_{10}$-$C_{22}$ et l'autre représente un groupe de formule (II):

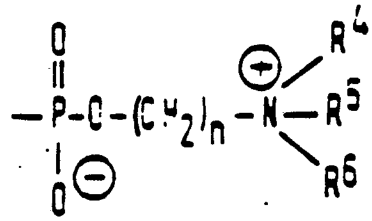

(II)

dans laquelle:
n représente un entier de 2 à 10; et
$R^4$, $R^5$ et $R^6$ sont semblables ou différents et représentent chacun un atome d'hydrogène ou un groupe alxyle en $C_1$-$C_6$, ou $R^4$ et $R^5$ ou $R^4$, $R^5$ et $R^6$, avec l'atome d'azote auquel ils sont fixés, forment un hétérocycle];
et leurs sels pharmaceutiquement acceptables par réaction d'un composé de formule (III):

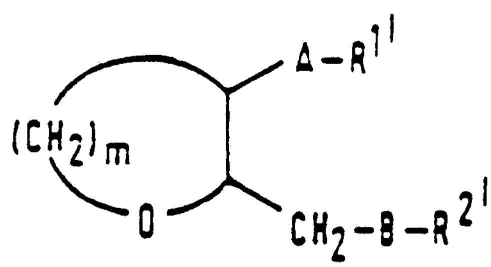

(III)

dans laquelle m, A et B sont comme défini ci-dessus; et un de $R^{1'}$ et $R^{2'}$ représente ledit groupe de formule -CONH-$R^3$, défini ci-dessus, et l'autre représente un groupe de formule (IV):

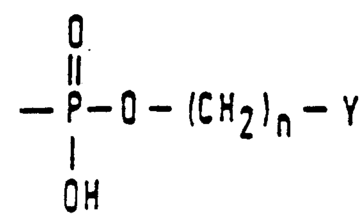

(IV)

(dans laquelle n est comme défini ci-dessus et Y représente un atome d'halogène) ou un groupe de formule (V):

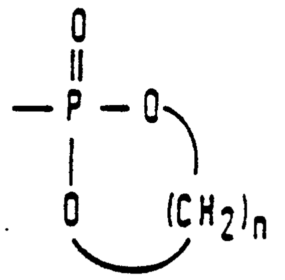

(V)

(dans laquelle n est comme défini ci-dessus) avec une amine de formule (VI):

$R^4NR^5R^6$ (VI)

(dans laquelle $R^4$, $R^5$ et $R^6$ sont comme défini ci-dessus) et, facultativement, la salification du produit.

2. Procédé selon la revendication 1 où, dans ledit composé de formule (III), m est 2 ou 3.

3. Procédé selon la revendication 1 ou la revendication 2 où, dans ledit groupe de formule (IV) ou (V), n est un entier de 2 à 6.

4. Procédé selon la revendication 1 ou la revendication 2 où, dans ledit groupe de formule (IV) ou (V), n est 2 ou 3.

5. Procédé selon l'une quelconque des revendications précédentes où, dans ledit composé de formule (VI), $R^4$, $R^5$ et $R^6$ sont semblables ou différents et représentent chacun un atome d'hydrogène ou un groupe alkyle en $C_1$-$C_6$.

6. Procédé selon l'une quelconque des revendications 1 à 4, où, dans ledit composé de formule (VI), $R^4$ et $R^5$, avec l'atome d'azote auquel ils sont fixés, représentent un groupe hétérocyclique non aromatique ayant 5 à 7 atomes nucléaires et $R^6$ représente un atome d'hydrogène ou un groupe alkyle en $C_1$-$C_6$.

7. Procédé selon l'une quelconque des revendications 1 à 4 où, dans ledit composé de formule (VI), $R^4$, $R^5$ et $R^6$, avec l'atome d'azote auquel ils sont fixés, représentent un groupe hétérocyclique aromatique ayant 5 à 10 atomes nucléaires, ledit groupe hétérocyclique étant non substitué ou ayant au moins un substituant alkyle en $C_1$-$C_5$, alcoxy en $C_1$-$C_5$, hydroxyalkyle en $C_1$-$C_5$, carbamoyle ou halogène;

8. Procédé selon l'une quelconque des revendications précédentes, où $R^{1'}$ représente ledit groupe de formule -$CONHR^3$ et $R^{2'}$ représente ledit groupe de formule (IV) ou (V).

9. Procédé selon l'une quelconque des revendications précédentes où $R^3$, dans ledit composé de formule (III), représente un groupe alkyle ayant 16 à 18 atomes de carbone.

10. Procédé selon la revendication 1, où:
$R^{1'}$ représente ledit groupe de formule -$CONHR^3$;
$R^{2'}$ représente ledit groupe de formule (IV) ou (V);
$R^4$, $R^5$ et $R^6$, dans ledit composé de formule (VI), sont semblables ou différents et représentent chacun un atome d'hydrogène ou un groupe alkyle en $C_1$-$C_6$;
n, dans ledit groupe de formule (IV) ou (V), est un entier de 2 à 6; et
m, dans ledit composé de formule (III), est 2 ou 3.

11. Procédé selon la revendication 1, où:
$R^{1'}$ représente ledit groupe de formule -$CONHR^3$;
$R^{2'}$ représente ledit groupe de formule (IV) ou (V);
dans ledit composé de formule (VI), $R^4$ et $R^5$, avec l'atome d'azote auquel ils sont fixés, représentent un groupe hétérocyclique non aromatique ayant 5 à 7 atomes nucléaires et $R^6$ représente un atome d'hydrogène ou un groupe alkyle en $C_1$-$C_6$;
n, dans ledit groupe de formule (IV) ou (V), est un entier de 2 à 6; et
m, dans ledit composé de formule (III), est 2 ou 3.

12. Procédé selon la revendication 1 où:
$R^{1'}$ représente ledit groupe de formule -$CONHR^3$;
$R^{2'}$ représente ledit groupe de formule (IV) ou (V); dans ledit composé de formule (VI), $R^4$, $R^5$ et $R^6$, avec l'atome d'azote auquel ils sont fixés, représentent un groupe hétérocyclique aromatique ayant 5 à 10 atomes nucléaires, ledit groupe étant non substitué ou ayant au moins un substituant alkyle en $C_1$-$C_5$, alcoxy en $C_1$-$C_5$, hydroxyalkyle en $C_1$-$C_5$, carbamoyle ou halogène;
n, dans ledit groupe de formule (IV) ou (V) est un entier de 2 à 6; et
m, dans ledit composé de formule (III), est 2 ou 3.

13. Procédé selon la revendication 1 où:
$R^{1'}$ représente un groupe de formule -$CONHR^3$, où $R^3$ représente un groupe alkyle en $C_{16}$-$C_{18}$;
$R^{2'}$ représente ledit groupe de formule (IV) ou (V); dans ledit composé de formule (VI), $R^4$, $R^5$ et $R^6$ sont semblables ou différents et représentent chacun un groupe alkyle en $C_1$-$C_6$;

n, dans ledit groupe de formule (IV) ou (V), est un entier de 2 à 6; et

m, dans ledit composé de formule (III), est 2 ou 3.

14. Procédé selon la revendication 1 où:

$R^{1'}$ représente un groupe de formule -CONHR$^3$, où $R^3$ représente un groupe alkyle en $C_{16}$-$C_{18}$;

$R^{2'}$ représente ledit groupe de formule (IV) ou (V);

dans ledit composé de formule (VI), $R^4$ et $R^5$, avec l'atome d'azote auquel ils sont fixés, représentent un groupe hétérocyclique non aromatique ayant 5 à 7 atomes nucléaires et $R^6$ représente un atome d'hydrogène ouun groupe alkyle en $C_1$-$C_6$;

n, dans ledit groupe de formule (IV) ou (V), est un entier de 2 à 6; et

m, dans ledit composé de formule (III), est 2 ou 3.

15. Procédé selon la revendication 1 où:

$R^{1'}$ représente un groupe de formule -CONHR$^3$, où $R^3$ représente un groupe alkyle en $C_{16}$-$C_{18}$;

$R^{2'}$ représente ledit groupe de formule (IV) ou (V);

dans ledit composé de formule (VI), $R^4$, $R^5$ et $R^6$, avec l'atome d'azote auquel ils sont fixés, représentent un groupe hétérocyclique aromatique ayant 5 à 7 atomes nucléaires, ledit groupe étant non substitué ou ayant au moins un substituant alkyle en $C_1$-$C_5$ ou hydroxyalkyle en $C_1$-$C_5$;

n, dans ledit groupe de formule (IV) ou (V), est un entier de 2 à 6; et

m, dans ledit composé de formule (III), est 2 ou 3.

16. Procédé selon la revendication 1 où:

$R^{1'}$ représente un groupe de formule -CONHR$^3$, où $R^3$ représente un groupe alkyle en $C_{16}$-$C_{18}$;

$R^{2'}$ représente ledit groupe de formule (IV) ou (V);

dans ledit composé de formule (VI), $R^4$ et $R^5$ avec l'atome d'azote auquel ils sont fixés, représentent un groupe pipéridino ou 1-pyrrolidinyle et 6 représente un atome d'hydrogène ou un groupe alkyle en $C_1$-$C_6$;

n, dans ledit groupe de formule (IV) ou (V), est un entier de 2 à 6; et

m, dans ledit composé de formule (III), est 2 ou 3.

17. Procédé selon la revendication 1 où:

$R^{1'}$ représente un groupe de formule -CONHR$^3$, ou $R^3$ représente un groupe alkyle en $C_{16}$-$C_{18}$;

$R^{2'}$ représente ledit groupe de formule (IV) ou (V);

dans ledit composé de formule (VI), $R^4$, $R^5$ et $R^6$, avec l'atome d'azote auquel ils sont fixés, représentent un groupe 1-pyridyle ou 1-thiazolyle, qui est non substitué ou a au moins un substituant alkyle en $C_1$-$C_5$ ou hydroxyalkyle en $C_1$-$C_5$;

n, dans ledit groupe de formule (IV) ou (V), est un entier de 2 à 6; et

m, dans ledit composé de formule (III), est 2 ou 3.

18. Procédé selon la revendication 1, où les composés réagissants et les conditions réactionnelles sont choisis de façon à produire un composé de formule:

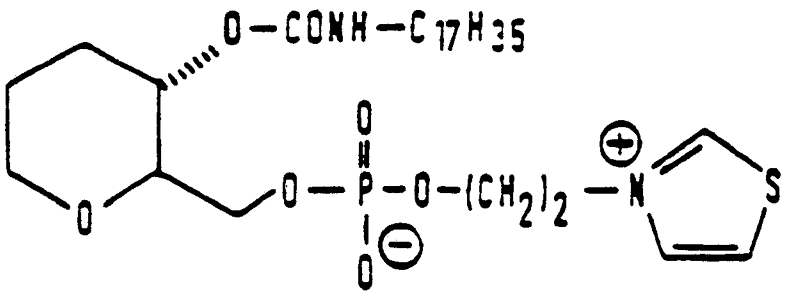

(b)

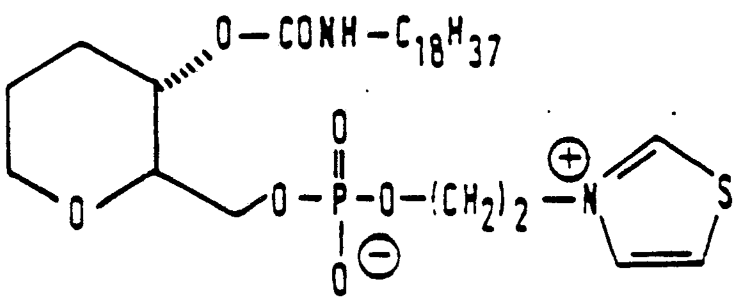

(c)

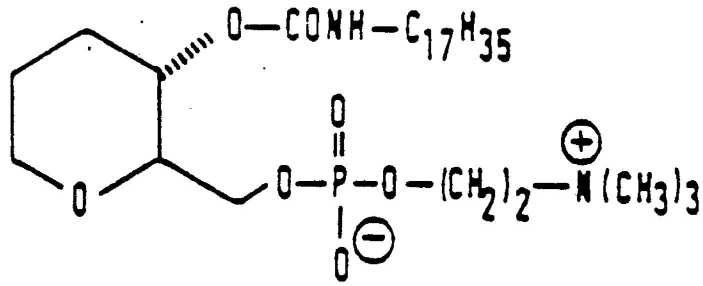

(d)

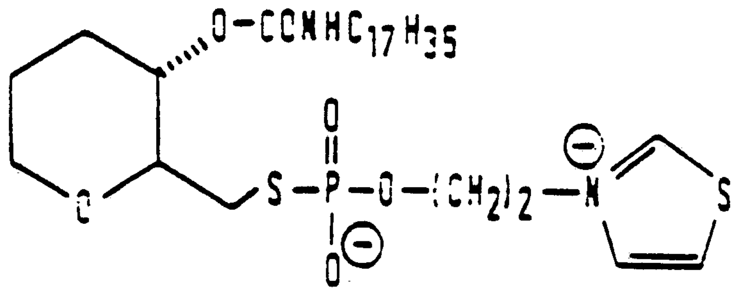

(g)

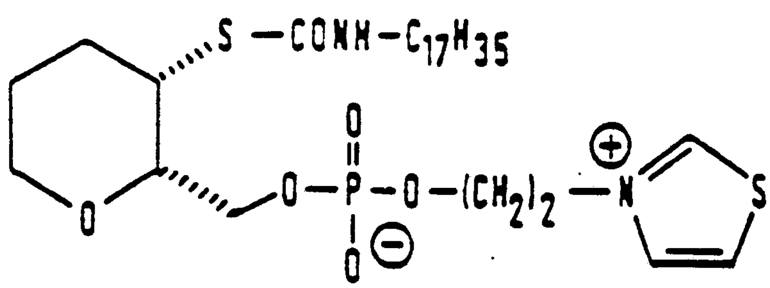

(j)

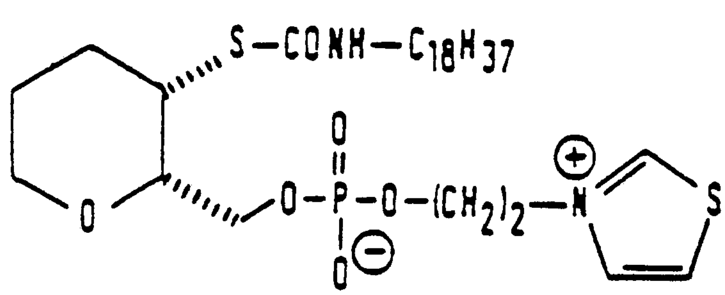

(k)

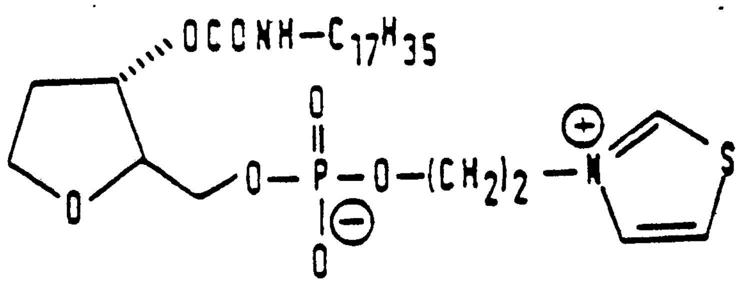

(p)

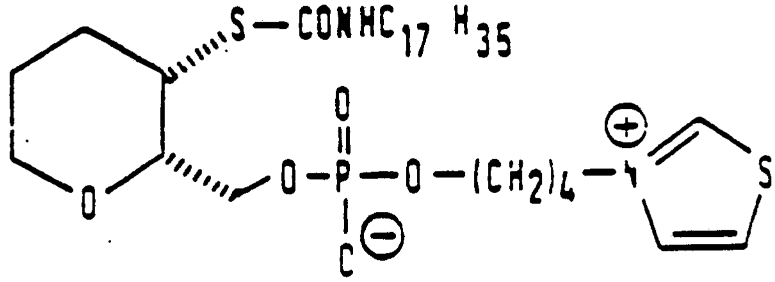

(u)

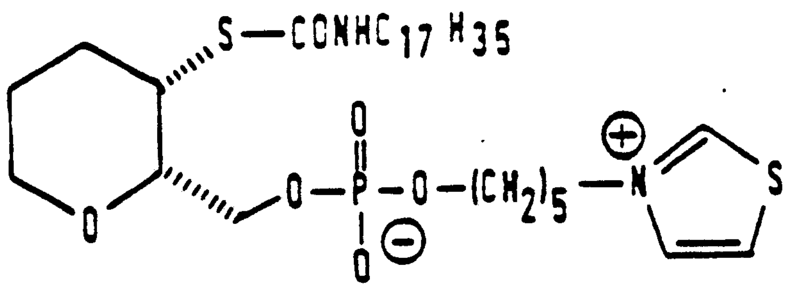

(v)

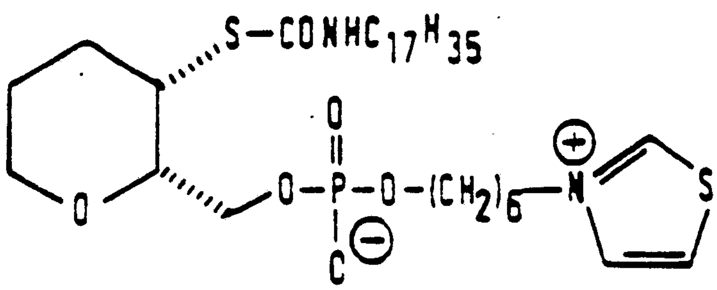

(w)

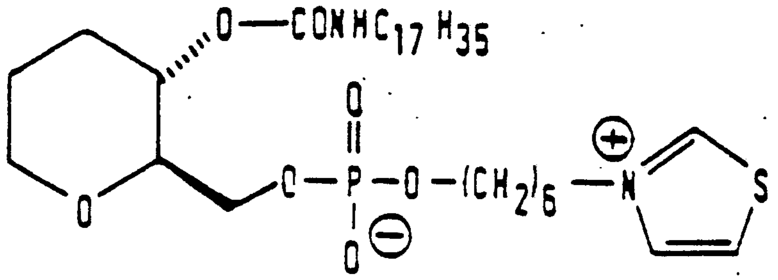

(x)

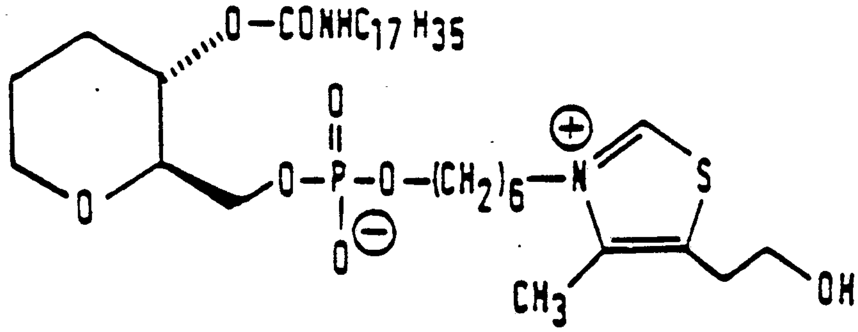

(y)

ou l'image spéculaire desdits composés ou les sels pharmaceutiquement acceptables desdits composés ou de leurs images spéculaires.

19. Utilisation, pour la préparation d'un médicament pour le traitement de l'asthme, de l'inflammation ou du choc, d'un composé de formule (I) ou d'un sel pharmaceutiquement acceptable de celui-ci comme défini dans la revendication 1.

20. Utilisation, pour la préparation d'un médicament pour le traitement de l'asthme, de l'inflammation ou du choc, d'un composé de formule (b), (c), (d), (g), (j), (k), (p), (u), (v), (w), (x) ou (y), comme indiqué dans la revendication 18, ou d'une image spéculaire de celui-ci ou d'un sel pharmaceutiquement acceptable dudit composé ou de son image spéculaire.